# FASCICULE DE BREVET EUROPEEN

(11) **EP 3 191 514 B1**
(45) Date de publication et mention de la délivrance du brevet: **08.04.2020**
(21) Numéro de dépôt: 15770570.8
(22) Date de dépôt: 10.09.2015
(51) Int. Cl.: C07K 16/26, A61P 15/08

(54) **LIGANDS POTENTIALISANTS DE LA BIOACTIVITE DES GONADOTROPHINES**
LIGANDEN ZUR VERSTÄRKUNG DER BIOAKTIVITÄT VON GONADOTROPINEN
LIGANDS THAT POTENTIATE THE BIOACTIVITY OF GONADOTROPINS

(30) Priorité: 10.09.2014 FR 1458469; 31.08.2015 FR 1558078
(43) Date de publication de la demande: 19.07.2017
(73) Titulaire: ReproPharm Vet, 37380 Nouzilly (FR)
(72) Inventeur: KARA, Elodie, 37250 Veigne (FR); DECOURTYE, Jérémye, 37000 Tours (FR); CASTERET, Sophie, 37190 Valleres (FR); MAUREL, Marie-Christine, 37000 Tours (FR)
(74) Mandataire: Novagraaf Technologies
(86) Numéro de dépôt international: PCT/FR2015/052414
(87) Numéro de publication internationale: WO 2016/038309

(56) Documents cités:
- WO-A1-97/12038
- WO-A1-2012/066519
- US-A1- 2002 102 613
- GLENCROSS R G ET AL: "Monoclonal antibody enhancement of FSH-induced uterine growth in snell dwarf mice", JOURNAL OF ENDOCRINOLOGY, vol. 136, no. 3, 1 mars 1993 (1993-03-01), pages R5-R7, XP009145848, SOCIETY FOR ENDOCRINOLOGY, GB ISSN: 0022-0795, DOI: 10.1677/JOE.0.136R005
- ALFREDO ULLOA-AGUIRRE ET AL: "Novel pathways in gonadotropin receptor signaling and biased agonism", REVIEWS IN ENDOCRINE AND METABOLIC DISORDERS, vol. 12, no. 4, 28 avril 2011 (2011-04-28) , pages 259-274, XP019972343, KLUWER ACADEMIC PUBLISHERS, BO ISSN: 1573-2606, DOI: 10.1007/S11154-011-9176-2
- MILLER K F ET AL: "Immunoaffinity chromatography of bovine FSH using monoclonal antibodies.", THE JOURNAL OF ENDOCRINOLOGY, vol. 115, no. 2, novembre 1987 (1987-11), pages 283-288, XP009187286, ISSN: 0022-0795

## Description

### Domaine technique

La présente invention se rapporte à des anticorps dirigés contre l'hormone folliculo-stimulante (FSH) capables de potentialiser la bioactivité des gonadotrophines.

La présente invention trouve ses applications principalement en médecine humaine et vétérinaire, pour induire l'ovulation chez un mammifère femelle.

Dans la description ci-dessous, les références entre crochets (**[ ]**) renvoient à la liste des références présentée à la fin du texte.

### Etat de la technique

Les gonadotrophines (ou gonadotropines) sont des hormones glycoprotéiques complexes jouant un rôle central dans la régulation de la reproduction chez les vertébrés en agissant sur les fonctions des gonades (ovaires et testicules). Deux de ces hormones sont sécrétées chez tous les vertébrés : l'hormone lutéinisante (LH) et l'hormone folliculo-stimulante (FSH). Chez deux groupes de mammifères, équidés et primates, il existe en outre une gonadotrophine chorionique (CG) sécrétée par le placenta : la choriogonadotropine humaine (hCG) et la choriogonadotropine équine (eCG) qui agissent toutes deux via des récepteurs LH.

L'hormone lutéinisante (LH) est produite par les cellules gonadotropes du lobe antérieur de l'hypophyse sous stimulation de la GnRH, elle-même produite par l'hypothalamus. La LH stimule la production de testostérone chez les mâles, tandis qu'elle intervient dans les modifications du cycle ovarien chez les femelles où elle est responsable de la croissance folliculaire terminale et de l'ovulation puis de la transformation du follicule ovulatoire rompu en corps jaune. Pendant la phase lutéale du cycle menstruel, la LH stimule la sécrétion de progestérone par le corps jaune, indispensable au développement précoce et à l'implantation de l'embryon. La LH est constituée d'une sous-unité α commune à toutes les hormones glycoprotéiques d'une même espèce (comme la FSH, la CG et l'hormone thyréostimulante, la TSH) et d'une sous-unité β responsable de la spécificité d'activité de l'hormone ; activité qui n'existe que si les deux sous-unités sont associées de manière non covalente sous-forme d'un dimère.

L'hormone folliculo-stimulante (ou FSH) est produite par l'anté-hypophyse sous stimulation de la GnRH produite par l'hypothalamus. Chez les mâles, elle stimule les cellules de Sertoli indispensables à la spermatogénèse. Chez les femelles, elle est responsable du recrutement des follicules primordiaux, immatures, de leur croissance et de leur différentiation en follicules pré-ovulatoires en stimulant les récepteurs FSH des cellules de la granulosa. La FSH est constituée de deux sous-unités α et β, et a une structure semblable à celle de la LH. Seul le dimère est capable de stimuler les récepteurs FSH.

Chez les femelles, les taux de LH et de FSH sont cycliques : très faibles en période de repos sexuel ou en dehors de la période ovulatoire, avec un pic de sécrétion en période préovulatoire.

Les gonadotrophines sont utilisées en médecine vétérinaire et humaine, pour induire l'ovulation chez les mammifères femelles. Bien qu'efficaces, ces traitements présentent un risque sanitaire du fait de l'utilisation d'hormones extraites à partir de fluides biologiques (sang, urine) ou de tissus (hypophyses), particulièrement dans le domaine vétérinaire. C'est le cas de la chorionic gonadotropine équine (eCG) extraite à partir de sang de juments gravides, et de la LH et FSH porcines extraites à partir d'hypophyses de porc. Dans le domaine vétérinaire, on utilise également une hCG extraite à partir d'urine de femmes enceintes, le Chorulon® (Laboratoire MSD).

Dans le domaine de la clinique humaine, et particulièrement de la Procréation Médicalement Assistée (ou PMA), on utilise des hormones extraites à partir d'urine de femmes ménopausées telles que Fostimon® (Laboratoire Genévrier) qui est une FSH purifiée et Menopur® (Laboratoire Ferring Pharmaceuticals) qui est une hMG (human menoposal gonadotropin), mélange de FSH et de LH et la Gonadotropine Chorionique Endo5000 qui est une hCG purifiée (Laboratoire Schering-Plough). On utilise également des FSH humaines recombinantes, telles que Gonal-F® (Laboratoire Merck Serono) et Puregon® (Laboratoire Merck Shering-Plough); des hCG et LH recombinantes telle que Ovidrel® et Luveris® (Laboratoire Merck Serono).

En outre l'usage répété de ces hormones induit le plus souvent une réaction immunitaire qui vient neutraliser l'effet des hormones conduisant ainsi à une baisse d'efficacité thérapeutique. Cependant, il a également été mis en évidence dans quelques cas que la réaction immunitaire pouvait produire des anticorps capables de potentialiser l'activité de l'hormone lorsqu'elle était co-administrée (Brevet EP 1518863) [1]. Depuis, il a également été mis en évidence trois anticorps monoclonaux anti-LH capables de potentialiser son action ainsi que celle de la FSH pour deux d'entre eux (Demande Internationale WO 2012/066519) [2].

### Description de l'invention

Les Inventeurs ont maintenant obtenu des anticorps monoclonaux produits contre la sous-unité β de la FSH, capables de potentialiser son action ainsi que celle de la LH et de la hCG.

Ces anticorps monoclonaux sont respectivement dénommés CA5 et CH10.

L'hybridome qui a produit l'anticorps CA5 a été déposé conformément au Traité de Budapest, le 03/10/2013 auprès de la CNCM (Collection Nationale de Culture de Microorganismes, Institut Pasteur, 25 rue du Docteur Roux, 75724 Paris Cedex 15, France), sous le numéro CNCM I-4801.

L'hybridome qui a produit l'anticorps CH10 a été déposé conformément au Traité de Budapest, le 03/10/2013 auprès de la CNCM (Collection Nationale de Culture de Microorganismes, Institut Pasteur, 25 rue du Docteur Roux, 75724 Paris Cedex 15, France), sous le numéro CNCM I-4802.

Les séquences nucléotidiques des régions variables des chaines lourdes et légères des anticorps CA5 et CH10 ont été déterminées, les séquences peptidiques correspondantes déduites. Elles sont présentées respectivement dans les tableaux 1 et 2 ci-dessous.

**Tableau 1**

| Anticorps monoclonal CA5 | |
|---|---|
| Chaîne lourde (VH) | |
| Séquence nucléotidique (SEQ ID NO : 1) | |
| Séquence peptidique (SEQ ID NO : 2) | |

| Chaîne légère (VL) | |
|---|---|
| Séquence nucléotidique (SEQ ID NO : 3) | |
| Séquence peptidique (SEQ ID NO : 4) | |

**Tableau 2**

| Anticorps monoclonal CH10 | |
|---|---|
| Chaîne lourde (VH) | |
| Séquence nucléotidique (SEQ ID NO : 5) | |
| Séquence peptidique SEQ ID NO : 6) | |

| Chaîne légère (VL) | |
|---|---|
| Séquence nucléotidique (SEQ ID NO : 7) | |
| | |
| Séquence peptidique (SEQ ID NO : 8) | |

Les séquences codant pour les CDRs (régions déterminant la complémentarité) ont été déterminées à partir des séquences des régions variables des chaînes lourdes (VH-CDR) et légères (VL-CDR) des anticorps CA5 et CH10 ci-dessus. Les séquences peptidiques correspondantes ont été déduites, et sont présentées respectivement dans les tableaux 3 et 4 ci-dessous.

**Tableau 3**

| Anticorps monoclonal CA5 | |
|---|---|
| VH-CDR1 (SEQ ID NO : 9) | GFTFSDFY |
| VH-CDR2 (SEQ ID NO : 10) | SRNKAKDYTT |
| VH-CDR3 (SEQ ID NO : 11) | ARDARFAY |
| VL-CDR1 (SEQ ID NO : 12) | QSLLYSSNQKNY |
| VL-CDR2 | WAS |
| VL-CDR3 (SEQ ID NO : 13) | QQYYSYPRT |

**Tableau 4**

| Anticorps monoclonal CH10 | |
|---|---|
| VH-CDR1 (SEQ ID NO : 14) | GFTFNTYA |
| VH-CDR2 (SEQ ID NO : 15) | IRSKSNNYAT |
| VH-CDR3 (SEQ ID NO : 16) | VRQDYYGSSYFDY |
| VL-CDR1 (SEQ ID NO : 17) | QSISDY |
| VL-CDR2 | YAS |
| VL-CDR3 (SEQ ID NO : 18) | QNGHSFPYT |

Un ligand de l'hormone folliculo-stimulante (FSH) potentialisant la bioactivité de la FSH, de l'hormone lutéinisante (LH) et de la gonadotropine chorionique (CG), caractérisé en ce qu'il comprend le paratope d'un anticorps anti-sous-unité β de la FSH, est décrit.

On entend par « anticorps anti-sous-unité β de la FSH » au sens de la présente invention, tout anticorps obtenu par immunisation d'un animal à partir de premières injections de FSH suivies de plusieurs rappels avec injection de la sous-unité β de la FSH. Les injections peuvent être réalisées à partir de FSH de différents mammifères, par exemple de FSH ovine, humaine, bovine, caprine ou porcine, équine, canine, murine etc... et des sous-unités β de la FSH d'origine homologue ou hétérologue. Ainsi les anticorps monoclonaux CA5 et CH10 ont été obtenus suite à une immunisation à partir de FSH ovine et de sous-unité β de la FSH ovine.

La présente invention a donc pour objet un ligand de l'hormone folliculo-stimulante (FSH) potentialisant la bioactivité de la FSH, de l'hormone lutéinisante (LH) et de la gonadotropine chorionique (CG), caractérisé en ce que le ligand est un anticorps ou un fragment de celui-ci et en ce que :
le domaine variable de la chaine lourde contient les CDRs suivants :
   - VH-CDR1, défini par la séquence GFTFSDFY (SEQ ID NO : 9) ;
   - VH-CDR2, défini par la séquence SRNKAKDYTT (SEQ ID NO : 10) ;
   - VH-CDR3, défini par la séquence ARDARFAY (SEQ ID NO : 11) ; et
le domaine variable de la chaine légère contient les CDRs suivants :
   - VL-CDR1, défini par la séquence QSLLYSSNQKNY (SEQ ID NO : 12) ;
   - VL-CDR2, défini par la séquence WAS ;
   - VL-CDR3, défini par la séquence QQYYSYPRT (SEQ ID NO : 13).

La présente invention a donc pour objet un ligand de l'hormone folliculo-stimulante (FSH) potentialisant la bioactivité de la FSH, de l'hormone lutéinisante (LH) et de la gonadotropine chorionique (CG), caractérisé en ce que le ligand est un anticorps ou un fragment de celui-ci et en ce que :
le domaine variable de la chaine lourde contient les CDRs suivants :
   - VH-CDR1, défini par la séquence GFTFNTYA (SEQ ID NO : 14) ;
   - VH-CDR2, défini par la séquence IRSKSNNYAT (SEQ ID NO : 15) ;
   - VH-CDR3, défini par la séquence VRQDYYGSSYFDY (SEQ ID NO : 16) ; et
le domaine variable de la chaine légère contient les CDRs suivants :
   - VL-CDR1, défini par la séquence QSISDY (SEQ ID NO : 17) ;
   - VL-CDR2, défini par la séquence YAS ;
   - VL-CDR3, défini par la séquence QNGHSFPYT (SEQ ID NO : 18).

On entend par « CDR » au sens de la présente invention, les trois régions hypervariables des régions variables des chaînes lourdes et légères d'un anticorps qui constituent les éléments du paratope et permettent de déterminer la complémentarité de l'anticorps avec l'épitope de l'antigène. Ces trois régions hypervariables sont encadrées par quatre régions constantes qui constituent la « charpente » (FR ou framework regions) et donnent une configuration stable au domaine variable.

En particulier, la présente invention a donc pour objet un ligand selon la revendication 2 ou 4.

Un ligand selon la présente invention est par exemple :
- l'anticorps monoclonal CA5 produit par l'hybridome CNCM I-4801;
- l'anticorps monoclonal CH10 produit par l'hybridome CNCM I-4802 ;
- un fragment VH ou VL d'un anticorps ci-dessus utilisé seul ou en mélange ;
- un fragment Fab, Fab', F(ab')2, Fv, dsFv ou scFv, d'un anticorps ci-dessus. De préférence, il s'agit d'un fragment Fab ou d'un fragment scFv, par exemple d'un fragment scFv de séquence peptidique SEQ ID NO : 20 ou SEQ ID NO : 22 ;
- une forme bi-, tri- ou tétravalente (diabodies, triabodies, tétrabodies) de deux, trois ou quatre fragments de scFv, respectivement.
- Un anticorps recombinant comprenant le paratope d'un anticorps ci-dessus et dont les régions constantes ont été modifiées de sorte à minimiser l'immunogénicité vis-à-vis de l'animal ou de l'homme auquel il est destiné, est décrit. Par exemple, il s'agit d'un anticorps chimérique (humanisé, ovinisé, caprinisé, bovinisé, porcinisé etc...) ou entièrement humanisé, ovinisé, caprinisé, bovinisé, porcinisé

A titre d'exemple non limitatif, les séquences nucléotidiques de scFv dérivés des anticorps CA5 et CH10 ont été déterminées, les séquences peptidiques correspondantes déduites, et sont présentées respectivement dans les tableaux 5 et 6 ci-dessous.

**Tableau 5**

| scFv CA5 | |
|---|---|
| Séquence nucléotidique (SEQ ID NO : 19) | |
| | |
| Séquence peptidique (SEQ ID NO 20) | |

**Tableau 6**

| scFv CH10 | |
|---|---|
| Séquence nucléotidique (SEQ ID NO : 21) | |
| Séquence peptidique (SEQ ID NO 22) | |
| | |

Une séquence nucléotidique codant pour un ligand selon l'invention, est décrite.

Un vecteur recombinant, en particulier un vecteur d'expression, comprenant une séquence nucléotidique telle que ci-dessus, est décrit.

Une cellule hôte comprenant une séquence nucléotidique telle que décrite ci-dessus ou un vecteur recombinant tel que décrit ci-dessus, est décrite. Par exemple, il s'agit des hybridomes CNCM I-4801 et CNCM I-4802 ou d'une cellule transformée par une séquence nucléotidique ou un vecteur recombinant selon l'invention.

Un procédé de production d'un ligand selon l'invention, caractérisé en ce qu'il comprend la mise en culture dans un milieu approprié de cellules hôte telles que décrites ci-dessus, et la récupération dudit ligand à partir de ladite culture, est décrit.

Les Inventeurs ont mis en évidence que l'anticorps CA5 potentialise fortement la FSH porcine, ovine, bovine et de façon moindre bien que significativement la FSH humaine. En outre, les Inventeurs ont mis en évidence que les scFv dérivés des anticorps CA5 et CH10 possèdent les mêmes propriétés de liaison et de potentialisation que les anticorps dont ils dérivent.

La présente invention a également pour objet un complexe formé d'un ligand et d'une gonadotrophine, ou d'un peptide actif de celle-ci, capable de se lier audit ligand et dont l'activité est potentialisée par ledit ligand. Par exemple, il s'agit du complexe d'un ligand avec la LH, avec l'hormone gonadotropine chorionique (CG), ou avec la FSH extraites de tissus ou de fluides biologiques, ou recombinantes, ou d'un peptide actif de celles-ci capable de se lier audit ligand et dont l'activité est potentialisée par ledit ligand.

La présente invention a également pour objet un ligand ou complexe selon l'invention pour utilisation comme médicament, en particulier pour potentialiser la bioactivité de la FSH, de la LH, et de la gonadotropine chorionique (CG) pour induire une ovulation voire une polyovulation chez un mammifère femelle ou pour réduire les problèmes d'infertilité ou d'hypofertilité hormono dépendants chez un mammifère mâle ou femelle. Ledit médicament permet également d'augmenter le taux de progestérone endogène circulant sécrété par un ou plusieurs corps jaunes chez un mammifère femelle, favorisant ainsi le développement embryonnaire précoce et diminuant le risque d'avortement.

Un procédé de production carnée, où ledit procédé comprend l'administration de ligand et/ou de complexe de l'invention à un mammifère femelle animal non humain, est décrit.

La présente invention a également pour objet un ligand et/ou complexe de l'invention pour une utilisation dans le traitement de l'infertilité ou de l'hypofertilité hormono dépendante chez un mammifère. Dans le cas d'un mammifère femelle souffrant d'infertilité ou d'hypofertilité, l'administration du ligand ou complexe de l'invention va permettre de stimuler une procréation naturelle, médicalement assistée ou artificielle. Il est à noter que l'administration du ligand ou complexe de l'invention à un mammifère femelle sain va également permettre de déclencher l'ovulation dans le cadre d'une procréation naturelle ou artificielle.

On entend par « infertilité/hypofertilité hormono dépendante » au sens de la présente invention, une infertilité/hypofertilité due à une insuffisance hormonale par exemple de faibles concentrations circulantes de FSH et LH ou une absence de ces hormones résultant par exemple d'une cause externe (par exemple les pesticides) ou interne (par exemple, insuffisance hypophysaire ou hypothalamique ou d'un problème de réceptivité des gonades à la LH et/ou la FSH due à une anomalie des récepteurs ou des gonadotropines LH, FSH, CG par exemple une mutation ou un polymorphisme des récepteurs).

Les ligands et complexes de l'invention peuvent être utilisés chez l'homme ou l'animal, en particulier les ovins, bovins, caprins, équins, porcins, murins, canins, camelins etc...

Les ligands, les hormones ou les complexes selon l'invention peuvent être administrés soit séparément, soit séquentiellement, soit conjointement, par injection, par exemple intramusculaire, intra veineuse, intrapéritonéale, sous-cutanée, transcutanée, intradermique, intraorbitaire, intraoculaire, ophtalmique, ou par voie transoculaire, sans altérer leur effet potentialisant.

La présente invention a également pour objet une composition pharmaceutique comprenant un ligand ou complexe de l'invention et un véhicule pharmaceutiquement acceptable. Ladite composition pharmaceutique peut comprendre en outre une FSH et/ou une LH et/ou une hormone gonadotropine chorionique (CG).

D'autres avantages pourront encore apparaître à l'homme du métier à la lecture des exemples ci-dessous, illustrés par les figures annexées, donnés à titre illustratif.

### Brève description des figures

- La figure 1 illustre l'effet potentialisant *in vitro* des anticorps monoclonaux CA5 (A) et CH10 (B) sur la bioactivité de la FSH ovine (oFSH), sur des cellules de granulosa bovines.
- La figure 2 représente l'effet potentialisant *in vitro* des anticorps monoclonaux CA5 (A) et CH10 (B) sur la bioactivité de la FSH ovine (oFSH) sur une lignée cellulaire HEK 293 transfectée de façon stable avec le récepteur FSH humain.
- La figure 3 représente l'effet potentialisant *in vitro* de l'anticorps monoclonal CA5 sur la bioactivité de la FSH ovine (oFSH) sur une lignée cellulaire HEK 293 transfectée de façon stable avec le récepteur FSH humain et le vecteur Glosensor®.
- La figure 4 représente l'effet potentialisant de l'anticorps monoclonal CA5 sur la bioactivité de la FSH porcine (pFSH) sur une lignée cellulaire HEK 293 transfectée de façon stable avec le récepteur FSH humain et le vecteur Glosensor®.
- La figure 5 représente l'effet potentialisant *in vitro* de l'anticorps monoclonal CA5 (A à E) et du scFv CA5 (F) sur la bioactivité de la FSH humaine (hFSH) sur une lignée cellulaire HEK 293 transfectée de façon stable avec le récepteur FSH humain et le vecteur Glosensor®.
- La figure 6 représente l'effet potentialisant *in vitro* de l'anticorps monoclonal CH10 sur la bioactivité de la FSH ovine (oFSH) sur une lignée cellulaire HEK 293 transfectée de façon stable avec le récepteur FSH humain et le vecteur Glosensor®.
- La figure 7 représente l'effet potentialisant de l'anticorps monoclonal CH10 sur la bioactivité de la FSH humaine (hFSH) *in vitro* sur une lignée cellulaire HEK 293 transfectée de façon stable avec le récepteur FSH humain et le vecteur Glosensor®.
- La figure 8 représente l'effet potentialisant *in vivo* de l'anticorps monoclonal CA5 sur la bioactivité de la FSH ovine (oFSH) (A) et sur la bioactivité des FSH humaines (hFSH) Gonal-F®, Puregon® et Fostimon® (B) chez le rat femelle.
- La figure 9 représente l'effet potentialisant *in vivo* de l'anticorps monoclonal CA5 sur la bioactivité de la FSH humaine (hFSH) Gonal-F® (A) et l'effet potentialisant du scFv CA5 sur la bioactivité des FSH humaines (hFSH) Gonal-F® (A), Puregon® et Fostimon® (B) chez le rat femelle.
- La figure 10 représente l'effet potentialisant *in vivo* de l'anticorps monoclonal CH10 sur la bioactivité de la FSH ovine (oFSH) (A) et sur la bioactivité de la FSH humaine (hFSH) Gonal-F® (B) chez le rat femelle.
- La figure 11 représente l'effet potentialisant *in vivo* de l'anticorps monoclonal CH10 sur la bioactivité des FSH humaines (hFSH) Puregon® et Fostimon® (A) et l'effet potentialisant du scFv CH10 sur la bioactivité de la FSH humaine (hFSH) Gonal-F® (B) chez le rat femelle.
- La figure 12 représente l'effet potentialisant *in vivo* de l'anticorps monoclonal CA5 sur la bioactivité des choriogonadotropines humaines (hCG) Chorulon® et Endo 5000 chez le rat mâle.
- La figure 13 représente l'effet potentialisant *in vivo* de l'anticorps monoclonal CH10 sur la bioactivité des choriogonadotropines humaines (hCG) Chorulon® et Endo 5000® chez le rat mâle.
- la figure 14 représente l'effet potentialisant *in vivo* de l'anticorps monoclonal CA5 sur la bioactivité des gonadotropines endogènes chez la brebis en fin de saison sexuelle.
- La figure 15 représente l'effet potentialisant *in vivo* de l'anticorps monoclonal CA5 sur la bioactivité des gonadotropines endogènes chez la brebis en période de saison sexuelle.
- La figure 16 représente l'effet potentialisant *in vivo* de l'anticorps monoclonal CA5 injecté seul sur les gonadotropines endogènes chez la génisse : effet sur la sécrétion d'oestradiol.
- La figure 17 représente l'effet potentialisant *in vivo* de l'anticorps monoclonal CA5 injecté seul sur les gonadotropines endogènes chez la génisse : effet sur les caractéristiques du pic préovulatoire de LH.
- La figure 18 représente l'effet potentialisant *in vivo* de l'anticorps monoclonal CA5 injecté seul sur les gonadotropines endogènes chez la génisse : effet sur la sécrétion de progestérone.
- la figure 19 représente l'effet potentialisant *in vivo* de l'anticorps monoclonal CA5 injecté seul après 25 UI de hFSH, sur la croissance folliculaire chez la guenon.
- La figure 20 représente l'épitope conformationnel du ligand CA5 sur les hormones hFSH, hCG, hLH, oLH, pLH, oFSH, pFSH et sur le récepteur FSH humain.
- La figure 21 représente l'épitope conformationnel du ligand CH10, sur les hormones hFSH, hCG, hLH, oLH, pLH, oFSH, pFSH et sur le récepteur FSH humain.
- La figure 22 représente l'effet potentialisant *in vitro* de différents fragments de l'anticorps monoclonal CA5 sur la bioactivité de la FSH ovine et de la FSH humaine.
- La figure 23 représente l'effet potentialisant *in vivo* chez la ratte de différents fragments de l'anticorps monoclonal CA5 sur la bioactivité de la FSH humaine.

### EXEMPLES

### EXEMPLE 1 : OBTENTION DES LIGANDS DE L'INVENTION, ET LEUR CARACTERISATION

### 1/ Stratégie d'immunisation des souris

Les injections ont toutes été réalisées par voie intra-péritonéale sur des souris (Balb/C). Cinq souris ont été utilisées pour chaque stratégie d'immunisation.

### Stratégie d'immunisation des souris pour l'anticorps CA5 et l'anticorps CH10

Une première injection (J0) a été réalisée avec 100µg de FSH ovine purifiée avec adjuvant de Freund complet. Plusieurs injections de rappel ont été ensuite réalisées selon la séquence suivante :
- J21 et J44 : 100µg de FSH ovine purifiée avec adjuvant de Freund incomplet ;
- J134 et J204 : 50µg de sous-unité béta de FSH ovine avec adjuvant de Freund incomplet ;
- J217, J218 et J219 : 30µg de sous-unité béta de FSH ovine sans adjuvant ;
- J220 : fusion.

### 2/ Isotypage

L'isotypage des anticorps CA5 et CH10 a été réalisé avec le kit d'isotypage FastElysa commercialisé par RD Biotech (référence RDB 3255) en suivant les recommandations du fabriquant.

L'anticorps CA5 est une immunoglobine de classe IgG2a et d'isotype Kappa. Les valeurs des densités optiques (DO) obtenues ont été 0,335 et 0,371 respectivement.

L'anticorps CH10 est une immunoglobine de classe IgM et d'isotype Kappa. Les valeurs des densités optiques (DO) obtenues ont été 0,2 et 0,124 respectivement.

### 3/ Séquençage

Les séquences nucléotidiques de la partie variable des chaînes lourdes (VH) et légères (VL) des anticorps CA5 et CH10 sécrétés par les hybridomes CNCM I-4801 et CNCM I-4802 respectivement, ont été déterminées à partir de leur ARN messager (ARNm) selon le protocole ci-après.

Les ARN ont été extraits des cellules à l'aide du kit Nucleospin® RNA (Macherey Nagel, Allemagne) en suivant les recommandations du fabriquant. Les concentrations en ARN purifiés ont été estimées par mesure de l'absorbance (A) à 260 nm et leur qualité par le rapport A260nm/280nm et visuellement après migration électrophorétique sur gel d'agarose.

Les ADN complémentaires des ARNm ont alors été synthétisés à l'aide d'un oligo-dT₁₈ comme amorce par réaction de rétrotranscription avec l'enzyme M-MLV (Réf. M1701,Promega, USA) en suivant les recommandations du fabriquant.

La synthèse du second brin d'ADN a été réalisée par une réaction de polymérisation en chaîne (PCR) selon le protocole suivant : à 4 µl de la réaction de rétrotranscription sont ajoutés dans un volume final de 50 µl ; le tampon de réaction (1X final), 200 µM de chaque dNTPs, 300 nM d'amorces sens et antisens, 1,25 U de GoTaq polymérase (Ref M3175, Promega, USA).

Pour l'amplification de la partie variable des chaînes légères, 9 couples d'amorces différents ont été utilisés (MKRev2 à 8 + MKC5For) et 3 couples différents pour celles des chaînes lourdes (CA5 : VHRev1 + VHFor, CH10 : VHRev1 + MµCFor).

**Tableau 7 : Séquences nucléotidiques des amorces utilisées pour séquencer les chaînes lourdes (VH) et légères (VL) de l'anticorps CA5.**

| Anticorps CA5 | | |
|---|---|---|
| Chaîne lourde (VH) | | |
| **Nom** | **Séquence 5'-3'** | **SEQ ID NO** |
| **VHRev1** | | SEQ ID NO : 23 |
| **VHFor** | CAGGGGCCAGTGGATAGAC | SEQ ID NO : 24 |

| Chaîne légère (VL) | | |
|---|---|---|
| **MKRev5** | GACATTGTGATGACCCAGTCT | SEQ ID NO : 25 |
| **MKC5For** | GGATACAGTTGGTGCAGCATC | SEQ ID NO : 26 |

**Tableau 8: Séquences nucléotidiques des amorces utilisées pour séquencer la partie 5' des chaînes lourdes (VH) et légères (VL) de l'anticorps CA5.**

| Anticorps CA5 | | |
|---|---|---|
| Chaîne lourde (VH) | | |
| **Nom** | **Séquence 5'-3'** | **SEQ ID NO** |
| **CA5VH_Fw** | CACTTTTACATGGTATCCAGTG | SEQ ID NO : 27 |
| **CA5VH_Rev** | GTTTCTACTTGCAGCAATCCACT | SEQ ID NO : 28 |

| Chaîne légère (VL) | | |
|---|---|---|
| **CA5VL_Fw** | GAWTCACAGRCCCAGGTYC | SEQ ID NO : 29 |
| **CA5VL_Rev** | CCCAGTAAATCAGCAGTTTAGGA | SEQ ID NO : 30 |

**Tableau 9 : Séquences nucléotidiques des amorces utilisées pour séquencer les chaînes lourdes (VH) et légères (VL) de l'anticorps CH10.**

| Anticorps CH10 | | |
|---|---|---|
| Chaîne lourde (VH) | | |
| **Nom** | **Séquence 5'-3'** | **SEQ ID NO** |
| **VHRev1** | | SEQ ID NO : 23 |
| **MµCFor** | GGGGAAGACATTTGGGAAGG | SEQ ID NO : 31 |

| Chaîne légère (VL) | | |
|---|---|---|
| **MKRev2** | GATATTGTGATGACGCAGGCT | SEQ ID NO : 32 |
| **MKRev3** | GATATTGTGATAACCCAG | SEQ ID NO : 33 |
| **MKRev4** | GACATTGTGCTGACCCAATCT | SEQ ID NO : 34 |
| **MKRev6** | GATATTGTGCTAACTCAGTCT | SEQ ID NO : 35 |
| **MKRev8** | GACATCCAGCTGACTCAGTCT | SEQ ID NO : 36 |
| **MKC5For** | GGATACAGTTGGTGCAGCATC | SEQ ID NO : 37 |

**Tableau 10: Séquences nucléotidiques des amorces utilisées pour séquencer la partie 5' des chaînes lourdes (VH) et légères (VL) de l'anticorps CH10.**

| Anticorps CH10 | | |
|---|---|---|
| Chaîne lourde (VH) | | |
| **Nom** | **Séquence 5'-3'** | **SEQ ID NO** |
| **CH10VH_Fw** | ATGGTGTTGGGGCTGAAGTG | SEQ ID NO : 38 |
| **CH10VH_Rev** | CAGTTCATGGCGTAGGTATTGA | SEQ ID NO : 39 |

| Chaîne légère (VL) | | |
|---|---|---|
| **CH10VL_ Fw** | TTCTGGAYTTCAGCCTCCAG | SEQ ID NO : 40 |
| **CH10VL_ Rev** | GATTGGGAAGCATATTTGATGAG | SEQ ID NO : 41 |

Le programme de PCR utilisé est composé d'une dénaturation initiale de 2 min à 95°C suivie de 30 cycles de dénaturation 30 sec à 95°C, hybridation 30 sec à 47°C et amplification 1 min à 72°C et enfin d'une amplification finale de 5 min à 72°C. Les produits de PCR obtenus ont été dessalés avec le kit QIAquick®Gel extraction kit (Ref 28704, Qiagen GmbH, Allemagne) puis ligaturés avec le plasmide pGEMT easy vector (Ref A1360, Promega, USA) pour être transformés en bactéries. L'ADN plasmidique extrait de différents clones bactériens a été envoyé pour analyse par séquençage (Macrogen Europe, Pays-Bas).

Les séquences nucléotidiques 5' terminales des VH et VL des 2 anticorps ont par la suite été déterminées grâce à la conception d'amorces spécifiques ancrées dans les séquences leader des ADNc (amorce Fw). Ces amorces ont été conçues suite à l'identification d'homologie par alignement entre les séquences VL et VH obtenues précédemment et la base de données du logiciel IMGT/V-QUEST (Brochet et al., Nucl. Acids Res., 36: W503-508, 2008; Giudicelli et al.,, Cold Spring Harb Protoc., 2011(6) : 695-715, 2011) [3, 4] et à l'extraction des séquences leader d'intérêt de IMGT/GENE-DB (Giudicelli et al., Nucl. Acids Res., 33 : D256-261, 2005) [5]. Les amorces anti-sens (Rev) ont été conçues dans les séquences VH et VL respectives précédemment déterminées de chacun des anticorps. Le protocole utilisé pour obtenir la partie 5'est le même que celui décrit au paragraphe précédent.

Les séquences nucléotidiques consensus ont été déduites de l'alignement des séquences en utilisant le logiciel MultAlin (Corpet, Nucl. Acids Res., 16(22) : 10881-10890, 1988) [6]. La transcription en séquences polypeptidiques et l'annotation des CDRs ont été réalisées à l'aide de logiciel IMGT/V-QUEST. Les résultats sont présentés dans les tableaux 11 à 14.

**Tableau 11 : Séquences nucléotidiques et peptidiques des parties variables lourdes (VH) et légères (VL) de l'anticorps CA5.**

| Anticorps (CA5) | |
|---|---|
| Chaîne lourde (VH) | |
| Séquence nucléotidique SEQ ID NO : 1 | |
| Séquence peptidique SEQ ID NO : 2 | |

| Chaîne légère (VL) | |
|---|---|
| Séquence nucléotidique SEQ ID NO : 3 | |
| Séquence peptidique SEQ ID NO : 4 | |

**Tableau 12 : Séquences nucléotidiques et peptidiques des parties variables lourdes (VH) et légères (VL) de l'anticorps CH10.**

| Anticorps CH10 | |
|---|---|
| Chaîne lourde (VH) | |
| Séquence nucléotidique SEQ ID NO : 5 | |
| Séquence peptidique SEQ ID NO : 6 | |

| Chaîne légère (VL) | |
|---|---|
| Séquence nucléotidique SEQ ID NO : 7 | |
| Séquence peptidique SEQ ID NO : 8 | |

**Tableau 13 : CDR des parties variables lourdes (VH) et légères (VL) de l'anticorps CA5**

| | |
|---|---|
| VH-CDR1 (SEQ ID NO: 9) | GFTFSDFY |
| VH-CDR2 (SEQ ID NO: 10) | SRNKAKDYTT |
| VH-CDR3 (SEQ ID NO: 11) | ARDARFAY |
| VL-CDR1 (SEQ ID NO: 12) | QSLLYSSNQKNY |
| VL-CDR2 | WAS |
| VL-CDR3 (SEQ ID NO: 13) | QQYYSYPRT |

**Tableau 14 : CDR des parties variables lourdes (VH) et légères (VL) de l'anticorps CH10**

| | |
|---|---|
| VH-CDR1 (SEQ ID NO: 14) | GFTFNTYA |
| VH-CDR2 (SEQ ID NO: 15) | IRSKSNNYAT |
| VH-CDR3 (SEQ ID NO: 16) | VRQDYYGSSYFDY |
| VL-CDR1 (SEQ ID NO: 17) | QSISDY |
| VL-CDR2 | YAS |
| VL-CDR3 (SEQ ID NO: 18) | QNGHSFPYT |

### 4/ Construction, production et caractérisation des scFv

### a/ Construction des fragments d'anticorps scFv

Les gènes de synthèse des fragments variables simple chaîne (scFv) dérivés des anticorps CA5 et CH10 ont été synthétisés par ATG:Biosynthetics GmbH (Allemagne).

Chaque séquence a été conçue de la fusion des parties variables lourdes et légères (SEQ ID NO : 1/ SEQ ID N0: 3 pour CA5; SEQ ID N0: 5/ SEQ ID N0 : 7 pour CH10) liées par une séquence codant pour le peptide (Gly₄Ser)₃ assurant la fonctionnalité de la protéine et terminées par une séquence codant le peptide His₆ (peptide HIS-tag) qui autorisera la purification des scFvs. Afin de permettre leur insertion dans le plasmide d'expression, les séquences ont été flanquées par les sites enzymatiques de restriction Pstl et SalI. Une séquence additionnelle a été ajoutée entre l'extrémité 3' du VL et le site Sall autorisant la suppression du peptide His₆ si désiré. Les codons ont été optimisés pour l'expression en *E. coli.* Une représentation schématique de la construction des gènes de synthèse des scFvs est détaillée ci-dessous :

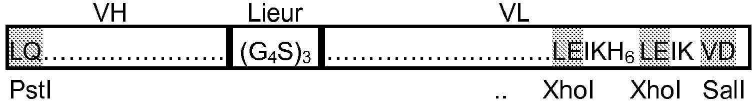

Les fragments d'anticorps ont été insérés entre les sites enzymatiques Pstl et Xhol du plasmide d'expression pSW1 (ATG:Biosynthetics GmbH, Allemagne) selon E. S. Ward et collaborateurs (Ward et al., Nature, 341: 544-546, 1989) [7] qui contient sous le contrôle d'un promoteur inductible LacZ, une séquence signal PelB qui fusionnée en phase de lecture avec le gène du fragment d'anticorps recombinant, permet l'adressage de la protéine synthétisée vers le périplasme bactérien. Dans le périplasme, cette séquence signal est éliminée par une peptidase.

Après contrôle par séquençage de la qualité des constructions, les plasmides pSW1-CA5 et pSW1-CH10 ont été transformés par choc thermique en bactéries HB2151 (T53040, Interchim, France) rendues compétentes (Li et al., Afr. J. Biotechnol., 9(50) : 8549-8554, 2010) [8].

**Tableau 15 : Séquences nucléotidique et peptidique du scFv CA5.**

| scFv CA5 | |
|---|---|
| Séquence nucléotidique SEQ ID NO : 19 | |
| | |
| Séquence peptidique SEQ ID NO : 20 | |

**Tableau 16 : Séquences nucléotidique et peptidique du scFv CH10.**

| scFv CH10 | |
|---|---|
| Séquence nucléotidique SEQ ID NO : 21 | |
| | |
| Séquence peptidique SEQ ID NO : 22 | |

### b/ Production des fragments d'anticorps recombinants

### - Culture bactérienne

Une pré-culture a été réalisée dans 5 ml de milieu 2xYT contenant 50 µg/ml d'ampicilline toute la nuit à 37°C. Le lendemain, 500 µl de cette préculture a été ensemencée dans 500 ml du même milieu et mis à croître à 37°C à 150 RPM jusqu'à obtenir une DO₆₀₀ₙₘ de 1,4. La synthèse du scFv a été induite par l'ajout de 0,1 mM d'IPTG 16 h à 16°C à 150 RPM.

### - Extraction

Le milieu de culture a été centrifugé 30 min à 4500 g à 4°C. La suite de la préparation a été réalisée à 4°C. Pour extraire le périplasme bactérien, le culot a été remis en suspension et incubé dans 10 ml de TES (Tris 0,2 M pH8, EDTA 0,5M, saccharose 0,5 M) pendant 30 min auxquels ont alors été ajoutés 15 ml de TES dilué au ¼ pour être de nouveau incubé 30 min. L'extrait bactérien a été centrifugé 30 min à 10 000g. Le surnageant a été mis à dialyser contre du PBS pendant une nuit. Le surnageant dialysé a été traité immédiatement pour purifier le scFv ou conservé à -20°C jusqu'à utilisation.

La production du scFv dans le périplasme a été analysée par Western blot en utilisant un anticorps anti - His-Tag HRP (Ref R93125 Life technologies, France) selon les conseils d'utilisation du fabricant.

### - Purification

Le périplasme a été centrifugé pendant 20 min à 5 000 g à 4°C. Le surnageant a été incubé avec HIS-Select® Nickel Affinity Gel (Sigma-Aldrich, MO, USA) sous agitation pendant 1h à 4°C. Le gel a été lavé avec un tampon phosphate de sodium 0,05 M, NaCl 0,3 M pH8 puis le même tampon additionné de 20 mM d'imidazole jusqu'à obtenir une DO₂₈₀ₙₘ proche de 0. Le scFv a alors été élué avec un tampon phosphate de sodium 0,05 M, NaCl 0,3 M, 250 mM imidazole pH8. L'éluat a été dialysé contre du PBS toute la nuit. Il est conservé à -20°C.

### - Contrôle qualité

Le scFv purifié a été analysé par électrophorèse sur gel de polyacrylamide à 15% après coloration au bleu de Coomassie et par chromatographie d'exclusion sur colonne Sephadex™ 75 10/300 GL (Ref 17-5174-01 GE Healthcare, Allemagne).

### 5/ Spécificité

La spécificité des anticorps et de leur scFv a été étudiée par technique ELISA. Chaque hormone évaluée a été préparée à la concentration de 10 µg/ml dans du tampon carbonate de sodium 0,1M pH 9,6 et distribuée à raison de 100 µl par puits sur une plaque ELISA. Le temps d'adsorption a été de 18 heures à +4°C. Après cinq lavages, les puits ont été traités avec 100 µl de PBS additionné de Tween 0,1% et de BSA 1% pendant 45 min à 37°C, puis chaque anticorps ou scFv a été distribué à raison de 100 µl/puits et incubé 1 heure à 37°C. Sur chaque hormone évaluée, les anticorps et les scFv ont été distribués à différentes concentrations selon une gamme de 10 à 250 µg/ml pour les anticorps et de 10 à 150 ou 200 µg/ml pour les scFv.

Après cinq lavages, un anticorps secondaire couplé à la peroxydase (HRP) a été distribué à raison de 100 µl/puits et incubé 1 heure à 37°C. Selon l'isotype de l'anticorps monoclonal étudié, l'anticorps secondaire a été un anti-IgG1 HRP (Réf. 115-035-205, Jackson ImmunoResearch Laboratories Inc), un anti-IgG2a HRP (Réf. 115-035-206, Jackson Laboratories) ou un anti-IgM HRP (Réf. 115-035-075, Jackson Laboratories). Pour les scFv, un anti-His Tag HRP (Réf. R93125 Life technologies, France) a été utilisé. Après cinq lavages, l'activité enzymatique a été révélée avec du TMB distribué à raison de 100 µl/puits. Le temps de révélation a été de 5 à 30 min à température ambiante selon la vitesse de la réaction. Après arrêt de la réaction avec H₂SO₄ 1M (50 µl/puits) l'intensité de la réaction colorée (Densité Optique) a été mesurée à l'aide d'un spectrophotomètre pour plaques ELISA.

Pour les anticorps CA5 et CH10 et leur scFv, le pourcentage de réaction croisée a été calculé par rapport aux valeurs obtenues avec la FSH ovine (oFSH) considérée comme la valeur 100% de référence. Le pourcentage de réaction croisée a été calculé de façon classique en comparant les courbes dose-réponse obtenues avec la gamme de concentration de l'anticorps ou du scFv. A partir de la courbe obtenue avec l'hormone référence :
- soit A la concentration donnant 50% de la densité optique maximale (ED 50). A partir de la courbe obtenue avec une autre hormone,
- soit B la concentration correspondante à la même valeur de densité optique que celle utilisée pour définir A.
Le pourcentage de réaction croisée est égal à A divisé par B et multiplié par 100 : [(A/B) X 100].

### - Spécificité de l'anticorps CA5 et de son scFv

Le tableau 17 illustre les pourcentages de réactions croisées de l'anticorps CA5 avec les sous-unités (s.u.) α et β de la FSH ovine et la sous-unité β de la FSH humaine :

**Tableau 17**

| CA5 | oFSH | s.u. α oFSH | s.u. β oFSH | s.u. β hFSH |
|---|---|---|---|---|
| Réaction croisée | 100% | 6% | 80% | 50% |

L'anticorps CA5 reconnaît très peu la sous-unité α ovine mais fortement la sous-unité β de la FSH ovine (80%) ; il croise également avec la sous-unité β de la FSH humaine, moins fortement (50%). Sa spécificité est anti-sous unité β FSH.

Le tableau 18 illustre les pourcentages de réactions croisées de CA5 et du scFv CA5 avec la FSH porcine (pFSH) et différentes FSH humaines :

**Tableau 18**

| | oFSH | pFSH | hFSH (Gonal F) | hFSH (Puregon) | hFSH (Fostimon) | hMG (Menopur) |
|---|---|---|---|---|---|---|
| CA5 | 100% | 134% | 128% | 70% | 76% | 61% |
| scFv CA5 | 100% | 61% | ND | ND | 10% | 10% |

L'anticorps CA5 présente une forte reconnaissance de la FSH porcine et de la FSH humaine Gonal-F. Il croise également de façon significative avec les autres FSH humaines entre 61 et 76%. L'anticorps CA5 reconnaît mieux les FSH testées sous leur forme dimérique ce qui tend à indiquer une spécificité contre un épitope conformationnel.

Le scFv CA5 reconnaît de façon significative la pFSH (61%) et plus faiblement une hFSH (Fostimon) et la hMG (Menopur). La réaction croisée sur les deux autres FSH humaines n'a pu être mesurée (ND) en raison d'une liaison trop faible. La liaison du scFv CA5, tout comme celle de l'anticorps entier, semble donc être dépendante de la conformation de l'hormone, probablement altérée lors de l'adsorption sur le plastique de la plaque ELISA.

La spécificité du scFv CA5 a été évaluée vis-à-vis des LH porcine (pLH), ovine (oLH), bovine (bLH), de la eCG et des hCG Chorulon et Endo 5000. Les résultats sont illustrés dans le tableau 19 :

**Tableau 19**

| | oLH | pLH | bLH | eCG | Chorulon | Endo 5000 |
|---|---|---|---|---|---|---|
| scFv CA5 | 25% | 29% | 33% | ND | 10% | ND |

La liaison du scFv CA5 est significative vis-à-vis des LH animales avec une réaction croisée entre 35 et 40%. A l'inverse, seule la hCG Chorulon est faiblement reconnue (10%). La trop faible liaison sur les deux autres hCG adsorbées n'a pas permis de quantifier une réaction croisée. Ces résultats renforcent l'hypothèse d'une spécificité envers un épitope conformationnel étant donné les effets biologiques de CA5 et de son scFv obtenus *in vitro* et *in vivo* sur l'activité des hCG (voir résultats dans Exemples 2 et 3).

Cette hypothèse est renforcée par les résultats obtenus par western blot en incubant l'anticorps CA5 sur de la oFSH migrée sur gel de polyacrilamide 5% en conditions dénaturantes ou non-dénaturantes. Seule la bande de β oFSH a été reconnue en conditions non-dénaturantes et a donné un signal important. Aucun signal n'a été observé sur la oFSH migrée en conditions dénaturantes.

Une estimation de la constante de dissociation Kd du scFv, vis-à-vis des différentes FSH, LH et CG étudiées, a été calculée sur GraphPad Prism (GraphPad Software Inc., San Diego, CA, USA, version 5) en utilisant la fonction *"One site* - *Specific binding"* dans un modèle de liaison à saturation (*"saturation binding experiment model",* GraphPad PRISM software). Les différentes valeurs obtenues sont indiquées dans les tableaux 20 et 21.

**Tableau 20**

| scFvCA5 | oFSH | pFSH | hFSH Gonal-F | hFSH Puregon | hFSH Fostimon | hMG Menopur |
|---|---|---|---|---|---|---|
| Kd (10⁻⁶M) | 0,54 | 1,24 | 1,43 | 2,67 | 2,03 | 2,41 |

**Tableau 21**

| scFv CA5 | oLH | pLH | bLH | eCG | hCG Chorulon | hCG Endo 5000 |
|---|---|---|---|---|---|---|
| Kd (10⁻⁶M) | 1,95 | 2,47 | 2,43 | 4,07 | 3,80 | 3,14 |

La comparaison des constantes de dissociation Kd ainsi estimées indique une plus forte affinité du scFv CA5 pour les FSH ovine et porcine avec une valeur de 0,54 et 1,24 µM respectivement. A l'exception de la FSH humaine recombinante Gonal F (Kd 1,43 µM), le scFv CA5 présente une affinité moins élevée pour les FSH humaines (Kd de 2,03 à 2,67µM). Comparativement à oFSH et pFSH, le scFv CA5 présente une affinité moyenne vis-à-vis des LH ovine et porcine (Kd de 1,95 et 2,47µM respectivement). Les Kd estimés vis-à-vis des hCG et de la eCG (Kd de 3,14 à 4,07 µM) indiquent une affinité plus faible du scFv CA5 pour ces hormones.

### - Spécificité de l'anticorps CH10 et de son scFv

Le tableau 22 illustre les pourcentages de réactions croisées de l'anticorps CH10 avec les sous-unités (s.u.) α et β de la FSH ovine et la sous-unité β de la FSH humaine :

**Tableau 22**

| CH10 | oFSH | s.u. α oFSH | s.u. β oFSH | s.u. β hFSH |
|---|---|---|---|---|
| Réaction croisée | 100% | 43% | 88% | 40% |

L'anticorps CH10 reconnaît préférentiellement la sous-unité β de la FSH ovine (88%) et deux fois moins la sous-unité β de la FSH humaine et la sous-unité α ovine (40% et 43%). Selon ces résultats, la spécificité de CH10 est anti-sous unité β FSH préférentiellement oFSH. Il reconnaît à un moindre degré, mais de façon non négligeable, la sous-unité α ovine à la différence de l'anticorps CA5. L'ensemble de ces résultats peuvent conduire à l'hypothèse d'un épitope impliquant principalement β mais également α, sur la zone d'association des deux sous-unités par exemple.

Le tableau 23 illustre les pourcentages de réactions croisées de CH10 et du scFv CH10 obtenus avec la FSH porcine (pFSH) et différentes FSH humaines

**Tableau 23**

| | oFSH | pFSH | hFSH (Gonal F) | hFSH (Puregon) | hFSH (Fostimon) | hMG (Menopur) |
|---|---|---|---|---|---|---|
| CH10 | 100% | 24% | 50% | 71% | 100% | 48% |
| scFv CH10 | 100% | 175% | 32% | 67% | 30% | 61% |

L'anticorps CH10 et son scFv présentent une forte reconnaissance des FSH animales et une réaction croisée variant de 30 à 100% pour les FSH humaines.

La spécificité du scFv CH10 a été évaluée vis-à-vis des LH porcine (pLH), ovine (oLH), bovine (bLH), de la eCG et des hCG Chorulon et Endo 5000. Les résultats sont illustrés dans le tableau 24 :

**Tableau 24**

| | oLH | pLH | bLH | eCG | Chorulon | Endo 5000 |
|---|---|---|---|---|---|---|
| scFv CH10 | 68% | 63% | 52% | ND | ND | ND |

La liaison du scFv CH10 vis-à-vis des LH animales est significative avec une réaction croisée entre 52 et 68%. A l'inverse, la trop faible liaison sur les hCG et la eCG adsorbées n'a pas permis de quantifier une réaction croisée. Ces résultats renforcent l'hypothèse d'une spécificité envers un épitope conformationel étant donné les effets biologiques de CH10 et de son scFv obtenus *in vitro* et *in vivo* sur l'activité des hCG Chorulon et Endo 5000 (voir résultats dans Exemples 2 et 3).

Une estimation de la constante de dissociation Kd du scFv CH10, vis-à-vis des différentes FSH, LH et CG étudiées, a été calculée sur GraphPad Prism (GraphPad Software Inc., San Diego, CA, USA, version 5) en utilisant la fonction *"One site* - *Specific binding"* dans un modèle de liaison à saturation ("*saturation binding experiment model",* GraphPad PRISM software). Les valeurs obtenues sont indiquées dans les tableaux 25 et 26.

**Tableau 25**

| scFv CH10 | oFSH | pFSH | hFSH Gonal-F | hFSH Puregon | hFSH Fostimon | hMG Menopur |
|---|---|---|---|---|---|---|
| Kd (10⁻⁶M) | 2,85 | 5,22 | 1,82 | 11,5 | 1,59 | 7,36 |

**Tableau 26**

| scFv CH10 | oLH | pLH | bLH | eCG | hCG Chorulon | hCG Endo 5000 |
|---|---|---|---|---|---|---|
| Kd (10⁻⁶M) | 1,55 | 2,47 | 1,94 | 1,97 | 1,47 | 2,09 |

Les constantes de dissociation Kd ainsi estimées indiquent une affinité du scFv CH10 tant pour les FSH ovine et porcine (Kd de 7,51 et 5,22 µM) que pour les FSH humaines Gonal F, Fostimon et l'hMG Menopur (Kd de 1,82, 1,59 et 7,36 µM respectivement). Les Kd estimées vis-à-vis des hCG et de la eCG (Kd de 1,47 à 2,09 µM) indiquent une bonne affinité du scFv CH10 pour ces hormones comparativement aux FSH.

### EXEMPLE 2 : MESURE IN VITRO DE L'EFFET POTENTIALISANT DES LIGANDS DE L'INVENTION SUR LA BIOACTIVITE DE LA FSH

La mise en évidence de l'effet potentialisant des ligands de l'invention sur la bioactivité de la FSH a été réalisée en comparant la réponse biologique obtenue avec différentes types ou lignées cellulaires stimulées soit avec la FSH seule soit avec le complexe FSH / anticorps monoclonal (AcM).

Dans chacun des cas, la comparaison des courbes dose-réponse obtenues a permis de quantifier l'effet potentialisant *in vitro* de l'AcM sur l'activité biologique de la FSH complexée. L'analyse statistique des résultats a été faite par le logiciel Prism (GraphPad Software Inc., San Diego, CA, USA, version 5).

### 1/ Sur cultures primaires de cellules de granulosa bovines

L'effet potentialisant des AcM CA5 et CH10 sur la bioactivité de la FSH ovine (oFSH) a tout d'abord été caractérisé sur des cellules de granulosa bovines exprimant de façon endogène le récepteur FSH bovin.

Des surnageants d'hybridomes à la concentration finale de 0,1µg/ml d'anticorps CA5 ou CH10 ont été incubés avec une gamme de FSH ovine ou humaine allant de 3 ng/ml à 25 ng/ml, 30 mn à 37°C.

Les cellules de granulosa bovines ont été ponctionnées sur des ovaires de vache à partir de follicules de diamètre allant de 2 à 6 mm, selon le protocole décrit par Chopineau et al. (Mol. Cell Endocrinol., 92(2) : 229-39, 1993) [8] et Wehbi et al. (Endocrinology, 151(6): 2788-2799, 2010) [9]. Les cellules de granulosa bovines en suspension dans un milieu McCoy's 5A (Lonza, Belgique, référence BE12-688F), préparées à raison de 80 000 cellules par 0,5 ml, ont été stimulées pendant 3 heures à 37°C, sous agitation, en présence d'IBMX 48 µg/ml (Sigma Aldrich, France, référence I5879), par une gamme de FSH allant de 3 ng/ml à 25 ng/ml, seule ou préalablement complexée à un anticorps monoclonal selon le protocole ci-dessus. La réponse biologique mesurée a été la sécrétion en AMPc.

Après centrifugation, l'AMPc produit a été dosé dans le surnageant de culture à l'aide d'un kit ELISA (Biomedical Technologies Inc., MA, USA, BT-730).

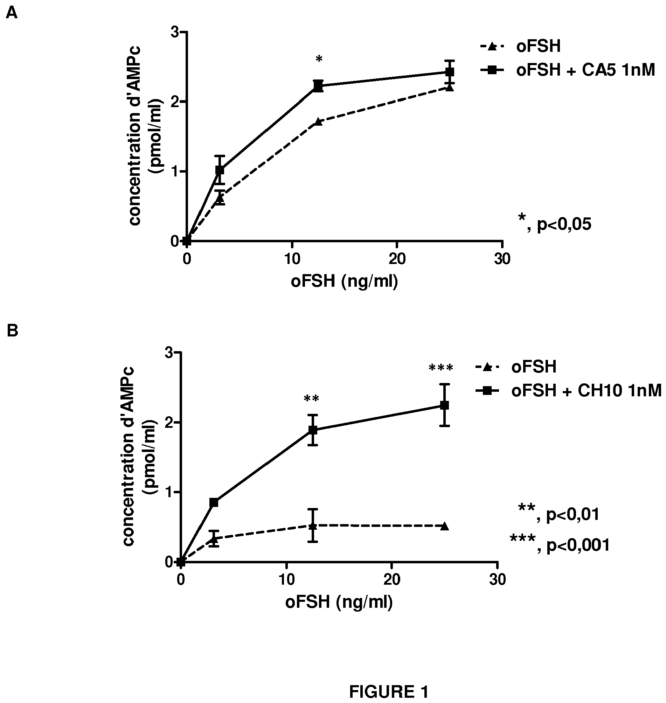

Les résultats sont présentés dans la figure 1.

Les résultats montrent une amplification de la sécrétion d'AMPc d'un facteur 1,3 fois pour CA5, d'un facteur 5,5 fois pour CH10 sur l'activité de la FSH ovine. L'analyse statistique par analyse de variance à deux variables (*two-way* ANOVA, GraphPad PRISM software) montre un effet significatif allant de p<0,05 (*) pour CA5 à p<0,01 (**) et p<0,001 (***) pour CH10.

### 2/ Sur lignée cellulaire HEK293 transfectées de façon stable avec le récepteur FSH humain

L'effet potentialisant des AcM sur la FSH de différentes espèces a été mesuré sur des cellules HEK 293 exprimant de manière stable le récepteur FSH humain. Ce système a permis de mesurer la production d'AMPc suite à l'activation du récepteur de la FSH après une stimulation par la FSH seule ou par le complexe FSH / ACM pendant 1 heure à 37°C.

Pour cela, 60 000 cellules ont été réparties dans des puits de plaques 96 puits (Becton Dickinson, NJ, USA, référence 353072) et cultivées 24h à 37°C, 5% CO₂ en atmosphère humide, dans 100 µl de milieu MEM (Ozyme, France, référence BE12-611F) contenant du SVF 10% (Lonza, Belgique, référence DE14-801F), pénicilline/streptomycine 1% (Sigma Aldrich, France, référence P-4333) et G418 400 µg/ml (Sigma Aldrich, France, référence A1720). Après 2h de sevrage dans du milieu MEM, les cellules ont été stimulées pendant 1h à 37°C. Le surnageant de culture a été récupéré et dosé à l'aide d'un kit ELISA (Biomedical Technologies Inc., MA, USA, BT-730). Les résultats expriment la quantité d'AMPc sécrétée en point final. Ils ont été analysés grâce au logiciel Prism (GraphPad Software Inc., San Diego, CA, USA, version 5).

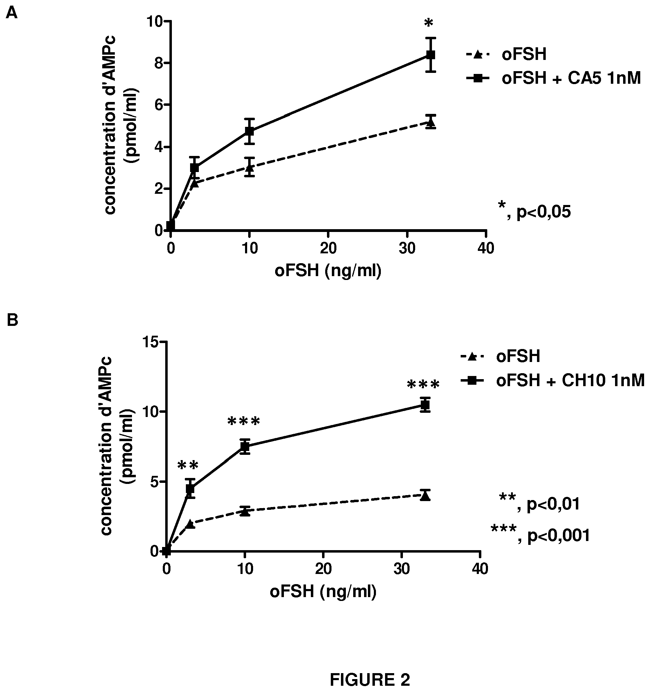

La figure 2 représente l'effet potentialisant des anticorps monoclonaux CA5 et CH10 sur la bioactivité de la FSH ovine (oFSH) *in vitro* sur des cellules HEK 293 transfectées de façon stable avec le récepteur FSH humain. Pour cela, les cellules ont été stimulées soit avec une gamme allant de 3 ng/ml à 32,5 ng/ml de FSH ovine, soit avec les mêmes points de gamme FSH préalablement incubés, 30 minutes à 37°C, avec l'anticorps monoclonal (concentration finale 0,1 µg/ml) avant la stimulation des cellules. Une analyse de variance à deux variables (*two-way* ANOVA, GraphPad PRISM software) a permis de comparer les courbes dose-réponse obtenues avec la FSH seule ou avec le complexe FSH/anticorps monoclonal. L'anticorps CA5 a montré un effet potentialisant allant de 160% à 200% sur l'activité de la oFSH ; cet effet est significatif pour la concentration 32,5 ng/ml de oFSH (p<0,05). L'anticorps CH10 exerce un effet potentialisant plus important sur la oFSH pour l'ensemble des concentrations testées allant de 225% pour le point 3 ng/ml (p<0,01) à 260% pour les points 10 ng/ml et 33 ng/ml respectivement (p<0,001).

### 3/ Sur lignée cellulaire HEK293 transfectées de façon stable avec le récepteur FSH humain et avec le système Glosensor®

L'effet potentialisant des AcM sur les FSH de différentes espèces a été mesuré en temps réel sur des cellules HEK 293 exprimant de manière stable le récepteur FSH humain et le vecteur GloSensor™ (Promega, France). Ce système cellulaire a permis de suivre la production d'AMPc suite à la stimulation du récepteur FSH par l'agoniste (FSH seule ou complexe FSH / anticorps monoclonal) en temps réel. Suite à la liaison de l'AMPc sur la protéine GloSensor™, le substrat GloSensor™ (Promega, France, référence E1291) a été hydrolysé et conduit à une émission de luminescence mesuré grâce à un lecteur PolarStar Optima (BMG Labtech, Allemagne) et exprimée en RLU (Relative Luminescence Unit). Cette lignée stable a été développée par l'équipe de Biologie et Biolnformatique des Systèmes de Signalisation au centre INRA Val de Loire, 37380 Nouzilly, France) et a été mise gracieusement à disposition pour ces essais.

Pour cela, les cellules HEK 293 ont été mises en culture à raison de 80 000 cellules par puits de microplaque 96 puits blanc à fond transparent (Dominique Dutscher, France, référence 655903) et cultivées dans 100 µl de milieu MEM (Ozyme, France, référence BE12-611F) supplémenté de SVF 10% (Lonza, Belgique, référence DE14-801F), pénicilline / streptomycine 1% (Sigma Aldrich, France, référence P-4333), Hygromycine B 200 µg/ml (Life Technologies™, France, référence 10687010) et G418 400 µg/ml (Sigma Aldrich, France, référence A1720) pendant une nuit. Après 2h de sevrage dans 100 µl de milieu MEM supplémenté de BSA 1% (PAA, France, référence K45012) et contenant 4% de substrat GloSensor™ pendant 2h à température ambiante à l'abri de la lumière, la plaque de cellules a été mise dans le lecteur PolarStar Optima et une première lecture a été faite pendant 5 minutes pour mesurer le niveau basal de luminescence. La plaque a ensuite été retirée du lecteur et 11 µl de ligand (FSH seule ou complexe FSH / anticorps monoclonal) y ont été ajoutés de manière à obtenir les concentrations indiquées. La luminescence émise a ensuite été mesurée pendant environ 1h30.

Les résultats obtenus ont été analysés grâce au logiciel Prism (GraphPad Prism Software Inc., San Diego, CA, USA, version 5). La fonction non linéaire « log (agoniste) versus response » a été utilisée pour tracer la réponse en fonction de la concentration de FSH. Ceci a permis de caractériser et comparer l'EC50 pour la FSH seule et la FSH complexée à l'anticorps monoclonal. Pour chaque exemple, l'effet significatif du complexe FSH / anticorps potentialisant a été mesuré par analyse de variance à deux variables (*two-way* ANOVA, GraphPad PRISM software) en comparant les deux courbes dans leur totalité.

### - Anticorps monoclonal CA5

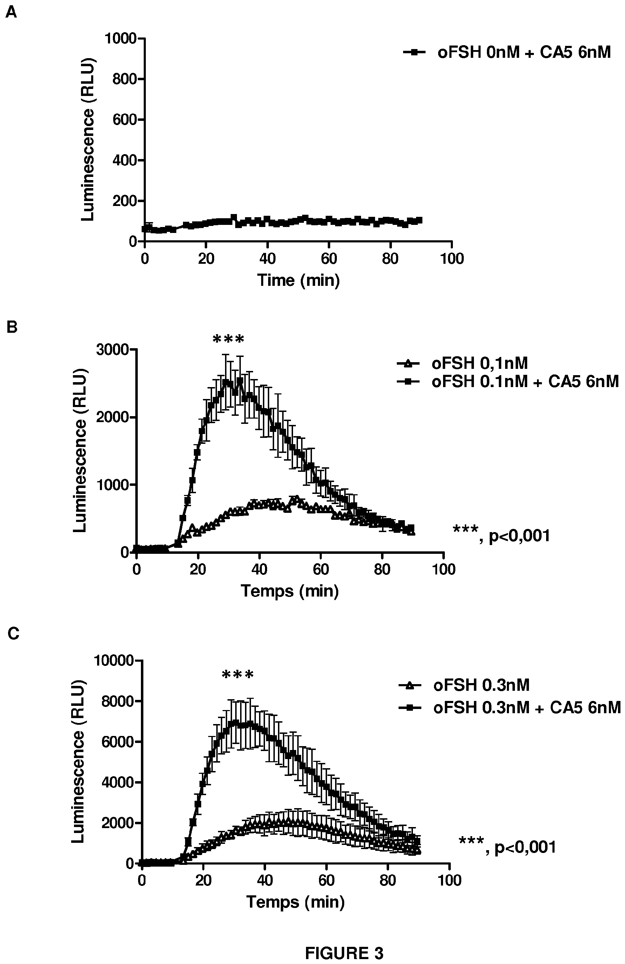

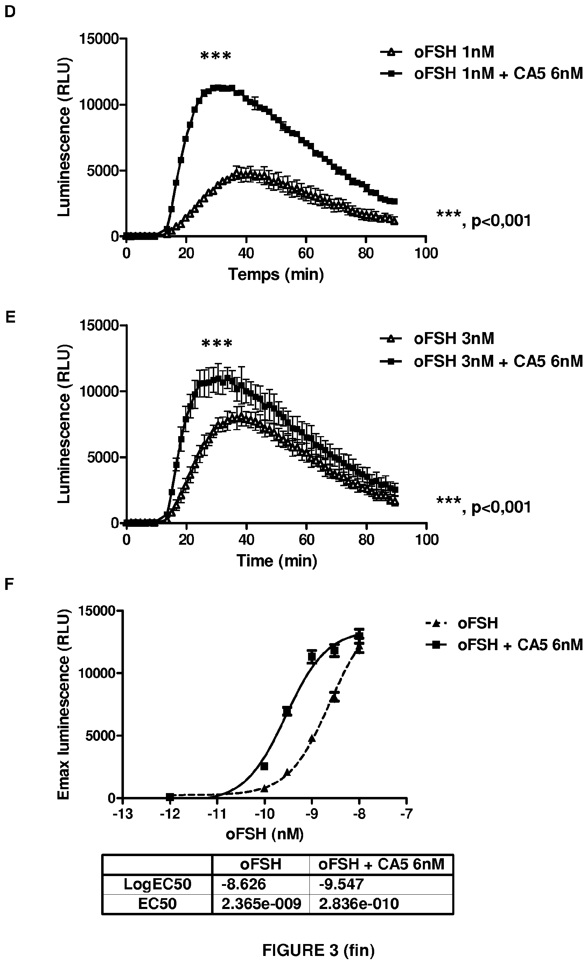

La figure 3 illustre les courbes de cinétique de production d'AMPc exprimées en unités relatives de luminescence en fonction du temps (en minutes) obtenues aux concentrations 0 - 0,1 - 0,3 - 1 et 3 nM de FSH ovine seule ou complexée à l'anticorps CA5. On observe que les cellules « stimulées » par l'anticorps seul, sans FSH, ne montrent aucune réponse : le signal luminescent reste à son niveau basal (courbe 3A). L'anticorps CA5 seul n'exerce aucun effet agoniste ou antagoniste sur le récepteur FSH humain exprimé par les cellules HEK 293. A l'inverse, l'anticorps monoclonal CA5 (concentration finale 6 nM), complexé à la oFSH amplifie de façon très significative et remarquable l'activité stimulante de l'hormone. On observe ainsi une augmentation de la réponse maximale cellulaire de 350% et 330% aux concentrations de 0,1 et 0,3 nM de oFSH (courbes B et C) et une augmentation de 230% et 140% respectivement pour les concentrations de 1 et 3 nM de oFSH (courbes D et E). L'amplitude de l'effet potentialisant devient moins importante avec les plus fortes concentrations de oFSH (1 et 3 nM) en raison d'une saturation de la réponse cellulaire et du signal luminescent à 11000 RLU dans ce cas. La valeur des EC50 mesurée par GraphPad Prism est de 2,36.10⁻⁹M pour la oFSH et de 2,83.10⁻¹⁰M pour le complexe oFSH/CA5 traduisant une augmentation de 1 unité de LogEC50 (de 10^{-8,6} à 10^{-9,54}) lorsque la FSH est complexée à l'anticorps CA5. La différence des deux courbes oFSH versus oFSH/CA5 est hautement significative pour l'ensemble des doses de oFSH testées (p<0,001).

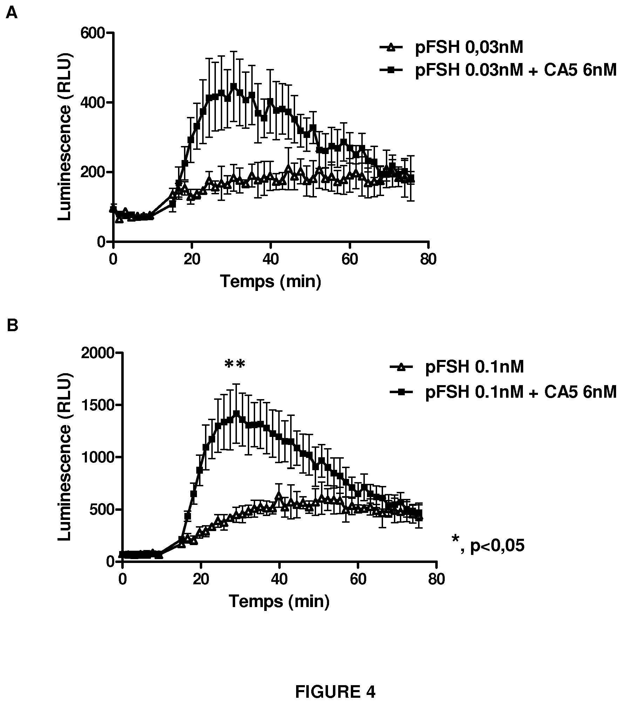

L'effet potentialisant de l'anticorps CA5 mesuré sur la FSH porcine est illustré par la figure 4. On observe une augmentation de 190% du maximum de la réponse à 0,03 nM de pFSH lorsque celle-ci est complexée à l'anticorps CA5 (concentration finale 6 nM) (courbe A), mais cette augmentation n'est pas significative. L'effet potentialisant atteint 250% à la concentration 0,1 nM de pFSH (courbe B) et est significatif (p<0,01). Le niveau de réponse obtenu avec pFSH 0,03 nM complexée à CA5 est équivalent à celui obtenu avec pFSH 0,1 nM seule (500 RLU versus 585 RLU respectivement) ; ce qui signifie que le complexe pFSH 0,03 nM / CA5 induit la même amplitude de réponse que la pFSH seule à une concentration 3,3 fois plus élevée (0,1 nM ; soit 0,5 Log).

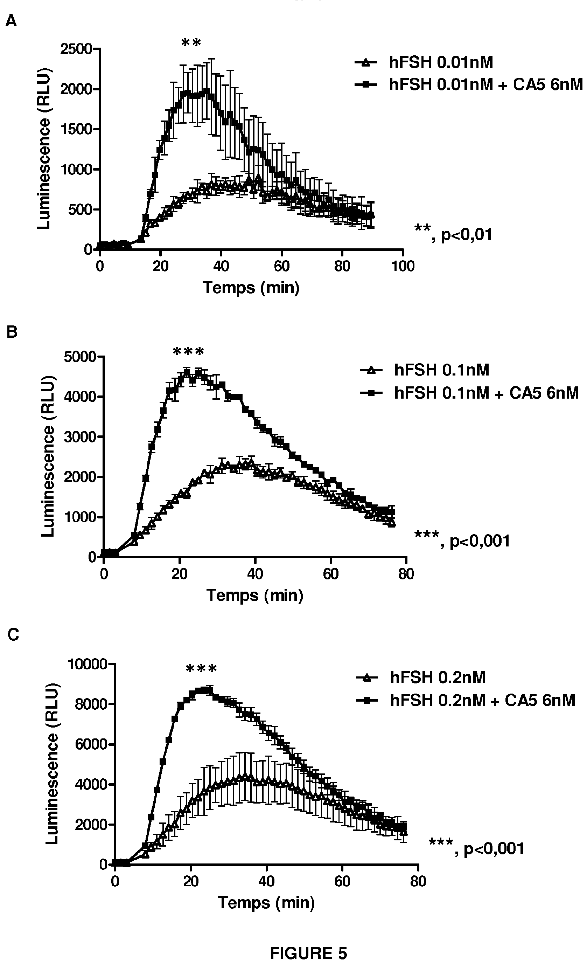

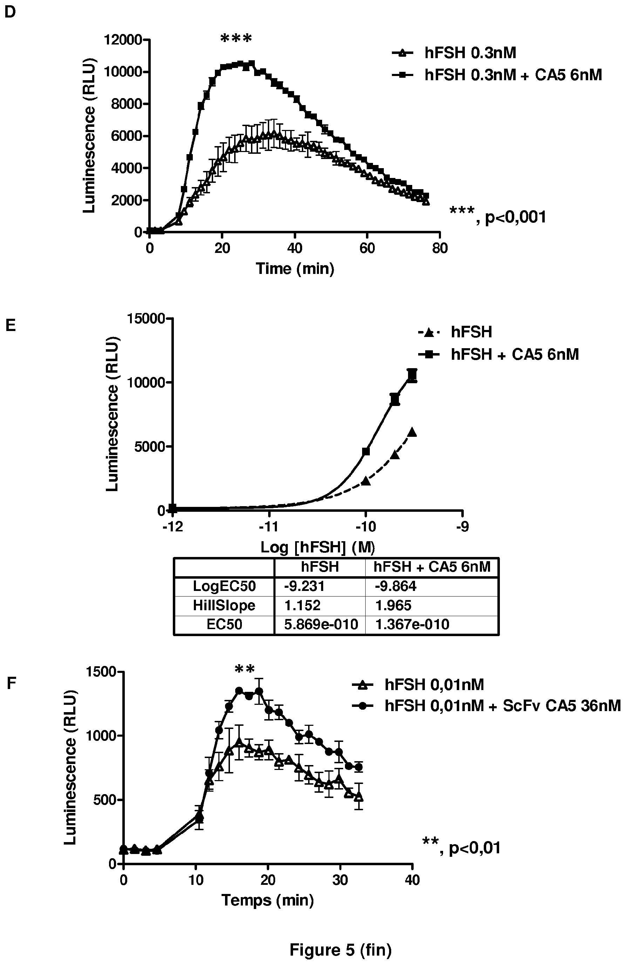

Enfin, l'effet potentialisant de CA5 a été étudié sur l'activité de la FSH humaine recombinante (Gonal-F, Laboratoire SERONO). La figure 5 illustre les courbes de cinétique de production d'AMPc exprimées en unités relatives de luminescence en fonction du temps (minutes) obtenues au cours d'une stimulation avec les concentrations 0,03 - 0,1 - 0,2 - 0,3 nM de FSH seule ou complexée à l'anticorps CA5 (6 nM). Un effet potentialisant de 235% a été observé avec la concentration 0,03 nM hFSH, 200% avec 0,1 et 0,2 nM de hFSH puis 170% avec la plus forte concentration 0,3 nM hFSH en raison d'une saturation du système cellulaire (maximum 10530 RLU). Le calcul des EC50 par GraphPad Prism a indiqué une valeur de 5,86.10⁻¹⁰M pour la hFSH et de 1,36.10⁻¹⁰M pour le complexe hFSH/CA5 traduisant une augmentation de 0,63 en LogEC50 (de 10^{-9,23} à 10^{-9,86}) lorsque la FSH est complexée avec l'anticorps CA5. L'effet potentialisant du scFv CA5 a également été étudié sur la bioactivité de la hFSH à 0,01 nM. Une augmentation significative de 160% (p<0,01) a été obtenue à la concentration de 36 nM de scFv (Figure 5). Cette augmentation était semblable à celle de l'anticorps entier, bivalent CA5. Ce résultat signifie que le fragment monovalent possède les mêmes propriétés potentialisantes de l'activité FSH que l'anticorps CA5.

Bien que significatif (p<001), l'effet potentialisant de l'anticorps CA5 sur la bioactivité de la FSH humaine reste moins important que sur la bioactivité de la FSH ovine pour laquelle une augmentation d'une unité de Log a été obtenue entre l'EC50 de la oFSH et celui du complexe oFSH / CA5.

### - Anticorps monoclonal CH10

L'effet modulateur de l'anticorps CH10 a été étudié sur la FSH ovine (oFSH) et sur la FSH humaine (hFSH) (Gonal F, Laboratoire SERONO).

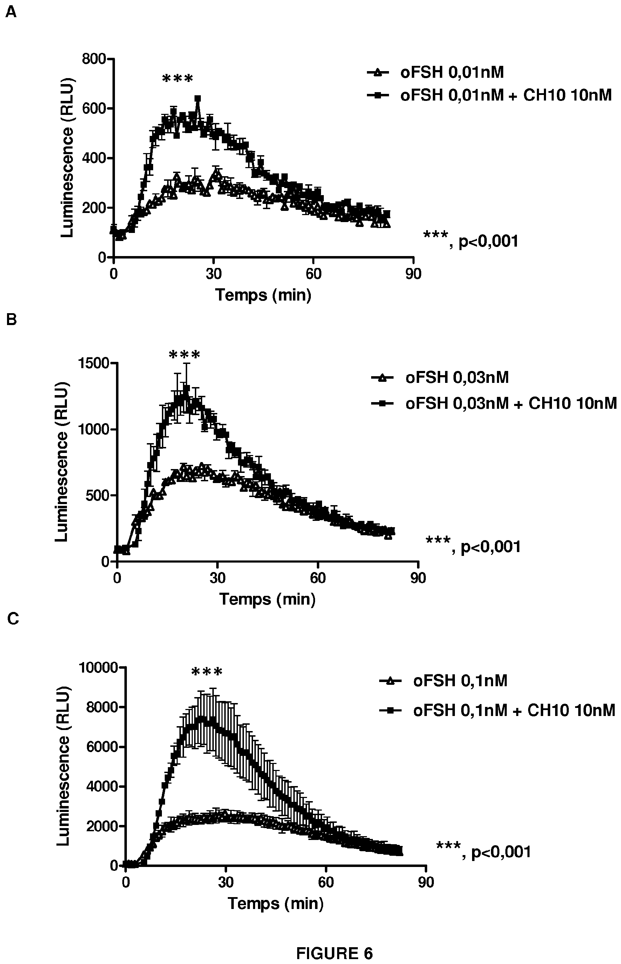

La Figure 6 illustre l'effet amplificateur de l'anticorps CH10 (10nM) sur la bioactivité de la oFSH préparée aux concentrations 0,01 - 0,03 et 0,1 nM. Sur les faibles concentrations (0,01 et 0,03 nM) on obtient une augmentation de 185% de la réponse cellulaire avec le complexe oFSH / CH10 (courbes A et B). A la concentration de 0,1 nM de oFSH, l'augmentation est de 312% avec le complexe oFSH / CH10 (courbe C). Ces augmentations sont très significatives (p<0,001).

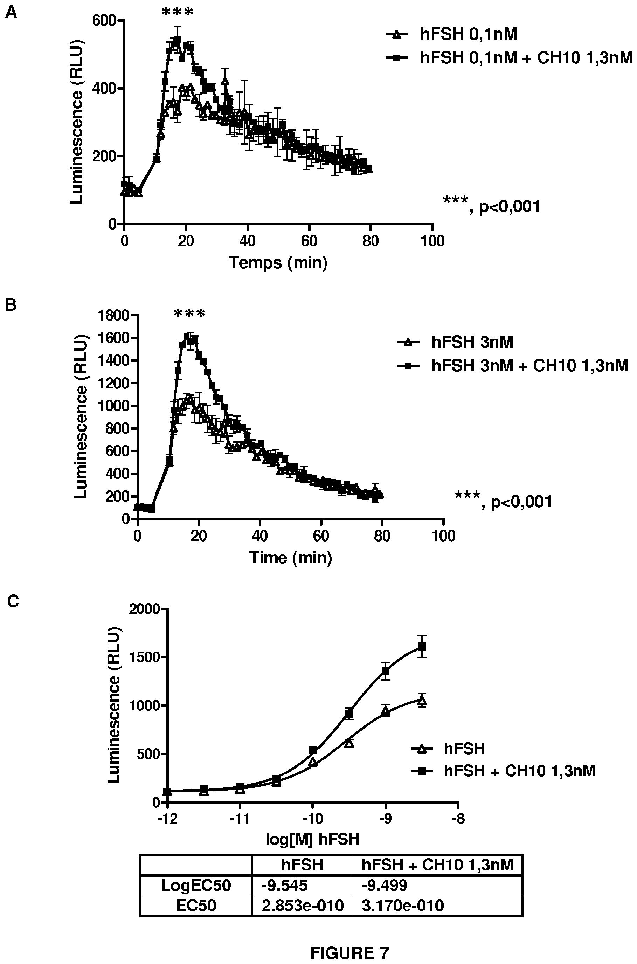

L'effet potentialisant de CH10 (1,3 nM) a été mesuré sur la FSH humaine préparée de 0,1 nM à 3 nM (Figure 7A et B). Une augmentation modérée mais significative (p<0,001) allant de 140% à 150% a été observée. Les EC50 mesurés par GraphPad Prism ont été de 2,85.10⁻¹⁰M pour la hFSH et de 3,17.10⁻¹⁰M pour le complexe hFSH / CH10 (Figure 7C).

L'effet potentialisant de CH10 s'exerce plus spécifiquement sur la FSH ovine, animale. Un faible effet de l'anticorps CH10 a été observé sur la FSH humaine.

### EXEMPLE 3 : MESURE IN VIVO DE L'EFFET POTENTIALISANT DES LIGANDS DE L'INVENTION SUR LA BIOACTIVITE DES FSH et LH/CG DANS LE MODELE RAT

Après avoir été caractérisé *in vitro,* l'effet potentialisant de chaque anticorps monoclonal a été caractérisé *in vivo,* chez le rat femelle pour leur effet sur la bioactivité de la FSH et chez le rat mâle pour leur effet sur la bioactivité de la LH/CG, qu'ils reconnaissent également.

Pour mesurer la bioactivité FSH, le protocole utilisé a été celui du dosage biologique décrit par Steelman et Pohley (Steelman SL, Pohley FM. Endocrinology, 53 :604-616. 1953) [12]. Pour mesurer la bioactivité LH, le protocole utilisé a été celui du dosage décrit par Scobey et collaborateurs (Scobey et al, Reprod. Biol. Endocr. 3 :61, 2005) [13].

L'effet des anticorps sur l'activité FSH a été évalué en utilisant des FSH ovine et humaines. L'effet des anticorps sur l'activité LH a été évalué sur deux préparations de hCG (human Chorionic Gonadotropin).

L'analyse statistique a été réalisée avec le logiciel GraphPad Prism (GraphPad Software Inc., San Diego, CA, USA, version 5). Les résultats portant sur des expériences réalisées sur des lots de 5 animaux, une analyse de variance à une variable, non paramétrique (test de Kruskal Wallis), suivie d'une correction de Dunns, a été appliquée ou un test-t non-paramétrique (test de Mann-Whitney). Pour les résultats portant sur des effectifs plus importants (n>30) issus de la compilation de plusieurs bioassays, un test paramétrique (test t de Student non apparié) suivi d'une correction de Bonferroni a été appliqué.

### 1/ Effet potentialisant des anticorps sur la bioactivité de la FSH chez la Ratte

L'effet potentialisant des anticorps CA5 etCH10 et de leur scFv a été étudié sur la FSH ovine et sur différentes préparations de FSH humaine utilisées en reproduction humaine la Gonal-F et la Puregon (FSH recombinantes des laboratoires Merck Serono et Merck Schering-Plough respectivement), la Fostimon et la Menopur (FSH extraites commercialisées par les laboratoires Genevrier et Merck Schering-Plough respectivement).

Comme décrit dans le protocole de Steelman et Pohley, des rattes immatures de 21 jours ont reçu pendant trois jours consécutifs 2 injections matin et soir, de 100 µl d'un mélange de hCG et FSH comportant une quantité constante de hCG (3,5 UI) additionnée d'une quantité variable de FSH allant de 0,5 à 1,5 UI pour la FSH humaine (Gonal F, Puregon, Fostimon, Menopur) ou de 0,5 à 2 µg pour la FSH ovine (hormone extraite). Les injections ont été réalisées par voie sous-cutanée au niveau de la nuque. Chaque expérience comprenait au minimum 4 lots : un lot traité avec du sérum physiologique (sérum Φ), un lot traité avec l'anticorps ou le scFv seul, un lot traité avec le mélange hCG+FSH, un lot traité avec le mélange hCG/FSH additionné de 2 µg d'anticorps ou de scFv purifié.

Dans le cas d'un traitement avec le complexe hormone/anticorps ou scFv, avant l'injection, le mélange FSH + anticorps a été préalablement incubé 20 min à 37°C ou à température ambiante indifféremment, puis ajouté à la hCG. La hCG peut indifféremment être mélangée à la FSH pendant l'incubation du complexe.

Le quatrième jour, les rattes ont été pesées, et leurs ovaires ont été prélevés, disséqués puis pesés. Les résultats sont exprimés en milligramme d'ovaire/100 grammes de poids corporel. L'augmentation du poids des ovaires est proportionnelle à la quantité de FSH bioactive injectée. Ceci permet de quantifier et de comparer la bioactivité d'une même quantité d'hormone injectée seule ou en complexe avec un anticorps.

La comparaison de la bioactivité de la FSH injectée seule ou complexée à l'anticorps ou au scFv permet de mesurer le différentiel de la réponse et de quantifier ainsi l'effet potentialisant de l'anticorps ou de son scFv.

### Effet de l'anticorps CA5 et de son scFv

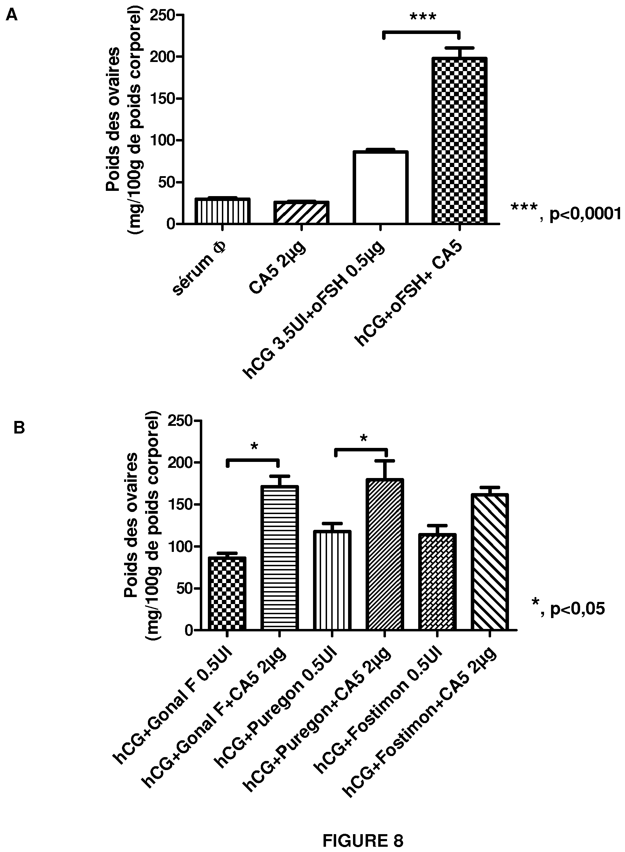

La figure 8A illustre un exemple représentatif de l'effet de l'anticorps CA5 sur la bioactivité FSH ovine. Chaque lot comportait 5 femelles. Le lot traité avec l'anticorps CA5 injecté seul a présenté un même poids moyen des ovaires que les femelles du lot contrôle ayant reçu du sérum physiologique (25,6 mg et 29,15 mg respectivement). Le lot ayant reçu le traitement hormonal classique (hCG 3,5 UI +oFSH 0,5 µg) a donné un poids moyen des ovaires de 85,7 mg significativement plus élevé que le lot contrôle (p<0.05). Le lot traité avec la FSH ovine préalablement complexée à l'anticorps CA5 a présenté un poids moyen de 198 mg soit une augmentation très significative de la bioactivité FSH de 231% par rapport au lot ayant reçu le traitement hormonal classique (p<0.0001). L'effet potentialisant de CA5 sur l'activité de la FSH ovine, *in vivo,* a été analysé sur plusieurs expériences dont l'ensemble des résultats est présenté dans le tableau 27. L'augmentation du poids moyen des ovaires, enregistré sur trois expériences, suite à une stimulation avec le complexe hormone / CA5 est hautement significative (p<0.0001).

**Tableau 27**

| lot | Moyenne ± sem | effectif | statistiques |
|---|---|---|---|
| Sérum Φ | 25,6 ± 2,4 | 13 | NS |
| CA5 | 29,15 ± 4,6 | 15 | |
| hCG+oFSH | 98,12 ± 6,37 | 13 | *** |
| hCG+oFSH+CA5 | 165,8 ± 11,54 | 12 | p<0.0001 |

L'effet de CA5 sur les FSH humaines a également été analysé sur un effectif important au cours de plusieurs expériences. Les résultats sont présentés dans le tableau 28 ci-dessous.

**Tableau 28**

| lot | Moyenne ± sem | effectif | statistiques |
|---|---|---|---|
| hCG+hFSH | 73,93 ± 1,525 | 77 | *** |
| hCG+hFSH+CA5 | 128,3 ± 4,323 | 80 | p<0.0001 |

L'augmentation du poids des ovaires moyen enregistrée chez les femelles traitées par le complexe hFSH Gonal F / CA5 est de 173% : le poids moyen des ovaires passe de 73,93 mg chez les femelles ayant reçu un traitement classique à 128,3 mg chez les femelles ayant été traitées avec le complexe hormone/CA5. Cette différence est hautement significative (p<0,0001, test-t non apparié).

Enfin, l'effet potentialisant a été étudié sur deux autres préparations de FSH humaine (Puregon et Fostimon). Les résultats illustrés sur la figure 8B sont un exemple représentatif. Une augmentation significative de 153% a été enregistrée sur l'activité de la FSH humaine Puregon (p<0,05) et de 142% sur l'activité de la FSH humaine Fostimon (NS). Par comparaison, l'augmentation sur l'activité de la FSH humaine Gonal F a été de 179% dans cette expérience (p<0,05).

En conclusion, l'anticorps CA5 exerce un effet potentialisant majeur sur la FSH ovine et un effet potentialisant également important sur l'activité de la FSH humaine, provenant de différentes préparations pharmaceutiques.

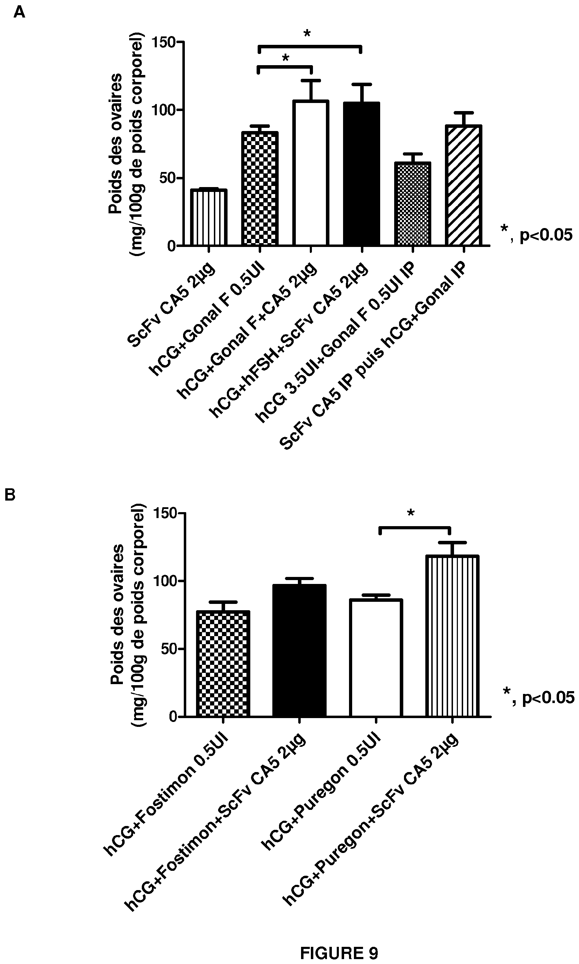

L'effet du scFv CA5 a été étudié dans le même protocole que l'anticorps entier. La figure 9A illustre les résultats obtenus. Un même effet potentialisant sur l'activité de la FSH humaine Gonal F a été obtenu avec l'anticorps entier ou avec son scFv de façon significative (p<0,05).

Différents modes d'injection des mélanges hormone/ scFv ont été évalués et comparés avec le protocole classique (injection par voie sous-cutanée). Ainsi un bioassay a visé à comparer une injection du mélange hormonal par voie intra-péritonéale avec une injection du mélange hormonal par voie intra-péritonéale suivie d'une deuxième injection différée du scFv CA5 15 minutes plus tard (figure 9A). Dans ce cas, une augmentation de 146% a été obtenue chez les femelles traitées avec le complexe hormone/scFv avec un poids moyen des ovaires passant de 61 mg (lot hCG+hFSH Gonal F) à 89 mg (lot scFv puis hCG+hFSH Gonal F). Ces résultats sont importants car ils démontrent que l'effet potentialisant du scFv CA5 peut se mettre en place *in vivo* même si le scFv est injecté seul, indépendamment de l'hormone et en différé. Le complexe hormone / scFv pouvant se constituer *in vivo* chez l'animal traité.

L'effet potentialisant du scFv CA5 a également été étudié sur l'activité des FSH humaines Fostimon et Puregon (Figure 9B). Une augmentation significative de 140% a été obtenue sur l'activité de la FSH Puregon (p<0.05) et une augmentation non significative de 126% sur l'activité de la FSH Fostimon (NS).

### Effet de l'anticorps CH10 et de son scFv

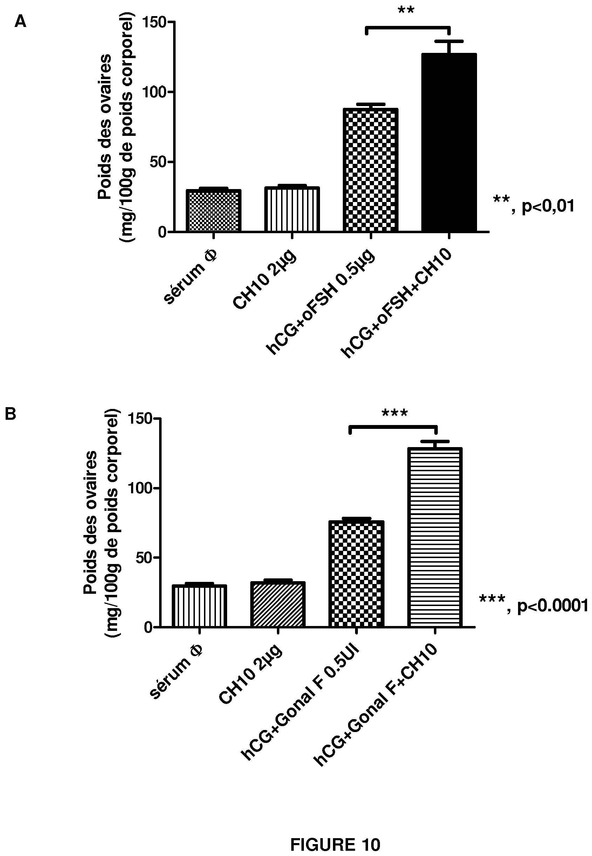

L'effet potentialisant de CH10 sur l'activité de la FSH ovine, *in vivo,* a été analysé au cours de plusieurs expériences dont l'ensemble des résultats est présenté sur la Figure 10A. La comparaison sur trois expériences de la réponse entre les rattes traitées, avec le mélange hCG+oFSH et celles traitées avec le complexe hormones / CH10 met en évidence une augmentation significative de 145% (p<0.01). Le poids moyen des ovaires enregistré a été de 87.55 ± 3,724 mg chez les femelles traitées avec le mélange hCG+oFSH (n=12) à 126,6 ± 9,6 mg/100gr de poids corporel chez les rattes traitées avec le complexe hormone / CH10 (n=13). On n'a observé aucun effet de l'anticorps CH10 seul sur la réponse ovarienne.

L'effet potentialisant de CH10 sur la FSH humaine Gonal-F a également été analysé sur un effectif important au cours de plusieurs expériences. Les résultats sont présentés dans le tableau 29 ci-dessous et sur la Figure 10B.

**Tableau 29**

| lot | Moyenne ± sem | effectif | statistiques |
|---|---|---|---|
| hCG+hFSH GONAL F | 73,93 ± 1,525 | 77 | *** |
| hCG+hFSH Gonal F+CA5 | 128,3 ± 4,323 | 80 | p<0.0001 |

Une augmentation de 170% du poids des ovaires moyen a été enregistrée chez les femelles traitées par le complexe Gonal F/CH10. Cette différence est hautement significative (p<0.0001, test-t non apparié).

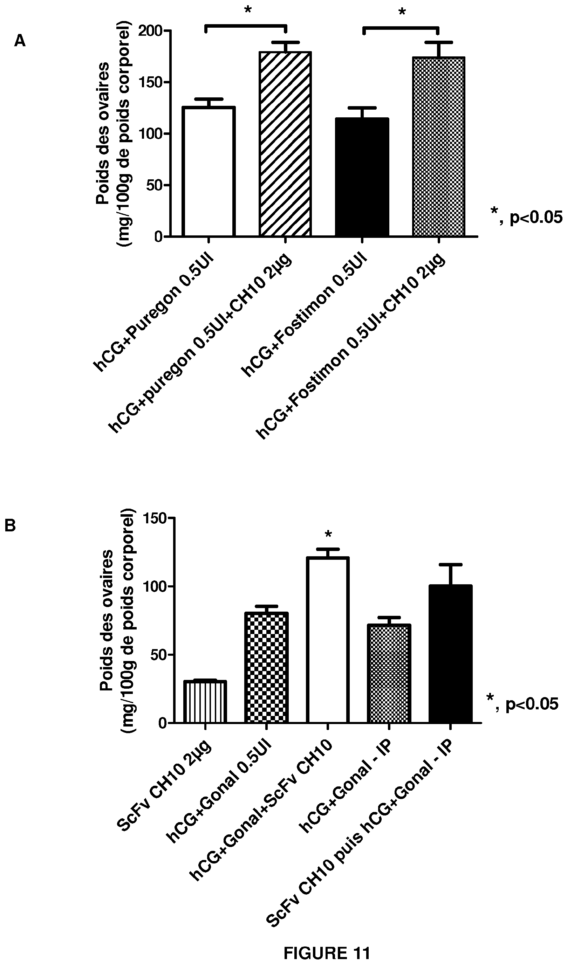

L'effet potentialisant de CH10 a également été recherché sur les FSH humaines Puregon et Fostimon (Figure 11A). Une augmentation significative de 145% et 174% a été obtenue respectivement (p<0,05). La figure 11B illustre l'effet du complexe hFSH Gonal F / scFv CH10 en injection classique par voie sous-cutanée et en injection différée en temps par voie intra-péritonéale. Une augmentation significative de 160% (p<0,05) a été obtenue par injection classique et une augmentation de 150%, bien que non-significative, par injections indépendantes du scFv CH10 et de la hFSH par voie intra-péritonéale. L'effet potentialisant du scFv CH10 peut donc se mettre en place *in vivo,* que celui-ci soit injecté séparément ou préincubé avec l'hormone.

### Effet potentialisant des anticorps sur la bioactivité de la LH/CG chez le Rat

En raison du coût très élevé de la LH ovine, ces dosages biologiques ont été réalisés avec de la hCG, facilement disponible, sous une forme très pure et peu onéreuse. L'effet des anticorps a été étudié sur deux préparations de hCG (human Chorionic Gonadotropin) humaines extraites, l'une utilisée en reproduction humaine dans le cadre des traitements de procréation médicalement assistée : l'ENDO 5000 (laboratoire Schering-Plough) et l'autre utilisée en médecine vétérinaire : la Chorulon (laboratoire MSD).

Selon le protocole de Scobey et collaborateurs [13], la bioactivité de la LH ou de la hCG a été quantifiée par rapport à l'augmentation du poids des vésicules séminales dont le développement est androgéno-dépendant. Le poids varie proportionnellement à l'activité de la hCG et permet donc de quantifier et comparer l'activité biologique de l'hormone injectée seule ou complexée avec l'anticorps étudié. Le protocole a été réalisé avec des ratons de 25 jours qui ont été injectés par voie sous-cutanée, une fois par jour pendant quatre jours avec 100 µl de 1,5 UI de hCG ou d'un mélange 1,5 UI hCG + 2 µg d'anticorps préalablement incubé 20 mn à 37°C. Le cinquième jour, les rats ont été pesés puis sacrifiés. Leurs vésicules séminales (VS) ont été prélevées, disséquées et pesées. Le poids des vésicules séminales est exprimé en mg/100g de poids corporel afin de pouvoir comparer et réunir les résultats obtenus avec différents lots. Dans chaque expérience, chacune des conditions a été testée sur un lot de 5 rats. Une même expérience a été répétée plusieurs fois.

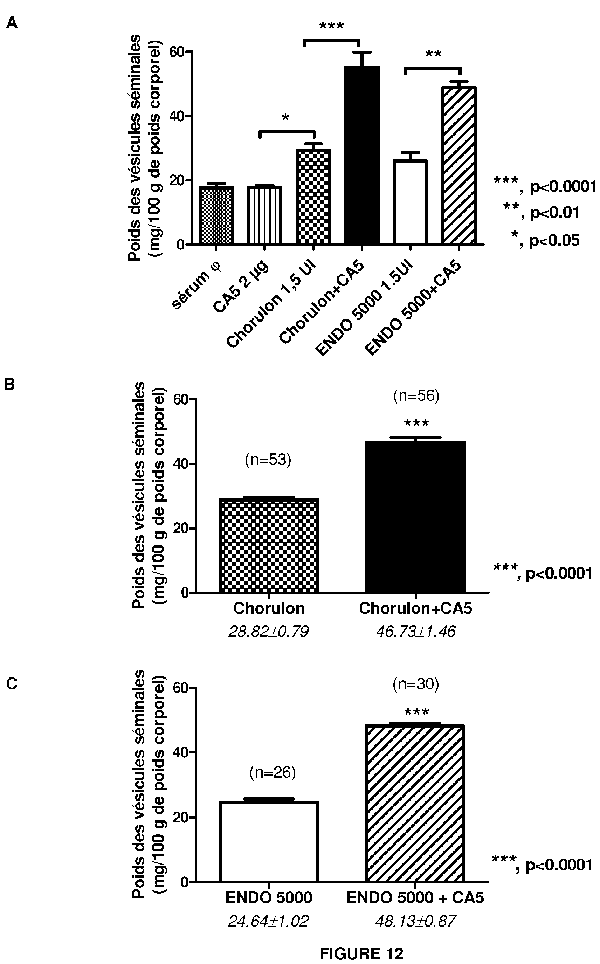

L'effet de CA5 sur les deux préparations de hCG : Chorulon et Endo 5000 est illustré sur la figure 12. L'histogramme A est un exemple représentatif d'un bioassay réalisé sur 6 lots de 5 rats. Un effet potentialisant très significatif (p<0,0001, test de Krustal et Wallis) a été obtenu avec le complexe hCG Chorulon / CA5 avec une augmentation de 196% du poids des vésicules séminales par rapport au lot traité avec hCG seule. Un effet significatif a également été obtenu avec le complexe hCG ENDO 5000 / CA5 avec une augmentation du poids de 193% (p<0,01). On observe que le lot traité avec l'anticorps CA5 seul ne présente aucun changement du poids des vésicules séminales par rapport aux animaux contrôles traités avec du sérum physiologique : l'anticorps seul n'exerce aucun effet sur l'organe cible contrairement au complexe.

La somme des résultats obtenus au cours des répétitions de ce bioassay avec les deux hCG confirme un effet potentialisant hautement significatif (p<0,0001, Test-t non apparié) du complexe hormone / CA5 :
- sur un effectif de 53 et 56 animaux respectivement, le poids moyen des VS a été de 28,8 mg/100 g chez les rats traités avec hCG Chorulon et 46,7 mg/100 g chez les rats traités avec le complexe (augmentation de 162%) (Figure 12B)
- sur un effectif de 26 et 30 animaux respectivement, le poids moyen des VS a été de 24,64 mg/100 g chez les rats traités avec hCG ENDO 5000 et 48,13 mg/100 g chez les rats traités avec le complexe (augmentation de 195%) (Figure 12C).

L'anticorps CH10 a présenté également un effet potentialisant significatif sur la hCG Chorulon et la hCG ENDO 5000, *in vivo,* chez le rat.

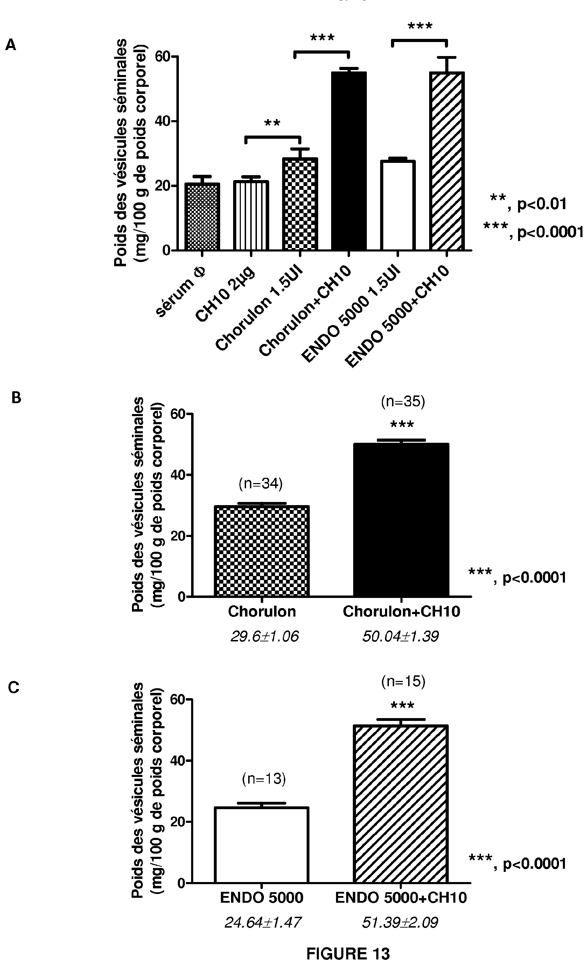

La figure 13A illustre un cas représentatif de bioassay réalisé sur 6 lots de 5 rats. Un effet potentialisant très significatif (p<0,0001, test de Krustal et Wallis) a été obtenu avec le complexe hCG Chorulon / CH10 avec une augmentation de 193% du poids des vésicules séminales par rapport au lot traité avec hCG seule. Un effet significatif a également été obtenu sur le lot traité avec le complexe hCG ENDO 5000 / CH10 avec une augmentation du poids de 199% (p<0,0001). On observe que le lot traité avec l'anticorps CH10 seul ne présente aucun changement du poids des vésicules séminales par rapport aux animaux contrôles traités avec du sérum physiologique. CH10 non complexé à l'hormone n'a donc aucun effet propre sur l'organe cible.

La compilation des résultats obtenus au cours des répétitions de ce bioassay avec les deux hCG confirme un effet potentialisant hautement significatif (p<0,0001, Test-t non apparié) du complexe hormone / CH10 :
- sur un effectif de 34 et 35 animaux respectivement, le poids moyen des VS a été de 29,6 mg/100 g chez les rats traités avec hCG Chorulon contre 50,04 mg/100 g chez les rats traités avec le complexe (augmentation de 169%) (Figure 13B)
- sur un effectif de 13 et 15 animaux respectivement, le poids moyen des VS a été de 24,64 mg/100 g chez les rats traités avec la hCG ENDO 5000 et 51,39 mg/100 g chez les rats traités avec le complexe (augmentation de 208%) (Figure 13C).

### EXEMPLE 4 : MESURE IN VIVO DE L'EFFET POTENTIALISANT DES LIGANDS DE L'INVENTION SUR LA BIOACTIVITE DES GONADOTROPINES ENDOGENES CHEZ LA BREBIS

Après avoir démontré et caractérisé l'effet potentialisant *in vivo,* des anticorps monoclonaux CA5 et CH10, chez un rongeur (animal de petite taille), l'objectif a été d'étudier l'effet de chaque anticorps sur l'activité de la FSH chez un animal de rente, de plus grande taille : la brebis.

Pour cela, une étude a été réalisée sur des brebis Ile de France, pubères, toutes du même âge, dans le but d'évaluer l'effet potentialisant des anticorps sur les propres hormones des brebis traitées (hormones endogènes). L'étude de la spécificité a montré en effet une forte liaison des anticorps CA5 et CH10 pour la FSH ovine et une liaison plus variable pour la LH ovine. Dans cet objectif, un traitement ne comprenant que l'injection d'un anticorps seul a été mis au point pour en évaluer son efficacité.

Dans les protocoles mis en place chez la brebis, chaque anticorps a donc été injecté seul et non préalablement incubé avec la FSH exogène comme cela a été réalisé dans les études chez la Ratte. De plus, chaque anticorps a été injecté chez des brebis indemnes de toute stimulation préalable de l'ovaire : les animaux n'ont reçu aucun traitement hormonal de stimulation de l'ovulation avec une gonadotropine préalablement à l'injection de l'anticorps.

L'effet potentialisant des anticorps anti-FSH CA5 et CH10 a été évalué au cours de protocoles réalisés en pleine saison sexuelle (janvier) ou en fin de saison sexuelle (fin du mois de mars). Les protocoles ont tous été réalisés sur des brebis dont le cycle ovulatoire a été préalablement synchronisé par la pose d'une éponge vaginale imprégnée d'un progestagène (45 mg d'acétate de fluorogestone (FGA) - MSD) pendant 14 jours. L'effet potentialisant a été analysé en comparant la réponse ovulatoire (nombre d'ovulations) et la mise en place d'un ou de plusieurs corps jaunes fonctionnels de bonne qualité (amplitude de la sécrétion de progestérone) chez des brebis contrôles (lot sérum physiologique), des brebis stimulées par un traitement de FSH porcine (lot FSH) et des brebis stimulées par un anticorps seul (lot anticorps).

Dans chaque protocole, un dosage de la LH plasmatique a été réalisé par méthode ELISA afin de détecter et dater le pic pré-ovulatoire de LH. Pour évaluer la réponse ovulatoire une observation endoscopique des ovaires a été réalisée par laparoscopie, sous anesthésie, huit jours après le retrait de l'éponge vaginale, afin de compter le nombre de corps jaunes et observer leur aspect.

Pour évaluer la fonctionnalité et la qualité du ou des corps jaunes, un dosage ELISA quantitatif de progestérone a été réalisé à partir de prises de sang quotidiennes du premier au 21^{ème} jour après le retrait de l'éponge.

Toutes les analyses statistiques ont été faites avec le logiciel GraphPad Prism Version 5.0 (GraphPad, San Diego, CA, USA).

### Anticorps CA5

L'effet potentialisant de l'anticorps CA5 (IgG) a été évalué dans deux protocoles (1 et 2) en période de saison sexuelle et en fin de saison sexuelle.

### Dans le protocole 1, réalisé en fin de saison sexuelle :

- le lot "anticorps CA5" (n=6) a reçu trois injections de CA5 purifié par voie intra-musculaire : 2 mg 4 jours avant le retrait de l'éponge, 1 mg avant le retrait et 1mg au moment du retrait de l'éponge.
- le lot "contrôle" (n=5) a reçu une injection de sérum physiologique, par voie intramusculaire, 24h avant le retrait de l'éponge
- le lot "FSH" (n=5) a reçu une injection par voie intra-musculaire de 100 µg de FSH porcine (pFSH) 24h avant le retrait de l'éponge et de 90 µg 12h avant le retrait de l'éponge.

L'analyse de la réponse ovulatoire a donné les résultats présentés dans le tableau 30 ci-dessous.

**Tableau 30**

| | Lot sérum Φ | Lot FSH | Lot CA5 seul | Statistiques |
|---|---|---|---|---|
| Nombre de brebis par lot | 5 | 5 | 6 | |
| Nombre de brebis ayant ovulé par lot | 2/5 (40%) | 2/5 (40%) | 2/6 (35%) | NS |
| Nombre de corps jaunes par brebis ayant ovulé | 1,5 ± 0,7 | 3 ± 2,8 | 1,5 ± 0,7 | NS |
| Moment du pic de LH (heures après retrait) | 72 ± 17 | 60 ± 0 | 78 ± 25 | NS |

L'analyse statistique a été faite par un test exact de Fisher.

Comparativement aux lots contrôle et FSH, les résultats obtenus dans le lot CA5 ne montrent pas d'effet significatif sur la réponse ovulatoire. Aucun des paramètres mesurés ne présentent de tendance.

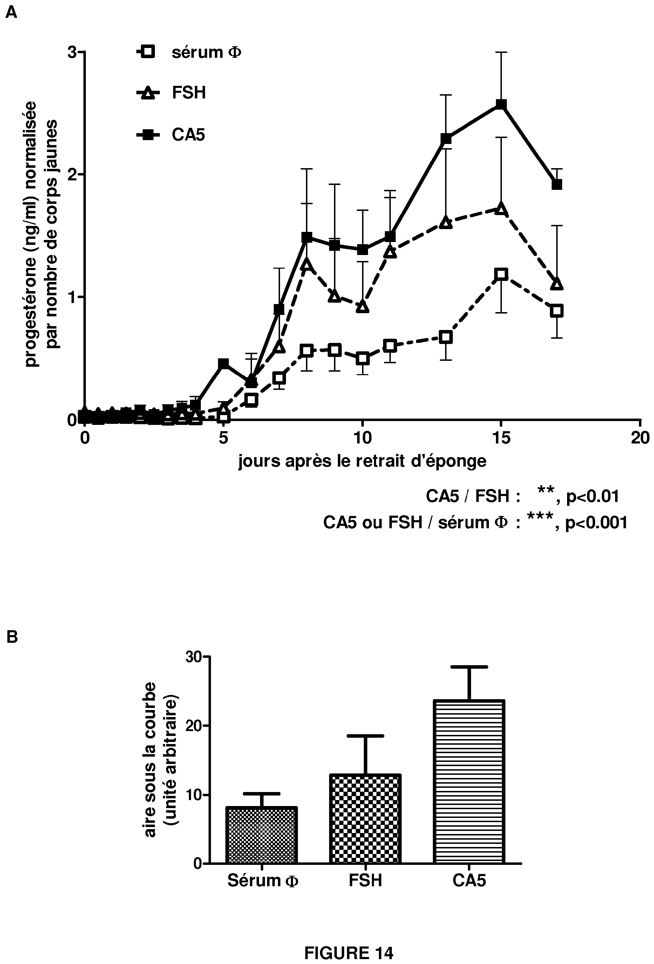

Le profil de sécrétion de la progestérone au cours de la phase lutéale obtenue dans les trois lots, est illustré dans la figure 14. Pour chaque individu, les valeurs de concentration en progestérone (ng/ml) ont été normalisées par nombre de corps jaunes. Chaque courbe représente la moyenne des valeurs de progestérone obtenue chez les femelles de chaque lot à un temps t.

Un effet significatif de l'anticorps CA5 a été observé sur l'intensité de la sécrétion de progestérone par corps jaune et sur le début de sa mise en place (Figure 14A). On observe en effet sur la courbe CA5, par rapport aux courbes des lots FSH et sérum Φ, un début de sécrétion de progestérone dès J4 suivie d'une augmentation significative de la sécrétion qui se maintient tout au long de la phase lutéale jusqu'en fin de cycle. Les valeurs moyennes de progestérone à J10 sont de 1,38 ng/ml - 0,92 et 0,52 ng/ml respectivement pour les lots CA5, FSH et sérum Φ et de 2,58 - 1,7 et 1,18 ng/ml à J15. La comparaison des trois courbes a été faite par un test t non paramétrique apparié (test de Wilcoxon). Ainsi, la courbe du lot CA5 est plus élevée que la courbe du lot FSH et significativement différente (p < 0.01), de même avec la courbe du lot contrôle (p< 0.001). La courbe du lot FSH est plus élevée et significativement différente de celle du lot contrôle (p< 0.001).

Pour quantifier cette augmentation significative et constante du niveau de sécrétion de la progestérone jusqu'en fin de cycle chez les brebis sous stimulation de CA5, un calcul d'aire sous la courbe (AUC) a été réalisé avec le logiciel GraphPad Prism version 5.0. Les résultats sont illustrés sur la figure 14B et montrent que l'AUC de la courbe CA5 (23,61 unités arbitraires) tend à être plus élevée que l'AUC de la courbe sérum Φ (8 unités) mais cette différence n'est pas significative. De même, l'AUC de la courbe FSH (12 unités) n'est pas significativement différente de la courbe contrôle.

En conclusion, l'injection de CA5 chez des brebis donne les mêmes résultats qu'un traitement classique avec FSH en termes d'induction de l'ovulation mais permet la mise en place plus rapide de la sécrétion de progestérone et le maintien d'un corps jaune fonctionnel plus efficace avec un niveau circulant de progestérone plus élevé, garant d'un meilleur succès du développement embryonnaire précoce et du maintien de la gestation (risque d'avortement diminué).

### Dans le protocole 2, réalisé en saison sexuelle :

- le lot "anticorps CA5" (n=7) a reçu une seule injection par voie intra-musculaire de 2 mg d'anticorps CA5 24h avant retrait de l'éponge.
- le lot "contrôle" (n=9) a reçu une injection de sérum physiologique, par voie intramusculaire, 24h avant le retrait de l'éponge
- le lot "FSH" (n=11) a reçu une injection par voie intra-musculaire de 100µg de FSH porcine (pFSH) 24h avant le retrait de l'éponge et de 90 µg 12h avant le retrait de l'éponge.

L'analyse de la réponse ovulatoire a donné les résultats présentés dans le tableau 31 ci-dessous. L'analyse statistique a été faite par un test exact de Fisher.

**Tableau 31**

| | Lot sérum Φ | Lot FSH | Lot CA5 seul | Statistiques |
|---|---|---|---|---|
| Nombre de brebis par lot | 9 | 11 | 7 | |
| Nombre de brebis ayant ovulé par lot | 4/9 (44%) | 4/11 (36%) | **7/7 (100%)** | ***, **p<0.0001** |
| Nombre de corps jaunes par brebis du lot | 0,67 ± 0,3 | 0,9 ± 0,5 | **1,5 ± 0,4** | NS, p=0.06 |
| Nombre de corps jaunes par brebis ayant ovulé | 1,5 ± 0,3 | 3 ± 1,4 | 1,5 ± 0,4 | NS |
| Moment du pic de LH (heures après retrait) | 64 ± 13 | 56 ± 7 | 57 ± 4,4 | NS |

Comparativement aux lots contrôle et FSH, les résultats obtenus dans le lot CA5 montrent un effet très significatif de l'anticorps injecté seul sur la réponse ovulatoire. En effet, 100% des femelles (7/7) ayant reçu une injection de 2 mg d'anticorps ont ovulé contre 44% et 36% respectivement pour le lot sérum Φ et le lot FSH (p<0.0001, test exact de Fisher). Le nombre de corps jaunes obtenu par femelle sur l'effectif total du lot est de même supérieur dans le lot "CA5" et de façon presque significative (p=0.06, test t de Mann-Whitney) : 1,5 corps jaune versus 0,9 (FSH) et 0,67 (sérum Φ) respectivement. Le nombre moyen de corps jaunes par femelle ayant ovulé n'est pas significativement différent entre les trois lots.

Le moment moyen d'apparition du pic de LH n'est pas significativement différent entre les trois lots. On observe malgré tout une tendance à moins de variabilité dans la venue du pic de LH (donc du moment de l'ovulation) dans le lot CA5 par rapport au lot FSH et surtout sérum Φ.

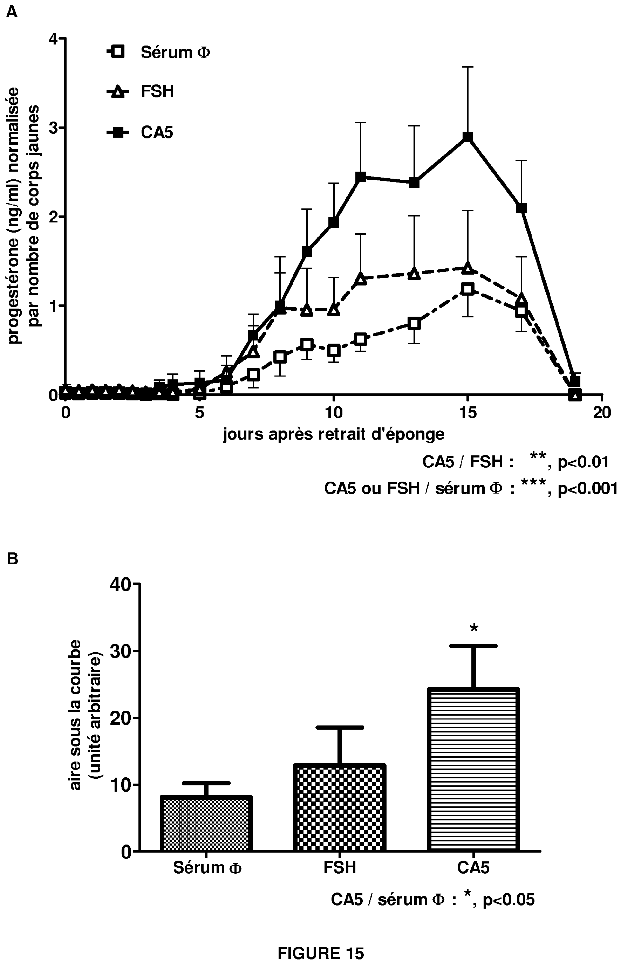

Le profil de sécrétion de la progestérone au cours de la phase lutéale suite à l'ovulation, est illustré dans la figure 15A. Pour chaque individu, les valeurs de concentration en progestérone (ng/ml) ont été normalisées par nombre de corps jaunes. Chaque courbe de la figure représente la moyenne des valeurs de progestérone obtenue chez les femelles de chaque lot. Un effet remarquable de l'anticorps CA5 a été observé sur l'intensité de la sécrétion de progestérone : une augmentation importante et très significative de la sécrétion de progestérone par corps jaune a été observée tout au long de la phase lutéale par rapport aux courbes du lot FSH et du lot sérum Φ. Les valeurs moyennes à J10 sont de 2,44 ng/ml, 1,3 et 0,62 ng/ml respectivement pour les lots CA5, FSH et sérum Φ, et de 2,88 - 1,42 et 1,18 ng/ml à J15. La comparaison des trois courbes a été faite par un test t non paramétrique apparié (test de Wilcoxon). La courbe du lot CA5 est significativement plus élevée et différente de la courbe du lot FSH (p < 0,01), de même avec la courbe du lot contrôle (p< 0,001). La courbe du lot FSH est significativement différente de celle du lot contrôle (p< 0,001).

Pour quantifier cette augmentation remarquable et constante du niveau de sécrétion de la progestérone par corps jaune tout au long de la phase lutéale du cycle chez les brebis sous stimulation de CA5, un calcul d'aire sous la courbe (AUC) a été réalisé avec le logiciel GraphPad Prism version 5.0. Les résultats sont illustrés sur la figure 15B et montrent que l'AUC de la courbe CA5 (24,20 unités) est significativement plus élevé d'un facteur 3 de l'AUC de la courbe sérum Φ (8,1) (p<0.05, test t non paramétrique de Mann-Whitney). A l'inverse, l'AUC de la courbe FSH n'est pas significativement différente de la courbe contrôle.

En conclusion, l'utilisation de l'anticorps CA5 sous forme d'une seule injection intra-musculaire de 2 mg a donné de façon très significative de meilleurs résultats qu'un traitement classique avec FSH en permettant :
1- l'induction de l'ovulation chez 100% des femelles stimulées
2- le développement de corps jaunes de meilleure qualité avec une sécrétion de progestérone très supérieure à celle observée suite à un traitement FSH et une mise en place très rapide dès J4. Il est à souligner que l'impact de cette propriété supplémentaire de CA5 par rapport à un traitement FSH est très important. En effet, la concentration plasmatique de progestérone est un facteur clé du développement embryonnaire, particulièrement dans ses phases précoces.

La mise en place plus rapide de la sécrétion de progestérone et le maintien d'un corps jaune fonctionnel plus efficace avec un niveau circulant de progestérone plus élevé est le garant d'un meilleur succès du développement embryonnaire précoce et du maintien de la gestation avec un risque d'avortement diminué.

L'ensemble des résultats indique que les anticorps potentialisants, particulièrement CA5, injecté *in vivo* chez la brebis est capable de complexer les hormones gonadotropes endogènes de l'animal et de potentialiser l'activité biologique des hormones propres à l'animal.

L'effet potentialisant de l'anticorps CA5 chez la brebis est capable d'induire une stimulation de l'ovaire plus forte que le traitement hormonal FSH classique : l'induction des ovulations est de 100% en saison sexuelle et dans tous les cas une augmentation importante de la concentration circulante de la progestérone, de 200 à 300% est maintenue tout au long de la phase lutéale. Cet effet supplémentaire est majeur pour diminuer les taux d'échec du développement embryonnaire progestagène dépendant et les risques d'avortement.

### EXEMPLE 5: MESURE IN VIVO DE L'EFFET POTENTIALISANT DU LIGAND CA5 DE L'INVENTION SUR LA BIOACTIVITE DES GONADOTROPINES ENDOGENES CHEZ LA GENISSE

Après avoir démontré et caractérisé l'effet potentialisant de l'anticorps monoclonal CA5 *in vivo* chez le rat et la brebis, l'objectif a été d'étudier son effet sur l'activité des gonadotropines endogènes chez un animal de plus grande taille : la génisse. Pour cela, un traitement ne comprenant que l'injection de l'anticorps seul a été mis au point chez la génisse Prim 'Holstein pour en évaluer son efficacité. Ces génisses étaient indemnes de toute stimulation préalable de l'ovaire, les animaux n'ayant reçu aucun traitement hormonal de stimulation de l'ovulation avec une gonadotropine préalablement à l'injection de l'anticorps.

Le protocole visant à évaluer l'effet potentialisant de l'anticorps CA5 a été réalisé sur des génisses Prim'Holstein de 20 à 22 mois, dont le cycle ovulatoire a été préalablement synchronisé par la pose d'un implant de progestagène (3,3 mg Norgestomet, Crestar® - MSD) pendant 7 jours. Une injection de GnRH (0,004 mg acétate de buséréline, Crestar® Pack - MSD) a été réalisé le jour de la pose de l'implant, suivi d'une injection de prostaglandines 24 heures avant le retrait de l'implant (Prosolvin® - Virbac).

Les animaux ont été séparés en deux lots :
- le lot "CA5" (n=4) a reçu deux injections de 11 mg d'anticorps CA5 purifié par voie intra-musculaire : la première 24 heures après la pose de l'implant et la deuxième 24 heures avant le retrait de l'implant de progestagène.
- le lot "contrôle" (n=3) a reçu deux injections de sérum physiologique, par voie intramusculaire, la première 24 heures après la pose de l'implant et la deuxième 24 heures avant le retrait de l'implant de progestagène.

L'effet potentialisant a été analysé en comparant la réponse ovulatoire (ovulation ou non) et la mise en place d'un corps jaune fonctionnel de bonne qualité (taille du corps jaune et amplitude de la sécrétion de progestérone) chez les génisses contrôles (lot sérum physiologique), et chez les génisses traitées par l'anticorps seul (lot CA5).

Un dosage de la LH et de l'œstradiol plasmatique a été réalisé par méthode ELISA afin de détecter et dater le pic pré-ovulatoire de LH, et suivre la sécrétion d'œstradiol. Pour évaluer la réponse ovulatoire et la taille des corps jaunes, des échographies ovariennes ont été réalisées quotidiennement. Enfin, pour évaluer la fonctionnalité et la qualité du corps jaune, un dosage ELISA quantitatif de progestérone a été réalisé à partir de prises de sang quotidiennes du jour de la pose de l'implant au 21^{ème} jour après le retrait de l'implant.

Les génisses ont été inséminées et un diagnostic de gestation a été effectué par échographie 35 jours après insémination.

Toutes les analyses statistiques ont été faites avec le logiciel GraphPad Prism Version 5.0 (GraphPad, San Diego, CA, USA).

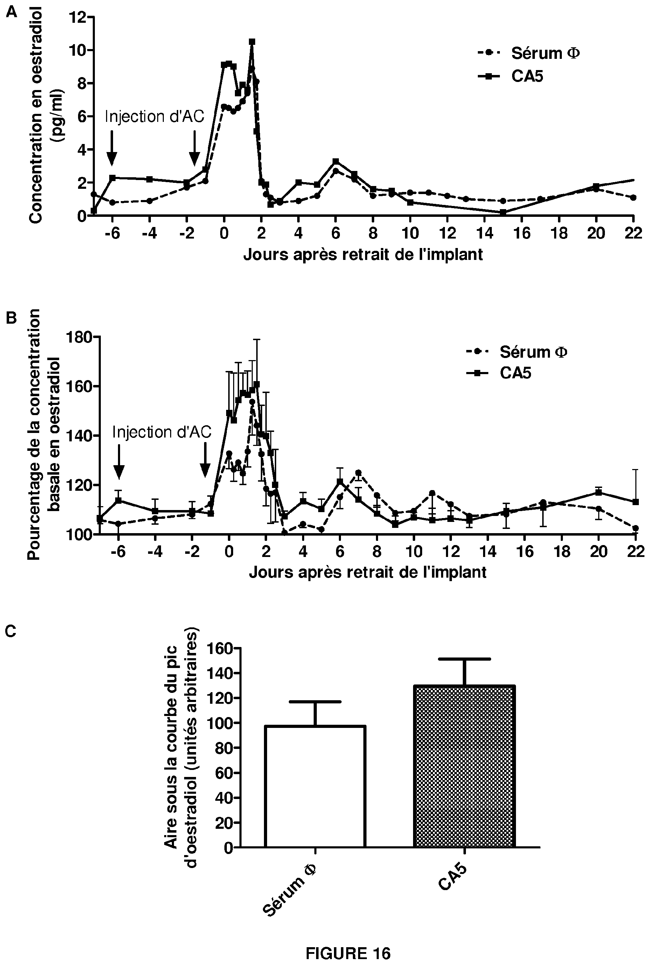

La réponse ovulatoire a tout d'abord été comparée dans les deux lots de génisses en mesurant les niveaux circulants d'oestradiol. Les résultats présentés sur la Figure 16A illustrent le profil de sécrétion de l'oestradiol chez deux génisses représentatives de chaque lot. Le pic d'oestradiol observé à J0 est d'une amplitude plus importante chez la traitée avec une concentration de 9,2 pg/ml contre 6,6 pg/ml chez la contrôle à J0 et de 10,5 pg/ml contre 8,8 pg/ml chez la contrôle à J1,5 (non-significatif). La moyenne des résultats des génisses de chaque lot est représentée en pourcentage de la concentration basale d'oestradiol mesurée à la pose de l'implant (Figure 16B). On retrouve les mêmes tendances avec un pic d'oestradiol dont l'amplitude tend à être plus importante dans le lot traité avec l'anticorps CA5 par rapport aux génisses du lot contrôle : au retrait de l'implant (J0) avec 149 ± 16% contre 133 ± 7% et, à J1,5 après retrait de l'implant, 161 ± 6% contre 144 ± 8% (non-significatif). Le niveau d'oestradiol entre J3 et J8 indique dans les deux lots un deuxième pic de sécrétion, plus réduit, traduisant une deuxième vague folliculaire. Dans le lot CA5, l'élévation de la concentration d'oestradiol tend à être plus précoce que dans le lot contrôle : à J6 elle est de 113 ± 3% chez les génisses traitées avec CA5 versus 104 ± 0,5 chez les contrôles.

L'analyse de l'aire sous la courbe du pic d'oestradiol dans les deux lots de génisses (Figure 16C) illustre également une tendance bien que non-significative, à obtenir un pic d'oestradiol plus élevé dans le lot traité avec CA5 (aire de 129,5 ± 20 unités) que dans le lot contrôle (aire de 97,5 ± 18 unités).

Globalement, les résultats montrent une tendance bien que non significative à avoir une meilleure sécrétion d'oestradiol chez les génisses traitées avec l'anticorps CA5, pouvant traduire une meilleure qualité de la phase folliculaire.

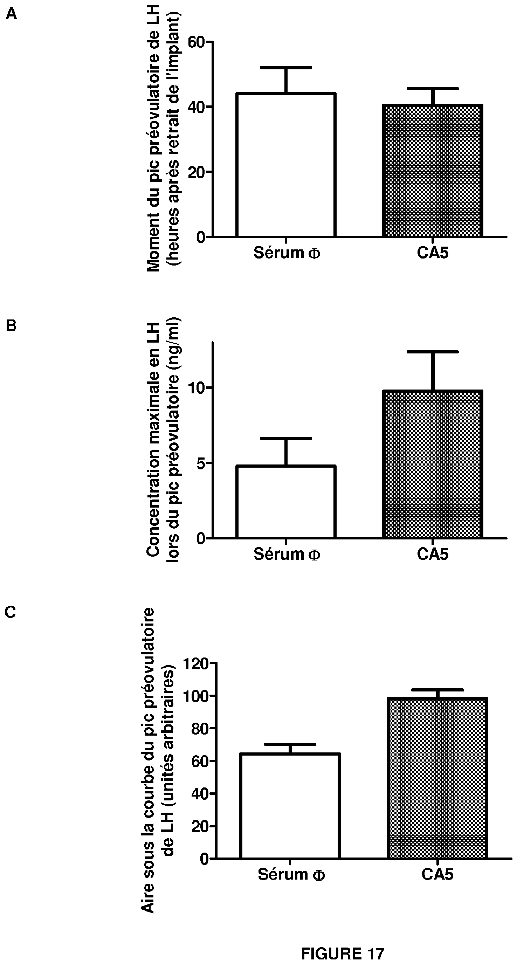

Les résultats concernant le pic préovulatoire de LH sont illustrés sur la Figure 17. Ils ne montrent aucune différence par rapport au moment d'apparition du pic de LH (Figure 17A) : dans le lot contrôle le pic de LH a eu lieu 44h ± 8h après retrait de l'implant contre 40,5h ± 5,1h dans le lot traité avec l'anticorps CA5. La concentration maximale du pic de LH est de 4,8 ± 1,8 ng/ml de LH dans le lot contrôle contre 9,8 ± 1,2 ng/ml chez les génisses traitées avec l'anticorps CA5 (Figure 17B). La concentration maximale de LH lors du pic pré ovulatoire tend à être supérieure dans le lot traité avec l'anticorps mais cette tendance n'est pas significative. Une même tendance est observée par rapport à l'aire sous la courbe du pic pré-ovulatoire de LH : elle est de 64,5 ± 5,8 unités arbitraires chez les génisses contrôles contre 98 ± 4,8 chez les génisses traitées avec l'anticorps CA5 (Figure 17C). Cette tendance à un pic de LH plus important chez les génisses traitées avec CA5 n'est cependant pas significative.

L'analyse échographique après l'ovulation a indiqué que toutes les génisses des deux lots avaient eu une mono-ovulation.

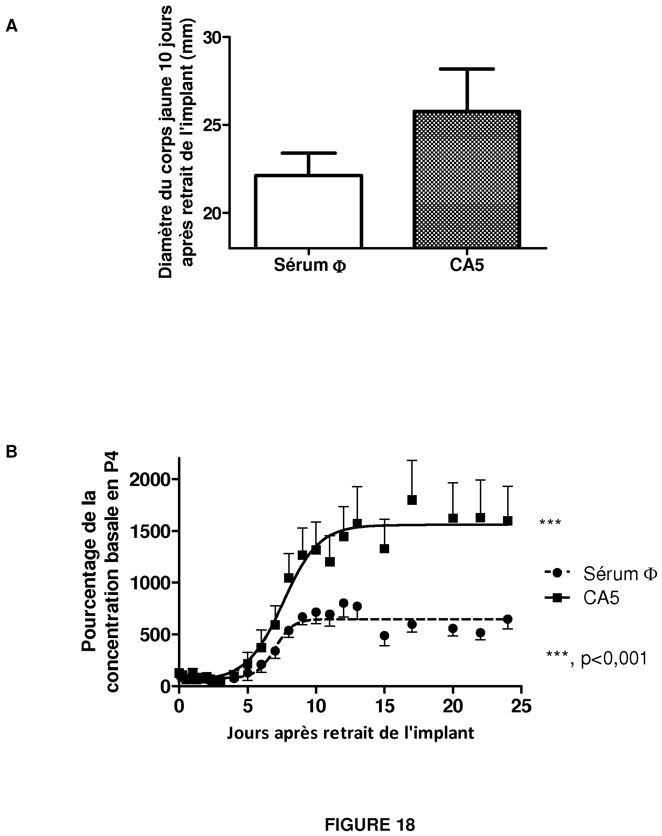

La qualité de la phase lutéale a été analysée ensuite en mesurant régulièrement la taille du corps jaune par échographie afin de suivre son développement. Les résultats illustrés sur la figure 18A indiquent que le lot de génisses traitées avec CA5 tendent à avoir un corps jaune de plus grande taille (26 ± 2 mm de diamètre) que le lot de génisses contrôles (22 ± 1 mm de diamètre). Cette différence n'est malgré tout pas significative.

La mesure de la concentration plasmatique de progestérone durant toute la phase lutéale est représentée dans la Figure 18B. Elle fait apparaître une différence très significative entre les deux lots (p<0,001). Le niveau circulant de progestérone est exprimé en pourcentage de la concentration basale mesurée au retrait de l'implant. A J9 après retrait, il est de 668 ± 85% dans le lot contrôle contre 1266 ± 254% dans le lot traité avec l'anticorps, soit une augmentation de 1,9 fois par rapport au lot contrôle. A J20 après retrait, il est de 646 ± 74% dans le lot contrôle contre 1598 ± 327% dans le lot traité avec l'anticorps, soit une augmentation de 2,47 fois par rapport au lot contrôle. Ces résultats traduisent une meilleure qualité du corps jaune avec une tendance à avoir un diamètre plus important et surtout une sécrétion plus élevée de progestérone chez les génisses traitées avec l'anticorps CA5. Il est à souligner qu'une meilleure qualité fonctionnelle du corps jaune au cours de la phase lutéale est prépondérante dans le succès de l'implantation de l'embryon et de son développement précoce. Elle diminue les risques d'avortements précoces.

Les diagnostics de gestation réalisés 35 jours après insémination artificielle ont indiqué que toutes les génisses dans chacun des lots étaient gestantes.

En conclusion, ces résultats montrent que les génisses traitées avec l'anticorps CA5 tendent à avoir une meilleure réponse oestrogénique bien que non significative pendant la phase folliculaire et ont une sécrétion significativement plus importante de progestérone pendant la phase lutéale.

### EXEMPLE 6: MESURE IN VIVO DE L'EFFET POTENTIALISANT DU LIGAND CA5 DE L'INVENTION SUR LA BIOACTIVITE DES GONADOTROPINES ENDOGENES CHEZ LA GUENON.

Après avoir démontré et caractérisé l'effet potentialisant de l'anticorps monoclonal CA5 *in vivo* chez le rat, la brebis et la génisse, son effet potentialisant a été étudié chez une espèce proche de l'humain : le singe Cynomolgus (*macaca fascicularis*). Pour cela, une étude a été réalisée sur des macaques pubères de 36 mois au moins, dans le but d'évaluer l'effet potentialisant de l'anticorps sur la FSH humaine et sur l'hormone endogène du macaque traité.

Pour cela, une étude a été réalisée sur quatre macaques pubères âgés de 36 mois au moins. L'anticorps CA5 a été injecté soit 20 minutes après une injection de FSH exogène, soit seul. Au premier jour des règles, les macaques ont reçu une injection de 1,5 mg de préparation de GnRH à libération prolongée (Décapeptyl® L.P. 3 mg - IPSEN Pharma) par voie intra-musculaire. Quinze jours après, les quatre guenons ont reçu un traitement différent :
- animal traité avec 25 UI pendant 8 jours (lot "25UI X 8") : une injection quotidienne de 25 UI de FSH humaine (Gonal-f® stylo pré-rempli - Merck Serono) a été réalisée par voie sous-cutanée, pendant 8 jours
- animal traité avec 25 UI un jour (lot "25UI X 1") : une injection unique de 25 UI de FSH humaine (Gonal-f® stylo pré-rempli - Merck Serono) par voie sous-cutanée, a été réalisée le premier jour de traitement
- animal traité avec CA5 + hFSH (lot "CA5+hFSH") : une injection unique d'anticorps CA5 (80µg) a été réalisé par voie sous-cutanée 20 minutes après l'injection de 25 UI de FSH humaine (Gonal-f® stylo pré-rempli - Merck Serono), le premier jour de traitement seulement
- animal traité avec CA5 seul (lot "CA5") : une injection unique d'anticorps CA5 seul (88,5µg), a été réalisée par voie sous-cutanée sans injection d'hormone exogène, le premier jour de traitement seulement.

L'effet potentialisant a été analysé en comparant la croissance folliculaire induite par le traitement. Pour cela, des échographies ovariennes transabdominales ont été pratiquées toutes les 48 heures afin de compter les follicules et mesurer leur surface (exprimée en mm²).

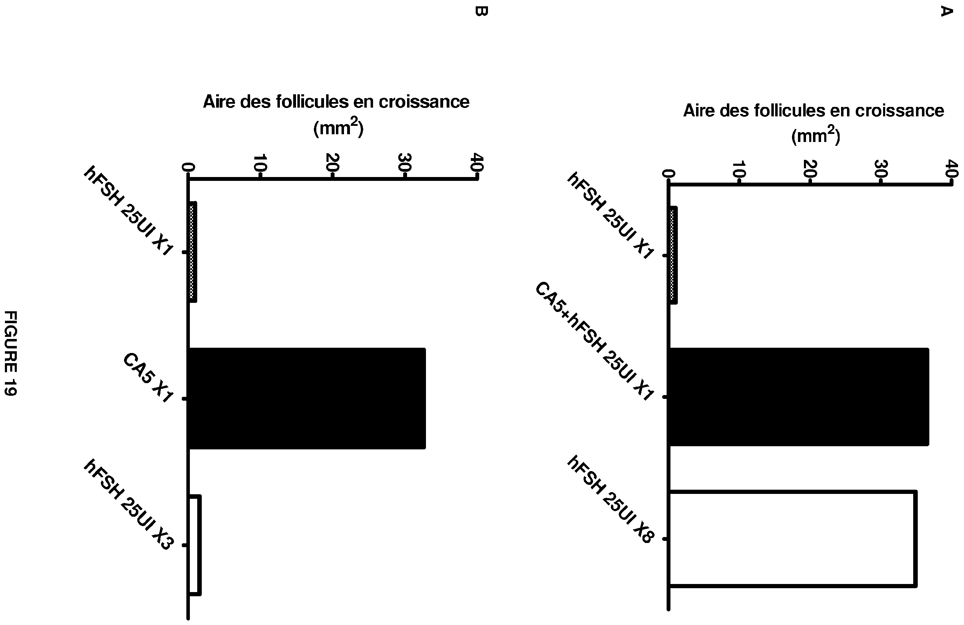

L'effet obtenu avec une injection de l'anticorps CA5 réalisée 20 minutes après l'injection de 25 UI de hFSH (lot "CA5+hFSH") a été comparé à celui d'une injection de 25 UI de hFSH (lot "25UI X1") et à celui de 8 injections quotidiennes de 25 UI de hFSH (lot "25UI X8"). Les résultats illustrés sur la Figure 19A montrent qu'au 9^{ème} jour après le début du traitement FSH, la guenon traitée avec une seule injection de 25 UI de hFSH ne présente aucun follicule stimulé (aire sous la courbe, nulle). A l'inverse, la guenon traitée une seule fois avec le mélange "CA5+hFSH" le premier jour de traitement, présentait 8 follicules en croissance avec une aire totale des follicules de 37 mm² égale à celle mesurée chez la guenon ayant reçu 8 injections de hFSH ("25UI X8") qui était de 35 mm² avec 11 follicules stimulés. Une seule injection du complexe CA5+hFSH 25 UI a donc stimulé les ovaires aussi efficacement que 8 injections de 25 UI de hFSH. Ce résultat met en évidence un effet potentialisant de CA5, *in vivo* chez la guenon, se traduisant par une stimulation folliculaire. En effet, la demi-vie de la FSH étant inférieure à 1 heure, les effets observés avec CA5 ne peuvent être attribués uniquement à un effet sur la hFSH injectée, mais reflètent également un effet sur la FSH endogène de la guenon.

L'effet d'une injection de l'anticorps CA5 injecté seul ("CA5") a ensuite été étudié et comparé à celui d'une injection de 25 UI de hFSH ("25UI X1") et à celui de 3 injections quotidiennes de 25 UI de hFSH (lot "25UI X3"). Un effet stimulant de l'anticorps injecté seul a été observé de façon optimale 3 jours après son injection. Les résultats illustrés sur la Figure 19B montrent qu'au 3éme jour du traitement la guenon ayant reçu une seule injection d'anticorps seul ("CA5") présente une stimulation ovarienne avec 5 follicules en croissance représentant une aire de 32,6 mm². Comparativement, la guenon ayant reçu trois injections de 25 UI de hFSH ("25UI X3") ne présentait qu'une très faible stimulation avec 1 seul follicule en croissance dont l'aire était de 1,6 mm². Au-delà du 3^{ème} jour de traitement, la stimulation ovarienne induite par CA5 seul ne s'est pas maintenue et les follicules stimulés ont disparu. Une nouvelle injection d'anticorps ou l'ajustement de la dose injectée à J1 auraient probablement était nécessaires pour maintenir l'effet de stimulation sur les ovaires. Ce résultat indique néanmoins que l'anticorps CA5 injecté seul a été capable de complexer la FSH endogène de la guenon et de potentialiser son activité biologique suffisamment pour induire un début de stimulation ovarienne.

### EXEMPLE 7: PREDICTION DE L'EPITOPE RECONNU PAR LES LIGANDS CA5 et CH10 DE L'INVENTION, ET PREDICTION DE LEUR PARATOPE.

L'épitope respectif des anticorps CA5 et CH10 a été déterminé sur les hormones gonadotropes de différentes espèces en utilisant un algorithme d'amarrage des protéines basé sur une modélisation de la structure des protéines par un diagramme de Voronoï et une optimisation par différentes méthodes d'apprentissages évolutionnaires de fonctions de score permettant de différencier les conformations natives et non-natives (Bernauer et al., Bioinformatics 2007, 5:555) [14], (Bernauer et al., Bioinformatics 2008, 24:652) [15], (Bourquard et al., PLoS One 2011, 6:e18541) [16] et (Bourquard et al., Sci. Reports 2015, 5 :10760) [17].

Chaque anticorps a été amarré avec la FSH humaine (hFSH), la LH humaine (hLH), la CG humaine (hCG), la FSH ovine (oFSH) et la LH ovine (oLH), la FSH porcine (pFSH) et la LH porcine (pLH). Les structures cristallographiques de la hFSH et de la hCG sont disponibles dans la Protein Data Bank (PDB) : 4MQW et 1QFW respectivement. La structure de la FSH humaine complexée avec le domaine extracellulaire du récepteur FSH humain a été utilisé (Fan et Hendrickson, Nature 2005, 433:269) [18]. Pour les autres hormones (hLH, oFSH, oLH, pFSH et pLH), des modèles par homologie ont été réalisés puis utilisés pour l'amarrage.

### 1/ Epitope et paratope du ligand CA5.

La structure 3D de l'anticorps CA5 n'étant pas disponible, l'étude a été faite à partir des séquences des fragments monovalents VH et VL de CA5. Pour cela, des modèles par homologie des parties variables ont été réalisés. Les modèles des VH et VL ont été réalisés séparément, à partir de structures différentes, et leur orientation relative a été déterminée à partir de la structure ayant servi de support pour la modélisation du VH. Les structures utilisées pour les modèles par homologie sont disponibles dans la Protein Data Bank (PDB) : 3OKK pour le VH de CA5 et 3MBX pour le VL de CA5.

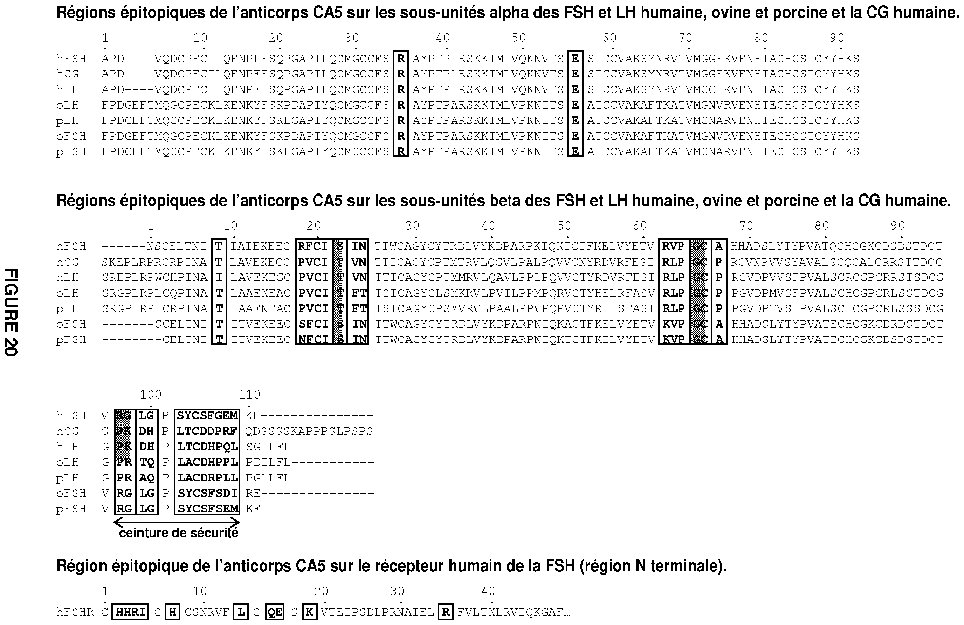

Les résultats d'amarrage sont illustrés sur la Figure 20. Il apparaît que le ligand CA5 s'amarre de manière similaire sur les sept hormones cibles. L'épitope est défini par plusieurs régions situées de façon discontinue sur la sous-unité béta essentiellement et pour deux résidus sur la sous-unité alpha des gonadotropines étudiées. L'épitope concerne également dix résidus de l'ectodomaine du récepteur FSH humain. L'épitope du ligand CA5 est donc très conformationnel : il est constitué par plusieurs régions séquentielles de l'hormone et d'une séquence du récepteur, qui se trouvent spatialement proches dans la conformation native de l'hormone et du récepteur activé.

Les différents résidus de l'hormone et du récepteur impliqués dans l'interface avec le ligand CA5 sont entourés par des rectangles sur la figure 20. Les cinq résidus notés en grisé sur la sous-unité béta de hFSH sont impliqués dans l'interaction principale et ont un rôle majeur dans la reconnaissance anticorps/antigène : il s'agit de la sérine en position 22 (Ser22), de la glycine en position 65 (Gly65), de la cystéine en position 66 (Cys66), de l'arginine 97 (Arg97) et de la Glycine 98 (Gly98) sur la sous-unité béta de hFSH. Les résidus Gly65 et Cys66 sont présents dans la séquence de toutes les autres hormones cibles.

Le ligand CA5 reconnaît également les résidus 97 à 100 et 102 à 109 dans l'extrémité C-terminale de la sous-unité béta FSH. Cette région qui constitue une ceinture de sécurité ("seat belt"), joue un rôle majeur pour stabiliser l'association du dimère alpha/béta de l'hormone. Il semble donc que la liaison du ligand CA5 sur l'hormone sécurise la fermeture de la ceinture de sécurité et contribue ainsi à la stabilité du dimère, indispensable à la bioactivité de l'hormone (seul le dimère est actif).

L'anticorps CA5 est essentiellement dirigé contre la sous-unité béta de la hFSH.

Seuls deux résidus de la sous-unité alpha sont impliqués dans l'interface : il s'agit des résidus arginine en position 35 (Arg35) et acide glutamique en position 56 (Glu56) spatialement proches dans l'hormone native. Leur rôle est de fixer l'extrémité C-terminale de la sous-unité béta afin de maintenir la "ceinture de sécurité" autour de la sous-unité alpha. Ces deux résidus sont présents et reconnus de façon constante dans toutes les hormones cibles. Ils jouent un rôle très important dans la bioactivité de l'hormone.

Une autre caractéristique de l'épitope de l'anticorps CA5 est l'implication des résidus His2-His3-Arg4-Ile5, His7, Leu14, Gln16-Glu17, Lys19 et Arg35 de la région N-terminale du récepteur FSH humain, dans l'interface reconnue par l'anticorps CA5.

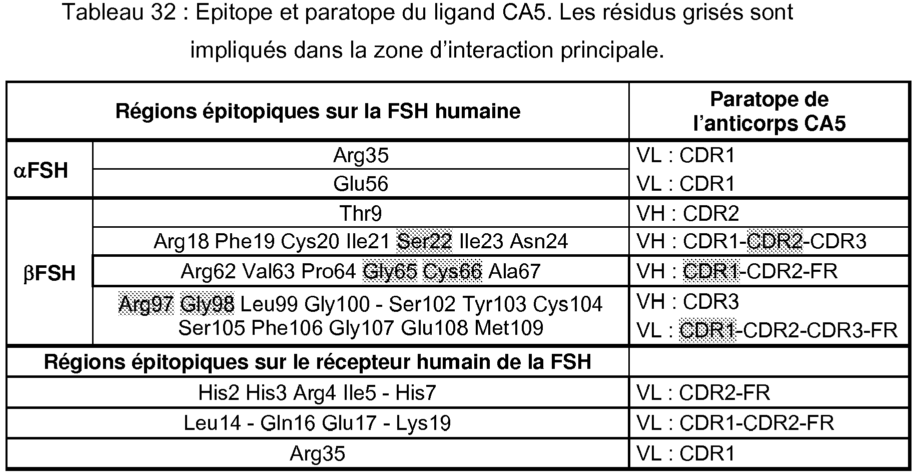

Le tableau 32, illustre les différentes régions constituant l'épitope du ligand CA5 et celles constituant son paratope.

Les deux chaînes VH et VL sont impliquées dans la reconnaissance de l'hormone, par leurs trois CDR et certains résidus de leurs frameworks.

Les cinq résidus impliqués dans l'interaction principale sont reconnus par l'Asn53 du CDR2 de VH, les résidus Asp31, Phe27 et Thr28 du CDR1 de VH et les résidus Ser33 et Asn34 du CDR1 de VL.

Seule la chaîne VL est impliquée dans la reconnaissance de l'ectodomaine du récepteur FSH, particulièrement les résidus Gln35 et Lys36 de son CDR1, le résidu Ser58 de son CDR2 et plusieurs résidus du framework 3.

En conclusion, le ligand CA5 reconnaît un épitope conformationnel impliquant la sous-unité alpha, la sous-unité béta de la hFSH et son extrémité C-terminale formant la ceinture de sécurité, ainsi que l'ectodomaine du récepteur FSH. La chaîne VL est la seule impliquée dans l'interaction avec l'ectodomaine du récepteur. Les deux chaînes VH et VL sont impliquées dans l'interaction avec les sous-unités de l'hormone. L'épitope conformationnel du ligand CA5 lui permet, d'une part, de stabiliser l'association du dimère de l'hormone et, d'autre part, de stabiliser la liaison de l'hormone sur son récepteur. Ces deux mécanismes sont complémentaires et fondamentaux pour aboutir à une meilleure interaction de l'hormone sur son récepteur et obtenir ainsi une meilleure efficacité. Ils constitueraient ainsi les bases mécanistiques de l'effet potentialisant du ligand CA5 sur les gonadotropines.

### 2/ Epitope et paratope du ligand CH10.

La structure 3D de l'anticorps CH10 n'étant pas disponible, l'étude a été faite à partir des séquences des fragments monovalents VH et VL de CH10. Pour cela, des modèles par homologie des parties variables ont été réalisés. Les modèles des VH et VL ont été réalisés séparément, à partir de structures différentes, et leur orientation relative a été déterminée à partir de la structure ayant servi de support pour la modélisation du VH. Les structures utilisées pour les modèles par homologie sont disponibles dans la Protein Data Bank (PDB) : 4QNP pour le VH de CH10 et 3D85 pour le VL de CH10.

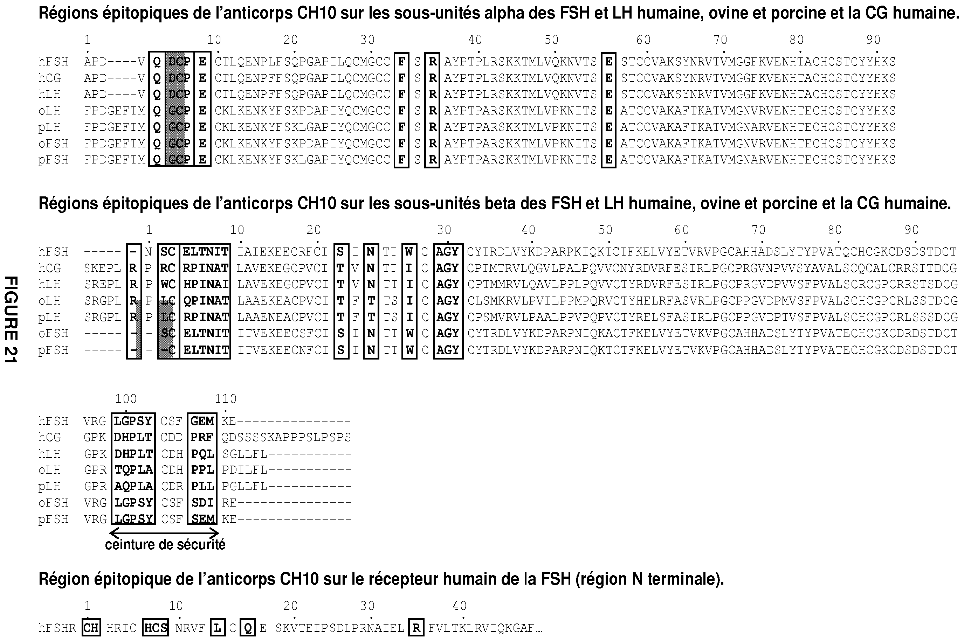

Les résultats d'amarrage sont illustrés sur la Figure 21. Il apparaît que le ligand CH10 s'amarre de manière similaire sur les sept hormones cibles. L'épitope est défini par plusieurs régions situées de façon discontinue sur les sous-unités alpha et béta de la FSH et concerne également huit résidus de l'extrémité N-terminale du récepteur FSH humain. L'épitope du ligand CH10 est donc conformationnel : il est constitué de plusieurs régions séquentielles de l'hormone et d'une séquence dans l'extrémité N-terminale du récepteur FSH. L'ensemble de ces régions se trouvent spatialement proches dans la conformation native de l'hormone et du récepteur activé.

Les différents résidus de l'hormone et du récepteur impliqués dans l'interface avec le ligand CH10 sont entourés par des rectangles sur la figure 21. Les quatre résidus notés en grisé sur la sous-unité alpha (Asp6-Cys7-Pro8 et Glu56 sur hFSH) et les deux résidus notés en grisé sur la sous-unité béta (Ser2-Cys3 sur hSFH) sont impliqués dans l'interaction principale et ont un rôle majeur dans la reconnaissance anticorps/antigène. Parmi les autres résidus impliqués dans l'interface, ceux situés sur la partie C-terminale de la sous-unité béta sont impliqués dans la région appelée ceinture de sécurité » ("seat belt") dont le rôle est de stabiliser l'association du dimère alpha/béta de l'hormone. Il semble donc que la liaison du ligand CH10 sur l'hormone sécurise la fermeture de la ceinture de sécurité et contribue ainsi à la stabilité du dimère, indispensable à la bioactivité de l'hormone. De plus le ligand CH10 reconnaît de façon majeure le résidu Glu56 sur la sous-unité alpha, qui est impliqué dans la fixation de la "ceinture de sécurité" sur la sous-unité alpha.

Une autre caractéristique de l'épitope de l'anticorps CH10 est l'implication des résidus Cys1-His2, His7-Cys8-Ser9, Lys14, Gln16 et Arg35 de la région N-terminale du récepteur FSH humain dans l'interface reconnue par l'anticorps.

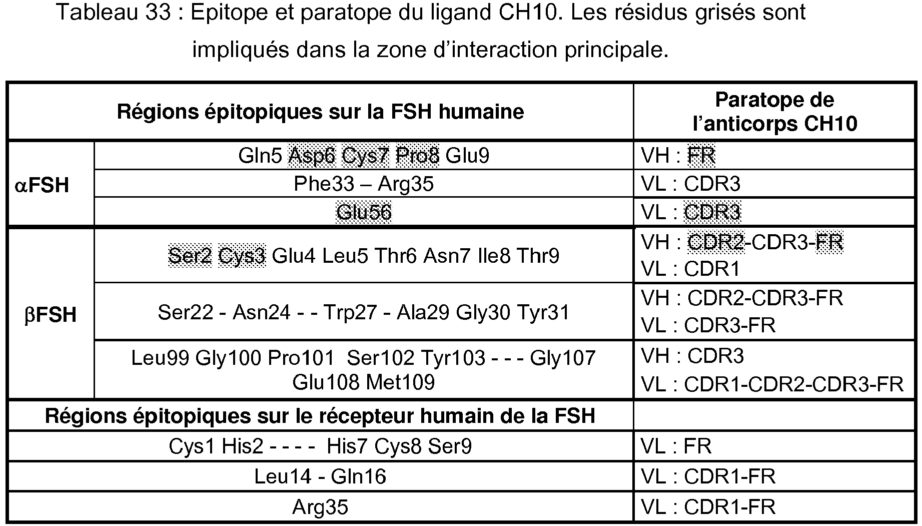

Le tableau 33, illustre les différentes régions constituant l'épitope du ligand CH10 et celles constituant son paratope.

Les CDR2 et CDR3 de la chaîne VH et les CDR1, CDR2 et CDR3 de la chaîne VL sont impliqués dans la reconnaissance de l'hormone, ainsi que certains résidus du framework 3 de la chaine VH et des frameworks 1 et 2 de la chaîne VL.

Les résidus impliqués dans l'interaction principale sont reconnus par les résidus Thr60 du CDR2, Tyr61-Tyr62-Asp64-Lys67 du framework 3 de la chaîne VH et les résidus His92 et Ser93 du CDR3 da la chaîne VL.

Seule la chaîne VL est impliquée dans l'interaction sur l'ectodomaine du récepteur par son CDR1 et ses frameworks 1 et 2.

### EXEMPLE 8: CONSTRUCTION, PRODUCTION ET CARACTERISATION DE DIFFERENTS FRAGMENTS DU LIGAND CA5 DE L'INVENTION.

Différents fragments de l'anticorps CA5 ont été construits afin d'évaluer leur capacité à potentialiser l'activité biologique de la FSH ovine et humaine (Gonalf® Merck Serono). Il a été produit un fragment comportant la chaîne variable légère seule appelé "VL CA5", un fragment comportant la chaîne variable lourde seule appelé "VH CA5" et un "scFv CA5 inverse" construit dans un ordre VL-VH inversé par rapport à la séquence VH-VL (SEQ ID N0 : 19 et SEQ ID N0 : 20) du scFv CA5 décrit dans l'exemple 1, paragraphe 4 de la présente invention.

### 1/ Construction et production des fragments d'anticorps

Les gènes de synthèse codant pour les fragments VL CA5 et scFv CA5 inverse dérivés de l'anticorps CA5 ont été synthétisés par ATG:Biosynthetics GmbH (Allemagne). Le scFv CA5 inverse est constitué de la fusion VL CA5 du scfv CA5-linker-VH CA5 du scFv CA5 (SEQ ID N°19). Chaque gène de synthèse est conçu par la fusion de la séquence du plasmide pSW1 [7], comprise entre le site Hindlll et la fin de la séquence codant pour la protéine PelB et la séquence de la protéine d'intérêt à synthétiser (SEQ ID N° 44 et SEQ ID N°48), flanquée du site de restriction Xhol. Les séquences sont insérées entre les sites Hindlll et Xhol du plasmide pSW1. Les codons ont été optimisés pour l'expression en *E. coli.*

Le plasmide d'expression pSW1-VH CA5 a été obtenu par l'insertion, dans le plasmide pSW1 [7] aux sites Pstl-Xhol, du fragment issu de la digestion par ces mêmes enzymes du plasmide pSW1 scFv CA5 inverse.

Après contrôle par séquençage de la qualité des constructions, les plasmides pSW1-VL CA5, pSW1-VH CA5 et pSW1 scFv CA5 inverse ont été transformés par choc thermique en bactéries HB2151 (T53040, Interchim, France) rendues compétentes [8].

**Tableau 34: Séquences nucléotidique et peptidique du VL CA5.**

| VL CA5 | |
|---|---|
| Séquence nucléotidique SEQ ID NO : 44 | |
| | |
| Séquence peptidique SEQ ID NO : 45 | |

**Tableau 35: Séquences nucléotidique et peptidique du VH CA5.**

| VH CA5 | |
|---|---|
| Séquence nucléotidique SEQ ID NO : 46 | |
| Séquence peptidique SEQ ID NO : 47 | |

**Tableau 36: Séquences nucléotidique et peptidique du scFv CA5 inverse.**

| scFv CA5 inverse | |
|---|---|
| Séquence nucléotidique SEQ ID NO : 48 | |
| | |
| Séquence peptidique SEQ ID NO : 49 | |

La production des fragments a été réalisée selon le procédé préalablement décrit dans l'exemple 1 de la présente invention.

### 2/ Mesure in vitro de l'effet des fragments VL CA5, VH CA5 et scFv CA5 inverse sur la bioactivité de la FSH

L'effet *in vitro* des fragments "VL CA5", "VH CA5" et "scFv CA5 inverse" sur la bioactivité de la FSH ovine et humaine a été étudié avec la lignée cellulaire HEK293 transfectée de façon stable avec le récepteur FSH humain et le système Glosensor® selon le protocole préalablement décrit dans l'exemple 2 de la présente invention. Les fragments "VL CA5" et "VH CA5" seuls ou en mélange ont été testés à 40 nM chacun. Le scFv CA5 inverse a été testé à la concentration de 80 nM tout comme le scFv CA5 de référence. La FSH ovine et la FSH humaine (Gonal f®) ont été testées à 0,1 nM.

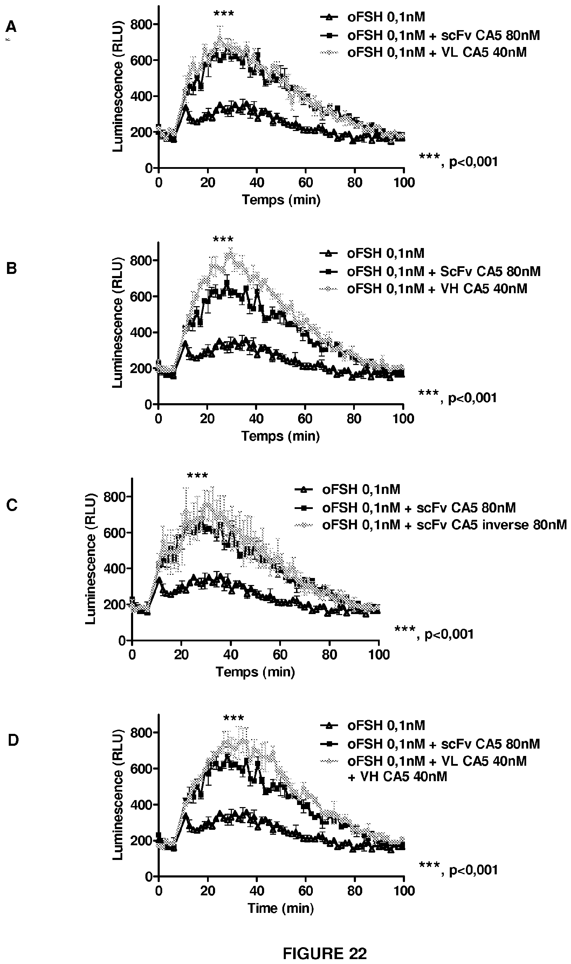

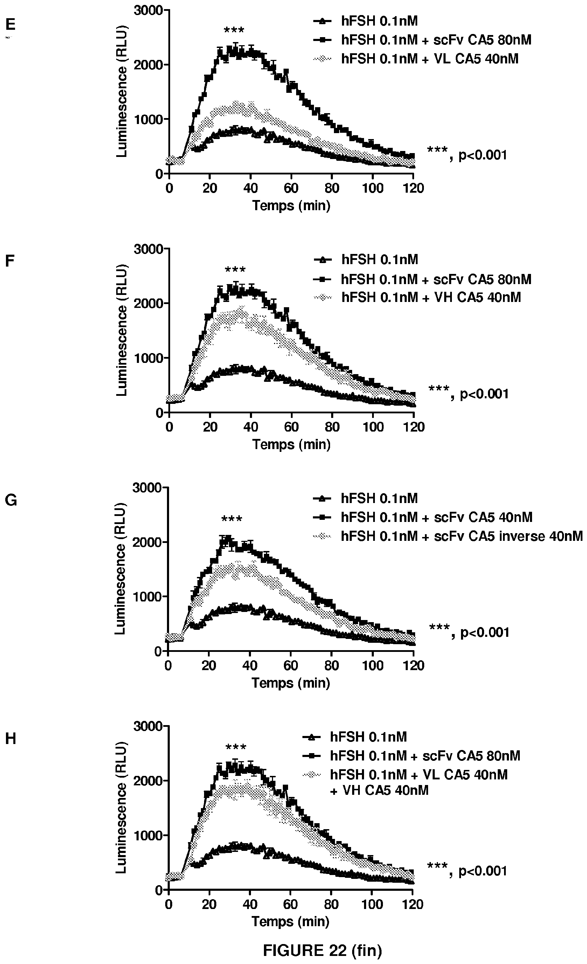

La figure 22 illustre les courbes de cinétique de production d'AMPc exprimées en unités relatives de luminescence en fonction du temps (en minutes) obtenues en présence de 0,1 nM de FSH ovine (oFSH) seule ou complexée aux différents fragments de CA5. Quatre conditions ont été comparées à la oFSH seule : le complexe oFSH+ VL CA5 (Figure 22A), le complexe oFSH+VH CA5 (Figure 22B), le complexe oFSH+scFv CA5 inverse (Figure 22C) et enfin le complexe de la oFSH avec un mélange équimolaire de 40 nM de VL CA5 et de 40 nM de VH CA5 (Figure 22D). On observe que dans tous les cas, un effet potentalisant très significatif est obtenu (p<0,001), augmentant la réponse cellulaire à 30 mn de stimulation d'un facteur 2,1 à 2,4 fois par rapport à la réponse induite par la FSH seule. Ces résultats indiquent que les fragments VH et VL seuls sont capables d'exercer un effet potentialisant sur la bioactivité de la oFSH au même titre que le scFv entier. Ils valident l'implication des deux chaînes variables dans l'effet potentialisant, décrit dans le modèle de l'exemple 7 de la présente invention. Le mélange VH CA5 + VL CA5 de même que le scFv CA5 inverse (VL-VH) exerce le même effet potentialisant que le scFv CA5 d'origine (VH-VL).

La même étude a été réalisée avec la FSH humaine (Gonal f®) à la concentration de 0,1 nM, seule ou complexée aux différents fragments de CA5. Quatre conditions ont été comparées à la hFSH seule : le complexe hFSH+ VL CA5 (Figure 22E), le complexe hFSH+VH CA5 (Figure 22F), le complexe hFSH+scFv CA5 inverse (Figure 22G) et enfin le complexe de la hFSH avec un mélange équimolaire de 40 nM de VL CA5 et de 40 nM de VH CA5 (Figure 22H). On observe que le fragment "VL CA5" à la concentration de 40 nM complexé à hFSH, n'exerce pas d'effet potentialisant significatif contrairement au fragment "VH CA5" qui amplifie de façon très significative (p<0,001) la réponse cellulaire maximale de 225% de façon comparable au scFv CA5 (275% d'augmentation) par rapport à une stimulation avec hFSH seule (Figures 22E et 22F). De même, le scFv CA5 inverse "VL-VH CA5" (Figure 22G) et le mélange des deux chaînes VH + VL (Figure 22H) complexés à la hFSH induisent une augmentation significative (p<0,001) de la réponse cellulaire de 225% et 230% respectivement, proche de celle obtenue avec le scFv CA5 complexé à hFSH (augmentation entre 250 et 260%). Ces résultats indiquent que le mélange VL CA5 + VH CA5 de même que le scFv CA5 inverse complexés à la hFSH induisent un effet potentialisant significatif proche de celui induit par le scFv CA5 de référence. Seul le fragment VH exerce un effet potentialisant significatif sur hFSH dans cet essai à la différence de VL CA5.

### 3/ Mesure in vivo de l'effet potentialisant des fragments VL CA5, VH CA5 et scFv CA5 inverse sur la bioactivité de la FSH chez la ratte

Après avoir été caractérisé *in vitro,* l'effet potentialisant des différents fragments de CA5 a été caractérisé *in vivo,* chez le rat femelle pour caractériser leur effet sur la bioactivité de la FSH.

Pour mesurer la bioactivité FSH, le protocole utilisé a été celui du dosage biologique de Steelman et Pohley (Steelman SL, Pohley FM. Endocrinology, 53 :604-616. 1953) [12] tel que décrit dans l'exemple 3 de la présente invention.

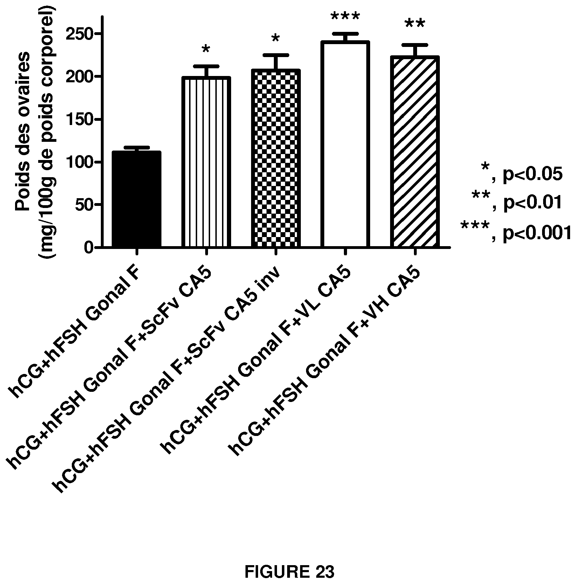

La figure 23 illustre l'effet des différents fragments sur la bioactivité de la hFSH. Chaque lot comportait 5 rattes. Le lot traité avec la hFSH complexée au scFv CA5 inverse a donné un poids moyen des ovaires de 210mg, identique à celui du lot traité avec le scFv CA5 complexé à hFSH (200mg) soit une augmentation de 190% par rapport au lot ayant reçu le traitement hormonal seul (p<0,05). Les lots traités avec les fragments VL CA5 ou VH CA5 complexés à la hFSH ont eu un poids moyen des ovaires de 240 mg et 230 mg respectivement soit une augmentation de 218% et 209% respectivement par rapport au lot ayant reçu le traitement hormonal seul (p<0,001 et p<0,01 respectivement).

Ces résultats démontrent significativement que l'ordre de construction du scFv (VL-VH versus VH-VL) complexé à la hFSH n'affecte pas les propriétés de potentialisation du scFv sur la bioactivité de la FSH. Ces résultats démontrent également de façon très significative que les chaînes VH CA5 ou VL CA5 complexées à l'hormone sont capables elles aussi de potentialiser la bioactivité de l'hormone. Ceci traduit l'implication respective des deux chaînes dans cet effet comme le prédit le modèle d'interaction décrit dans l'exemple 7 de la présente invention.

### Listes des références

1- Brevet EP 1518863
2- Demande Internationale WO 2012/066519
3- Brochet et al., Nucl. Acids Res., 36 : W503-508, 2008
4- Giudicelli et al., Cold Spring Harb Protoc., 2011(6) : 695-715, 2011
5- Giudicelli et al., Nucl. Acids Res., 33 : D256-261, 2005
6- Corpet, Nucl. Acids Res., 16(22) : 10881-10890, 1988
7- Ward et al. Nature, 341 : 544-546, 1989)
8- Li et al., Afr. J. Biotechnol., 9(50) : 8549-8554, 2010
9- Chopineau et al., Mol. Cell Endocrinol., 92(2) : 229-239, 1993
10- Wehbi et al., Endocrinology, 151(6) : 2788-2799, 2010
11- Reverchon et al., Human Reprod., 27(6) : 1790-1800, 2012
12- Steelman SL, Pohley FM., Endocrinology, 53 :604-616, 1953
13- Scobey et al, Reprod. Biol. Endocr. 3 :61, 2005
14- Bernauer et al., Bioinformatics, 5:555, 2007
15- Bernauer et al., Bioinformatics, 24:652, 2008
16- Bourquard et al., PLoS One, 6:e18541, 2011
17- Bourquard et al., Sci. Reports, 5 :10760, 2015
18- Fan et Hendrickson, Nature, 433:269, 2005.

## Revendications

1. Ligand de l'hormone folliculo-stimulante (FSH) potentialisant la bioactivité de la FSH, de l'hormone lutéinisante (LH) et de la gonadotropine chorionique (CG), **caractérisé en ce que** ledit ligand est un anticorps ou un fragment de celui-ci et **en ce que** :
le domaine variable de la chaine lourde contient les CDRs suivants :
- VH-CDR1, défini par la séquence GFTFSDFY (SEQ ID NO : 9) ;
- VH-CDR2, défini par la séquence SRNKAKDYTT (SEQ ID NO : 10) ;
- VH-CDR3, défini par la séquence ARDARFAY (SEQ ID NO : 11) ; et
le domaine variable de la chaine légère contient les CDRs suivants :
- VL-CDR1, défini par la séquence QSLLYSSNQKNY (SEQ ID NO : 12) ;
- VL-CDR2, défini par la séquence WAS ;
- VL-CDR3, défini par la séquence QQYYSYPRT (SEQ ID NO : 13).

2. Ligand de l'hormone folliculo-stimulante (FSH) potentialisant la bioactivité de la FSH, de l'hormone lutéinisante (LH) et de la gonadotropine chorionique (CG), **caractérisé en ce que** ledit ligand est un anticorps ou un fragment de celui-ci et **en ce que** :
- le domaine variable de la chaine lourde contient la séquence SEQ ID NO : 2 ou 47 ;
et/ou
- le domaine variable de la chaine légère contient la séquence SEQ ID NO : 4 ou 45.

3. Ligand de l'hormone folliculo-stimulante (FSH) potentialisant la bioactivité de la FSH, de l'hormone lutéinisante (LH) et de la gonadotropine chorionique (CG), **caractérisé en ce que** ledit ligand est un anticorps ou un fragment de celui-ci et **en ce que** :
le domaine variable de la chaine lourde contient les CDRs suivants :
- VH-CDR1, défini par la séquence GFTFNTYA (SEQ ID NO : 14) ;
- VH-CDR2, défini par la séquence IRSKSNNYAT (SEQ ID NO : 15) ;
- VH-CDR3, défini par la séquence VRQDYYGSSYFDY (SEQ ID NO : 16) ; et
le domaine variable de la chaine légère contient les CDRs suivants :
- VL-CDR1, défini par la séquence QSISDY (SEQ ID NO : 17) ;
- VL-CDR2, défini par la séquence YAS ;
- VL-CDR3, défini par la séquence QNGHSFPYT (SEQ ID NO : 18).

4. Ligand de l'hormone folliculo-stimulante (FSH) potentialisant la bioactivité de la FSH, de l'hormone lutéinisante (LH) et de la gonadotropine chorionique (CG), **caractérisé en ce que** ledit ligand est un anticorps ou un fragment de celui-ci et **en ce que** :
- le domaine variable de la chaine lourde contient la séquence SEQ ID NO : 6 ;
et/ou
- le domaine variable de la chaine légère contient la séquence SEQ ID NO : 8.

5. Ligand selon l'une quelconque des revendications 1 à 4, **caractérisé en ce que** le ligand est choisi dans le groupe constitué de Fab, Fab', F(ab')2, Fv, dsFv, scFv, diabodies, triabodies, tétrabodies.

6. Ligand selon la revendication 1, **caractérisé en ce que** le ligand est l'anticorps monoclonal CA5 produit par l'hybridome CNCM I-4801.

7. Ligand selon la revendication 3, **caractérisé en ce que** le ligand est l'anticorps monoclonal CH10 produit par l'hybridome CNCM I-4802.

8. Ligand selon la revendication 5, **caractérisé en ce que** la séquence peptidique du scFv est la séquence SEQ ID NO : 20 ou SEQ ID NO : 22.

9. Ligand selon l'une quelconque des revendications 1 à 8, pour utilisation comme médicament.

10. Complexe ligand-gonadotrophine choisi parmi :
un complexe d'un ligand selon l'une quelconque des revendications 1 à 8 avec la FSH ou un peptide actif de celle-ci ;
un complexe d'un ligand selon l'une quelconque des revendications 1 à 8 avec la LH ou l'hormone gonadotropine chorionique (CG) ou un peptide actif de celles-ci.

11. Complexe selon la revendication 10, pour utilisation comme médicament.

12. Ligand pour une utilisation selon la revendication 9 ou complexe pour une utilisation selon la revendication 11, où ledit médicament est destiné à induire l'ovulation ou une polyovulation chez un mammifère femelle.

13. Ligand pour une utilisation selon la revendication 9 ou complexe pour une utilisation selon la revendication 11, où ledit médicament est destiné à augmenter le taux de progestérone endogène circulant chez un mammifère femelle.

14. Composition pharmaceutique destinée à être utilisée pour l'induction de l'ovulation ou d'une polyovulation chez un mammifère femelle, **caractérisée en ce qu'**elle comprend un ligand selon l'une quelconque des revendications 1 à 8 et/ou un complexe selon la revendication 10 et un véhicule pharmaceutiquement acceptable.

15. Composition pharmaceutique pour son utilisation selon la revendication 14, **caractérisée en ce qu'**elle comprend en outre une FSH, une LH et/ou une CG.

16. Ligand selon l'une quelconque des revendications 1 à 8 ou complexe selon la revendication 10, pour une utilisation dans le traitement et/ou la prévention de l'infertilité ou de l'hypofertilité chez un mammifère.

17. Ligand selon l'une quelconque des revendications 1 à 8 ou complexe selon la revendication 10, pour une utilisation pour stimuler la procréation chez un mammifère femelle sain.

## Patentansprüche

1. Ligand von follikelstimulierendem Hormon (FSH), der die Bioaktivität von FSH, luteinisierendem Hormon (LH) und Choriogonadotropin(CG)-Hormon verstärkt, **dadurch gekennzeichnet, dass** es sich bei dem Liganden um einen Antikörper oder ein Fragment davon handelt und dass:
die variable Domäne der schweren Kette die folgenden CDRs enthält:
- VH-CDR1, definiert durch die Sequenz GFTFSDFY (SEQ ID NO: 9);
- VH-CDR2, definiert durch die Sequenz SRNKAKDYTT (SEQ ID NO: 10);
- VH-CDR3, definiert durch die Sequenz ARDARFAY (SEQ ID NO: 11); und
die variable Domäne der leichten Kette die folgenden CDRs enthält:
- VL-CDR1, definiert durch die Sequenz QSLLYSSNQKNY (SEQ ID NO: 12);
- VL-CDR2, definiert durch die Sequenz WAS;
- VL-CDR3, definiert durch die Sequenz QQYYSYPRT (SEQ ID NO: 13).

2. Ligand von follikelstimulierendem Hormon (FSH), der die Bioaktivität von FSH, luteinisierendem Hormon (LH) und Choriogonadotropin(CG)-Hormon verstärkt, **dadurch gekennzeichnet, dass** es sich bei dem Liganden um einen Antikörper oder ein Fragment davon handelt und dass:
- die variable Domäne der schweren Kette die Sequenz SEQ ID NO: 2 oder 47 enthält und/oder
- die variable Domäne der leichten Kette Kette die Sequenz SEQ ID NO: 4 oder 45 enthält.

3. Ligand von follikelstimulierendem Hormon (FSH), der die Bioaktivität von FSH, luteinisierendem Hormon (LH) und Choriogonadotropin(CG)-Hormon verstärkt, **dadurch gekennzeichnet, dass** es sich bei dem Liganden um einen Antikörper oder ein Fragment davon handelt und dass:
die variable Domäne der schweren Kette die folgenden CDRs enthält:
- VH-CDR1, definiert durch die Sequenz GFTFNTYA (SEQ ID NO: 14);
- VH-CDR2, definiert durch die Sequenz IRSKSNNYAT (SEQ ID NO: 15);
- VH-CDR3, definiert durch die Sequenz VRQDYYGSSYFDY (SEQ ID NO: 16); und
die variable Domäne der leichten Kette die folgenden CDRs enthält:
- VL-CDR1, definiert durch die Sequenz QSISDY (SEQ ID NO: 17);
- VL-CDR2, definiert durch die Sequenz YAS;
- VL-CDR3, definiert durch die Sequenz QNGHSFPYT (SEQ ID NO: 18).

4. Ligand von follikelstimulierendem Hormon (FSH), der die Bioaktivität von FSH, luteinisierendem Hormon (LH) und Choriogonadotropin(CG)-Hormon verstärkt, **dadurch gekennzeichnet, dass** es sich bei dem Liganden um einen Antikörper oder ein Fragment davon handelt und dass:
- die variable Domäne der schweren Kette die Sequenz SEQ ID NO: 6 enthält
und/oder
- die variable Domäne der leichten Kette Kette die Sequenz SEQ ID NO: 8 enthält.

5. Ligand nach einem der Ansprüche 1 bis 4, **dadurch gekennzeichnet, dass** der Ligand aus der Gruppe bestehend aus Fab, Fab', F(ab')2, Fv, dsFv, scFv, Diabodies, Triabodies und Tetrabodies ausgewählt ist.

6. Ligand nach Anspruch 1, **dadurch gekennzeichnet, dass** es sich bei dem Liganden um den durch das Hybridom CNCM I-4801 produzierten monoklonalen CA5-Antikörper handelt.

7. Ligand nach Anspruch 3, **dadurch gekennzeichnet, dass** es sich bei dem Liganden um den durch das Hybridom CNCM I-4802 produzierten monoklonalen CHI0-Antikörper handelt.

8. Ligand nach Anspruch 5, **dadurch gekennzeichnet, dass** es sich bei der Peptidsequenz von scFv um die Sequenz SEQ ID NO: 20 oder SEQ ID NO: 22 handelt.

9. Ligand nach einem der Ansprüche 1 bis 8 zur Verwendung als Medikament.

10. Ligand-Gonadotropin-Komplex, ausgewählt aus:
einem Komplex eines Liganden nach einem der Ansprüche 1 bis 8 mit FSH oder einem aktiven Peptid davon;
einem Komplex eines Liganden nach einem der Ansprüche 1 bis 8 mit LH oder Choriogonadotropin(CG)-Hormon oder einem aktiven Peptid davon.

11. Komplex nach Anspruch 10 zur Verwendung als Medikament.

12. Ligand zur Verwendung nach Anspruch 9 oder Komplex zur Verwendung nach Anspruch 11, wobei das Medikament zur Induktion der Ovulation oder einer Polyovulation bei einem weiblichen Säugetier vorgesehen ist.

13. Ligand zur Verwendung nach Anspruch 9 oder Komplex zur Verwendung nach Anspruch 11, wobei das Medikament zur Erhöhung des Spiegels von zirkulierendem endogenem Progesteron bei einem weiblichen Säugetier vorgesehen ist.

14. Pharmazeutische Zusammensetzung zur Verwendung zur Induktion der Ovulation oder einer Polyovulation bei einem weiblichen Säugetier, **dadurch gekennzeichnet, dass** sie einen Liganden nach einem der Ansprüche 1 bis 8 und/oder einen Komplex nach Anspruch 10 und ein pharmazeutisch unbedenkliches Vehikel umfasst.

15. Pharmazeutische Zusammensetzung zur Verwendung nach Anspruch 14, **dadurch gekennzeichnet, dass** sie außerdem ein FSH, ein LH und/oder ein CG umfasst.

16. Ligand nach einem der Ansprüche 1 bis 8 oder Komplex nach Anspruch 10 zur Verwendung bei der Behandlung und/oder Prävention von Unfruchtbarkeit oder verminderter Fruchtbarkeit bei einem Säugetier.

17. Ligand nach einem der Ansprüche 1 bis 8 oder Komplex nach Anspruch 10 zur Verwendung zur Stimulation der Fortpflanzung bei einem gesunden weiblichen Säugetier.

## Claims

1. Follicle-stimulating hormone (FSH) ligand which potentiates the bioactivity of FSH, of luteinizing hormone (LH) and of chorionic gonadotropin (CG), **characterized in that** said ligand is an antibody or a fragment thereof and **in that**:
the heavy chain variable domain contains the following CDRs:
- VH-CDR1, defined by the sequence GFTFSDFY (SEQ ID NO : 9);
- VH-CDR2, defined by the sequence SRNKAKDYTT (SEQ ID NO : 10);
- VH-CDR3, defined by the sequence ARDARFAY (SEQ ID NO : 11); and
the light chain variable domain contains the following CDRs:
- VL-CDR1, defined by the sequence QSLLYSSNQKNY (SEQ ID NO : 12);
- VL-CDR2, defined by the sequence WAS;
- VL-CDR3, defined by the sequence QQYYSYPRT (SEQ ID NO : 13).

2. Follicle-stimulating hormone (FSH) ligand which potentiates the bioactivity of FSH, of luteinizing hormone (LH) and of chorionic gonadotropin (CG), **characterized in that** said ligand is an antibody or a fragment thereof and **in that**:
- the heavy chain variable domain contains the sequence SEQ ID NO: 2 or 47; and/or
- the light chain variable domain contains the sequence SEQ ID NO: 4 or 45.

3. Follicle-stimulating hormone (FSH) ligand which potentiates the bioactivity of FSH, of luteinizing hormone (LH) and of chorionic gonadotropin (CG), **characterized in that** said ligand is an antibody or a fragment thereof and **in that**:
the heavy chain variable domain contains the following CDRs:
- VH-CDR1, defined by the sequence GFTFNTYA (SEQ ID NO : 14);
- VH-CDR2, defined by the sequence IRSKSNNYAT (SEQ ID NO : 15);
- VH-CDR3, defined by the sequence VRQDYYGSSYFDY (SEQ ID NO : 16); and
the light chain variable domain contains the following CDRs:
- VL-CDR1, defined by the sequence QSISDY (SEQ ID NO : 17);
- VL-CDR2, defined by the sequence YAS;
- VL-CDR3, defined by the sequence QNGHSFPYT (SEQ ID NO : 18).

4. Follicle-stimulating hormone (FSH) ligand which potentiates the bioactivity of FSH, of luteinizing hormone (LH) and of chorionic gonadotropin (CG), **characterized in that** said ligand is an antibody or a fragment thereof and **in that**:
- the heavy chain variable domain contains the sequence SEQ ID NO: 6;
and/or
- the light chain variable domain contains the sequence SEQ ID NO: 8.

5. Ligand according to any one of claims 1 to 4, **characterized in that** the ligand is chosen from the group consisting of Fab, Fab', F(ab')2, Fv, dsFv, scFv, diabodies, triabodies, tetrabodies.

6. Ligand according to claim 1, **characterized in that** the ligand is the CA5 monoclonal antibody produced by the CNCM I-4801 hybridoma.

7. Ligand according to claim 3, **characterized in that** the ligand is the CH10 monoclonal antibody produced by the CNCM I-4802 hybridoma.

8. Ligand according to claim 5, **characterized in that** the peptide sequence of the scFv is the sequence SEQ ID NO : 20 or SEQ ID NO : 22.

9. Ligand according to any one of claims 1 to 8, for use as a medicament.

10. Lligand-gonadotropin complex chosen from:
a complex of a ligand according to in any one of claims 1 to 8 with FSH or an active peptide thereof;
a complex of a ligand according to in any one of claims 1 to 8 with LH or the chorionic gonadotropin (CG) hormone, or an active peptide of said hormones.

11. Complex according to claim 10, for use as a medicament.

12. Ligand for use according to claim 9 or complex for use according to claim 11, wherein said medicament is intended to induce ovulation or polyovulation in a female mammal.

13. Ligand for use according to claim 9 or complex for use according to claim 11, wherein said medicament is intended to increase the circulating endogenous progesterone level in a female mammal.

14. Pharmaceutical composition intended to be used for inducing ovulation or polyovulation in a female mammal, **characterized in that** it comprises a ligand according to any one of claims 1 to 8 and/or a complex according to claim 10 and a pharmaceutically acceptable carrier.

15. Pharmaceutical composition for use according to claim 14, **characterized in that** it also comprises an FSH, an LH and/or a CG.

16. Ligand according to any one of claims 1 to 8 or complex according to claim 10, for use in the treatment and/or prevention of infertility or of hypofertility in a mammal.

17. Ligand according to any one of claims 1 to 8 or complex according to claim 10, for use for stimulating procreation in a healthy female mammal.


### SEQUENCE LISTING

<110> REPROPHARM
   KARA, Elodie
   DECOURTYE, Jérémye
   CASTERET, Sophie
   MAUREL, Marie-Christine
<120> LIGANDS POTENTIALISANTS DE LA BIOACTIVITE DES GONADOTROPHINES
<130> BNT213486PC01
<150> FR1558078
   <151> 2014-09-10
<160> 70
<170> PatentIn version 3.5
<210> 1
   <211> 351
   <212> DNA
   <213> Artificial Sequence
<220>
   <223> VH CA5
<400> 1
   gaggtgaagc tggtggaatc tggaggaggc ttggtacagc ctgggggttc tctgagactc      60
   tcctgtgcaa cttctgggtt caccttcagt gatttctaca tggagtgggt ccgccagcct     120
   ccagggaaga gactggagtg gattgctgca agtagaaaca aagctaagga ttatacaaca     180
   gagtacagtg catctgtgaa gggtcggttc atcgtctcca gagacacttc ccaaagcatc     240
   ctctaccttc agatgaatgc cctgagagct gaggacactg ccatttattt ctgtgcaaga     300
   gatgcaaggt ttgcttactg gggccaaggg actctggtca ctgtctctgc a              351
<210> 2
   <211> 117
   <212> PRT
   <213> Artificial Sequence
<220>
   <223> VH CA5
<400> 2
   Glu Val Lys Leu Val Glu Ser Gly Gly Gly Leu Val Gln Pro Gly Gly
   1               5                   10                  15
   Ser Leu Arg Leu Ser Cys Ala Thr Ser Gly Phe Thr Phe Ser Asp Phe
               20                  25                  30
   Tyr Met Glu Trp Val Arg Gln Pro Pro Gly Lys Arg Leu Glu Trp Ile
           35                  40                  45
   Ala Ala Ser Arg Asn Lys Ala Lys Asp Tyr Thr Thr Glu Tyr Ser Ala
       50                  55                  60
   Ser Val Lys Gly Arg Phe Ile Val Ser Arg Asp Thr Ser Gln Ser Ile
   65                  70                  75                  80
   Leu Tyr Leu Gln Met Asn Ala Leu Arg Ala Glu Asp Thr Ala Ile Tyr
                   85                  90                  95
   Phe Cys Ala Arg Asp Ala Arg Phe Ala Tyr Trp Gly Gln Gly Thr Leu
               100                 105                 110
   Val Thr Val Ser Ala
           115
<210> 3
   <211> 339
   <212> DNA
   <213> Artificial Sequence
<220>
   <223> VL CA5
<400> 3
   gacattgtga tgtcacagtc tccatcctcc ctagctgtgt cagttggaga gaagattact 60
   atgagctgca agtccagtca gagcctttta tatagtagca atcaaaagaa ctacttggcc 120
   tggtaccagc agaaaccagg gcagtctcct aaactgctga tttactgggc atccactagg 180
   gaatctgggg tccctgatcg cttcacaggc agtggatctg ggacagattt cactctcacc 240
   atcagcagtg tgaaggctga agacctggca gtttattact gtcagcaata ttatagctat 300
   cctcggacgt tcggtggagg caccaagctg gaaatcaaa 339
<210> 4
   <211> 113
   <212> PRT
   <213> Artificial Sequence
<220>
   <223> VL CA5
<400> 4
   Asp Ile Val Met Ser Gln Ser Pro Ser Ser Leu Ala Val Ser Val Gly
   1 5 10 15
   Glu Lys Ile Thr Met Ser Cys Lys Ser Ser Gln Ser Leu Leu Tyr Ser
   20 25 30
   Ser Asn Gln Lys Asn Tyr Leu Ala Trp Tyr Gln Gln Lys Pro Gly Gln
   35 40 45
   Ser Pro Lys Leu Leu Ile Tyr Trp Ala Ser Thr Arg Glu Ser Gly Val
   50 55 60
   Pro Asp Arg Phe Thr Gly Ser Gly Ser Gly Thr Asp Phe Thr Leu Thr
   65 70 75 80
   Ile Ser Ser Val Lys Ala Glu Asp Leu Ala Val Tyr Tyr Cys Gln Gln
   85 90 95
   Tyr Tyr Ser Tyr Pro Arg Thr Phe Gly Gly Gly Thr Lys Leu Glu Ile
   100 105 110
   Lys
<210> 5
   <211> 366
   <212> DNA
   <213> Artificial Sequence
<220>
   <223> VH CH10
<400> 5
   gaggtgcagc ttgttgagtc tggtggagga ttggtgcagc ctaaagggtc attgaaactc 60
   tcatgtgcag cctctggatt caccttcaat acctacgcca tgaactgggt ccgccaggct 120
   ccaggaaagg gtttggaatg ggttgctcgc ataagaagta aaagtaataa ttatgcaaca 180
   tattatgccg attcagtgaa agacaggttc accatctcca gagatgattc acaaagcatg 240
   ctctatctgc aaatgaacaa cttgaaaact gaggacacag ccatgtatta ctgtgtgaga 300
   caggattact acggtagtag ctactttgac tactggggcc aaggcaccac tctcacagtc 360
   tcctca 366
<210> 6
   <211> 122
   <212> PRT
   <213> Artificial Sequence
<220>
   <223> VH CH10
<400> 6
   Glu Val Gln Leu Val Glu Ser Gly Gly Gly Leu Val Gln Pro Lys Gly
   1 5 10 15
   Ser Leu Lys Leu Ser Cys Ala Ala Ser Gly Phe Thr Phe Asn Thr Tyr
   20 25 30
   Ala Met Asn Trp Val Arg Gln Ala Pro Gly Lys Gly Leu Glu Trp Val
           35                  40                  45
   Ala Arg Ile Arg Ser Lys Ser Asn Asn Tyr Ala Thr Tyr Tyr Ala Asp
       50                  55                  60
   Ser Val Lys Asp Arg Phe Thr Ile Ser Arg Asp Asp Ser Gln Ser Met
   65                  70                  75                  80
   Leu Tyr Leu Gln Met Asn Asn Leu Lys Thr Glu Asp Thr Ala Met Tyr
                   85                  90                  95
   Tyr Cys Val Arg Gln Asp Tyr Tyr Gly Ser Ser Tyr Phe Asp Tyr Trp
               100                 105                 110
   Gly Gln Gly Thr Thr Leu Thr Val Ser Ser
           115                 120
<210> 7
   <211> 321
   <212> DNA
   <213> Artificial Sequence
<220>
   <223> VL CH10
<400> 7
   gacattgtga tgactcagtc tccagccacc ctgtctgtga ctccaggaga tagagtctct       60
   ctttcctgca gggccagcca gagtattagc gactacttac actggtatca acaaaaatca      120
   catgagtctc caaggcttct catcaaatat gcttcccaat ccatctctgg gatcccctcc      180
   aggttcagtg gcagtggatc agggtcagat ttcactctca gtatcaacag tgtggaacct      240
   gaagatgttg gagtgtatta ctgtcaaaat ggtcacagct ttccgtacac gttcggaggg      300
   gggaccaagc tggaaataaa a                                                321
<210> 8
   <211> 107
   <212> PRT
   <213> Artificial Sequence
<220>
   <223> VL CH10
<400> 8
   Asp Ile Val Met Thr Gln Ser Pro Ala Thr Leu Ser Val Thr Pro Gly
   1               5                   10                  15
   Asp Arg Val Ser Leu Ser Cys Arg Ala Ser Gln Ser Ile Ser Asp Tyr
               20                  25                  30
   Leu His Trp Tyr Gln Gln Lys Ser His Glu Ser Pro Arg Leu Leu Ile
           35                  40                  45
   Lys Tyr Ala Ser Gln Ser Ile Ser Gly Ile Pro Ser Arg Phe Ser Gly
       50                  55                  60
   Ser Gly Ser Gly Ser Asp Phe Thr Leu Ser Ile Asn Ser Val Glu Pro
   65                  70                  75                  80
   Glu Asp Val Gly Val Tyr Tyr Cys Gln Asn Gly His Ser Phe Pro Tyr
                   85                  90                  95
   Thr Phe Gly Gly Gly Thr Lys Leu Glu Ile Lys
               100                 105
<210> 9
   <211> 8
   <212> PRT
   <213> Artificial Sequence
<220>
   <223> VH-CDR1 CA5
<400> 9
   Gly Phe Thr Phe Ser Asp Phe Tyr
   1               5
<210> 10
   <211> 10
   <212> PRT
   <213> Artificial Sequence
<220>
   <223> VH-CDR2 CA5
<400> 10
   Ser Arg Asn Lys Ala Lys Asp Tyr Thr Thr
   1               5                   10
<210> 11
   <211> 8
   <212> PRT
   <213> Artificial Sequence
<220>
   <223> VH-CDR3 CA5
<400> 11
   Ala Arg Asp Ala Arg Phe Ala Tyr
   1 5
<210> 12
   <211> 12
   <212> PRT
   <213> Artificial Sequence
<220>
   <223> VL-CDR1 CA5
<400> 12
   Gln Ser Leu Leu Tyr Ser Ser Asn Gln Lys Asn Tyr
   1 5 10
<210> 13
   <211> 9
   <212> PRT
   <213> Artificial Sequence
<220>
   <223> VL-CDR3 CA5
<400> 13
   Gln Gln Tyr Tyr Ser Tyr Pro Arg Thr
   1 5
<210> 14
   <211> 8
   <212> PRT
   <213> Artificial Sequence
<220>
   <223> VH-CDR1 CH10
<400> 14
   Gly Phe Thr Phe Asn Thr Tyr Ala
   1 5
<210> 15
   <211> 10
   <212> PRT
   <213> Artificial Sequence
<220>
   <223> VH-CDR2 CH10
<400> 15
   Ile Arg Ser Lys Ser Asn Asn Tyr Ala Thr
   1 5 10
<210> 16
   <211> 13
   <212> PRT
   <213> Artificial Sequence
<220>
   <223> VH-CDR3 CH10
<400> 16
   Val Arg Gln Asp Tyr Tyr Gly Ser Ser Tyr Phe Asp Tyr
   1 5 10
<210> 17
   <211> 6
   <212> PRT
   <213> Artificial Sequence
<220>
   <223> VL-CDR1 CH10
<400> 17
   Gln Ser Ile Ser Asp Tyr
   1 5
<210> 18
   <211> 9
   <212> PRT
   <213> Artificial Sequence
<220>
   <223> VL-CDR3 CH10
<400> 18
   Gln Asn Gly His Ser Phe Pro Tyr Thr
   1 5
<210> 19
   <211> 771
   <212> DNA
   <213> Artificial Sequence
<220>
   <223> scFv CA5
<400> 19
   caggtgcagc tgcagcagtc aggcggcggc ctggtacaac ctggtggctc actgcgcctg        60
   agctgcgcaa ccagcggttt tacctttagc gatttctaca tggaatgggt tcgccaaccg       120
   ccgggtaagc gtctggaatg gatcgcggcg agccgtaaca aggcgaaaga ttataccact       180
   gaatatagcg cgtcggtgaa aggtcgcttc attgtctcgc gcgataccag ccagtcgatt       240
   ctgtatctgc aaatgaatgc cctgcgtgcc gaagacacgg ccatctactt ctgtgcgcgt       300
   gatgcacgct ttgcctattg gggccaaggc accctggtga ccgttagcgc cggtggtggc       360
   ggttcaggtg gtggcggtag cggtggcggt ggctcagata ttcagatgac ccagaccccg       420
   tcaagcctgg cggtgtcagt cggcgaagag attactatga gctgtaaaag ctcgcagagc       480
   ctgctgtact catcgaacca gaaaaattac ctggcatggt atcaacagaa gccgggtcag       540
   tcgccgaaac tgctgatcta ctgggcctca acccgtgaga gcggcgtacc ggatcgcttt       600
   actggcagcg gcagcggcac ggactttacg ctgacgatta gctcggtgaa ggccgaagac       660
   ctggcggttt attattgcca acagtactat agctaccctc gtaccttcgg cggcggcacg       720
   aaactcgaga ttaaacatca ccatcaccat cactaactcg agatcaagta a                771
<210> 20
   <211> 251
   <212> PRT
   <213> Artificial Sequence
<220>
   <223> scFv CA5
<400> 20
   Gln Val Gln Leu Gln Gln Ser Gly Gly Gly Leu Val Gln Pro Gly Gly
   1               5                   10                  15
   Ser Leu Arg Leu Ser Cys Ala Thr Ser Gly Phe Thr Phe Ser Asp Phe
               20                  25                  30
   Tyr Met Glu Trp Val Arg Gln Pro Pro Gly Lys Arg Leu Glu Trp Ile
           35                  40                  45
   Ala Ala Ser Arg Asn Lys Ala Lys Asp Tyr Thr Thr Glu Tyr Ser Ala
       50                  55                  60
   Ser Val Lys Gly Arg Phe Ile Val Ser Arg Asp Thr Ser Gln Ser Ile
   65                  70                  75                  80
   Leu Tyr Leu Gln Met Asn Ala Leu Arg Ala Glu Asp Thr Ala Ile Tyr
                   85                  90                  95
   Phe Cys Ala Arg Asp Ala Arg Phe Ala Tyr Trp Gly Gln Gly Thr Leu
               100                 105                 110
   Val Thr Val Ser Ala Gly Gly Gly Gly Ser Gly Gly Gly Gly Ser Gly
           115                 120                 125
   Gly Gly Gly Ser Asp Ile Gln Met Thr Gln Thr Pro Ser Ser Leu Ala
       130                 135                 140
   Val Ser Val Gly Glu Glu Ile Thr Met Ser Cys Lys Ser Ser Gln Ser
   145                 150                 155                 160
   Leu Leu Tyr Ser Ser Asn Gln Lys Asn Tyr Leu Ala Trp Tyr Gln Gln
                   165                 170                 175
   Lys Pro Gly Gln Ser Pro Lys Leu Leu Ile Tyr Trp Ala Ser Thr Arg
                   180                 185                 190
   Glu Ser Gly Val Pro Asp Arg Phe Thr Gly Ser Gly Ser Gly Thr Asp
           195                 200                 205
   Phe Thr Leu Thr Ile Ser Ser Val Lys Ala Glu Asp Leu Ala Val Tyr
       210                 215                 220
   Tyr Cys Gln Gln Tyr Tyr Ser Tyr Pro Arg Thr Phe Gly Gly Gly Thr
   225                 230                 235                 240
   Lys Leu Glu Ile Lys His His His His His His
                   245                 250
<210> 21
   <211> 768
   <212> DNA
   <213> Artificial Sequence
<220>
   <223> scFv CH10
<400> 21
   caggtgcagc tgcagcaatc aggcggcggc ctggtccaac cgaaaggtag cctgaaactg      60
   tcgtgcgccg ccagcggctt tacgttcaac acttacgcga tgaattgggt gcgtcaggcg     120
   cctggtaaag gcctggaatg ggtggcacgc atccgttcaa aaagcaacaa ttacgcgacg     180
   tattatgcag acagcgtaaa agatcgcttt accatcagcc gtgatgattc acagtcaatg     240
   ctgtacctgc aaatgaataa cctgaaaact gaagacactg cgatgtatta ttgtgttcgc     300
   caggactatt acggtagctc gtatttcgat tactggggcc aaggcaccac cctgacggtg     360
   agctcgggtg gcggtggctc aggtggtggt ggtagcggcg gtggcggtag cgatatccag     420
   atgacccaga ccccggcaac cctgagcgtt acccctggtg accgcgtttc gctgagctgc     480
   cgtgcctcgc agagcatttc ggactatctg cactggtatc agcaaaaatc acacgaatca     540
   ccgcgtctgc tgattaagta cgccagccaa tcgattagcg gtattccgag ccgcttttcg     600
   ggctcgggtt cgggctcgga ttttaccctg tcaattaata gcgtagagcc ggaagatgta     660
   ggcgtctact attgtcagaa cggccattca ttcccgtaca cgtttggcgg cggcaccaag     720
   ctcgagatta agcatcacca tcatcaccat taactcgaga tcaagtaa                  768
<210> 22
   <211> 250
   <212> PRT
   <213> Artificial Sequence
<220>
   <223> scFv CH10
<400> 22
   Gln Val Gln Leu Gln Gln Ser Gly Gly Gly Leu Val Gln Pro Lys Gly
   1               5                   10                  15
   Ser Leu Lys Leu Ser Cys Ala Ala Ser Gly Phe Thr Phe Asn Thr Tyr
               20                  25                  30
   Ala Met Asn Trp Val Arg Gln Ala Pro Gly Lys Gly Leu Glu Trp Val
           35                  40                  45
   Ala Arg Ile Arg Ser Lys Ser Asn Asn Tyr Ala Thr Tyr Tyr Ala Asp
       50                  55                  60
   Ser Val Lys Asp Arg Phe Thr Ile Ser Arg Asp Asp Ser Gln Ser Met
   65                  70                  75                  80
   Leu Tyr Leu Gln Met Asn Asn Leu Lys Thr Glu Asp Thr Ala Met Tyr
                   85                  90                  95
   Tyr Cys Val Arg Gln Asp Tyr Tyr Gly Ser Ser Tyr Phe Asp Tyr Trp
               100                 105                 110
   Gly Gln Gly Thr Thr Leu Thr Val Ser Ser Gly Gly Gly Gly Ser Gly
           115                 120                 125
   Gly Gly Gly Ser Gly Gly Gly Gly Ser Asp Ile Gln Met Thr Gln Thr
       130                 135                 140
   Pro Ala Thr Leu Ser Val Thr Pro Gly Asp Arg Val Ser Leu Ser Cys
   145                 150                 155                 160
   Arg Ala Ser Gln Ser Ile Ser Asp Tyr Leu His Trp Tyr Gln Gln Lys
                   165                 170                 175
   Ser His Glu Ser Pro Arg Leu Leu Ile Lys Tyr Ala Ser Gln Ser Ile
               180                 185                 190
   Ser Gly Ile Pro Ser Arg Phe Ser Gly Ser Gly Ser Gly Ser Asp Phe
           195                 200                 205
   Thr Leu Ser Ile Asn Ser Val Glu Pro Glu Asp Val Gly Val Tyr Tyr
       210                 215                 220
   Cys Gln Asn Gly His Ser Phe Pro Tyr Thr Phe Gly Gly Gly Thr Lys
   225                 230                 235                 240
   Leu Glu Ile Lys His His His His His His
   245 250
<210> 23
   <211> 35
   <212> DNA
   <213> Artificial Sequence
<220>
   <223> VHRev1
<400> 23
   cgggatcctc tagaggtcca actgcaggag tcagg 35
<210> 24
   <211> 19
   <212> DNA
   <213> Artificial Sequence
<220>
   <223> VHFor
<400> 24
   caggggccag tggatagac 19
<210> 25
   <211> 21
   <212> DNA
   <213> Artificial Sequence
<220>
   <223> MKRev5
<400> 25
   gacattgtga tgacccagtc t 21
<210> 26
   <211> 21
   <212> DNA
   <213> Artificial Sequence
<220>
   <223> MKC5For
<400> 26
   ggatacagtt ggtgcagcat c 21
<210> 27
   <211> 22
   <212> DNA
   <213> Artificial Sequence
<220>
   <223> CA5VH_Fw
<400> 27
   cacttttaca tggtatccag tg 22
<210> 28
   <211> 23
   <212> DNA
   <213> Artificial Sequence
<220>
   <223> CA5VH_Rev
<400> 28
   gtttctactt gcagcaatcc act 23
<210> 29
   <211> 19
   <212> DNA
   <213> Artificial Sequence
<220>
   <223> CA5VL_Fw
<220>
   <221> misc_feature
   <222> (3)..(3)
   <223> W = A ou T
<220>
   <221> misc_feature
   <222> (10)..(10)
   <223> R = G ou A
<220>
   <221> misc_feature
   <222> (18)..(18)
   <223> Y = T ou C
<400> 29
   gawtcacagr cccaggtyc 19
<210> 30
   <211> 23
   <212> DNA
   <213> Artificial Sequence
<220>
   <223> CA5VL_Rev
<400> 30
   cccagtaaat cagcagttta gga 23
<210> 31
   <211> 20
   <212> DNA
   <213> Artificial Sequence
<220>
   <223> MuCFor
<400> 31
   ggggaagaca tttgggaagg 20
<210> 32
   <211> 21
   <212> DNA
   <213> Artificial Sequence
<220>
   <223> MKRev2
<400> 32
   gatattgtga tgacgcaggc t 21
<210> 33
   <211> 18
   <212> DNA
   <213> Artificial Sequence
<220>
   <223> MKRev3
<400> 33
   gatattgtga taacccag 18
<210> 34
   <211> 21
   <212> DNA
   <213> Artificial Sequence
<220>
   <223> MKRev4
<400> 34
   gacattgtgc tgacccaatc t 21
<210> 35
   <211> 21
   <212> DNA
   <213> Artificial Sequence
<220>
   <223> MKRev6
<400> 35
   gatattgtgc taactcagtc t 21
<210> 36
   <211> 21
   <212> DNA
   <213> Artificial Sequence
<220>
   <223> MKRev8
<400> 36
   gacatccagc tgactcagtc t 21
<210> 37
   <211> 21
   <212> DNA
   <213> Artificial Sequence
<220>
   <223> MKC5for
<400> 37
   ggatacagtt ggtgcagcat c 21
<210> 38
   <211> 20
   <212> DNA
   <213> Artificial Sequence
<220>
   <223> CH10VH_Fw
<400> 38
   atggtgttgg ggctgaagtg 20
<210> 39
   <211> 22
   <212> DNA
   <213> Artificial Sequence
<220>
   <223> CH10VH_Rev
<400> 39
   cagttcatgg cgtaggtatt ga 22
<210> 40
   <211> 20
   <212> DNA
   <213> Artificial Sequence
<220>
   <223> CH10VL_Fw
<400> 40
   ttctggaytt cagcctccag 20
<210> 41
   <211> 23
   <212> DNA
   <213> Artificial Sequence
<220>
   <223> CH10VL_Rev
<400> 41
   gattgggaag catatttgat gag 23
<210> 42
   <211> 14
   <212> PRT
   <213> Artificial Sequence
<220>
   <223> Lieur
<400> 42
   Gly Gly Gly Gly Ser Gly Gly Gly Gly Ser Gly Gly Gly Ser
   1               5                   10
<210> 43
   <211> 16
   <212> PRT
   <213> Artificial Sequence
<220>
   <223> Fragment VL de scFv
<400> 43
   Leu Glu Ile Lys His His His His His His Leu Glu Ile Lys Val Asp
   1               5                   10                  15
<210> 44
   <211> 360
   <212> DNA
   <213> Artificial Sequence
<220>
   <223> VL CA5
<400> 44
   gatattcaga tgacccagac cccgtcaagc ctggcggtgt cagtcggcga agagattact      60
   atgagctgta aaagctcgca gagcctgctg tactcatcga accagaaaaa ttacctggca     120
   tggtatcaac agaagccggg tcagtcgccg aaactgctga tctactgggc ctcaacccgt     180
   gagagcggcg taccggatcg ctttactggc agcggcagcg gcacggactt tacgctgacg     240
   attagctcgg tgaaggccga agacctggcg gtttattatt gccaacagta ctatagctac     300
   cctcgtacct tcggcggcgg cacgaaactt gagattaaac atcaccatca ccatcactaa     360
<210> 45
   <211> 119
   <212> PRT
   <213> Artificial Sequence
<220>
   <223> VL CA5
<400> 45
   Asp Ile Gln Met Thr Gln Thr Pro Ser Ser Leu Ala Val Ser Val Gly
   1               5                   10                  15
   Glu Glu Ile Thr Met Ser Cys Lys Ser Ser Gln Ser Leu Leu Tyr Ser
               20                  25                  30
   Ser Asn Gln Lys Asn Tyr Leu Ala Trp Tyr Gln Gln Lys Pro Gly Gln
           35                  40                  45
   Ser Pro Lys Leu Leu Ile Tyr Trp Ala Ser Thr Arg Glu Ser Gly Val
       50                  55                  60
   Pro Asp Arg Phe Thr Gly Ser Gly Ser Gly Thr Asp Phe Thr Leu Thr
   65                  70                  75                  80
   Ile Ser Ser Val Lys Ala Glu Asp Leu Ala Val Tyr Tyr Cys Gln Gln
                   85                  90                  95
   Tyr Tyr Ser Tyr Pro Arg Thr Phe Gly Gly Gly Thr Lys Leu Glu Ile
               100                 105                 110
   Lys His His His His His His
           115
<210> 46
   <211> 372
   <212> DNA
   <213> Artificial Sequence
<220>
   <223> VH CA5
<400> 46
   caggtgcagc tgcagcagtc aggcggcggc ctggtacaac ctggtggctc actgcgcctg      60
   agctgcgcaa ccagcggttt tacctttagc gatttctaca tggaatgggt tcgccaaccg     120
   ccgggtaagc gtctggaatg gatcgcggcg agccgtaaca aggcgaaaga ttataccact     180
   gaatatagcg cgtcggtgaa aggtcgcttc attgtctcgc gcgataccag ccagtcgatt     240
   ctgtatctgc aaatgaatgc cctgcgtgcc gaagacacgg ccatctactt ctgtgcgcgt     300
   gatgcacgct ttgcctattg gggccaaggc accctggtga ccgttagcgc ccatcaccat     360
   caccatcact aa                                                         372
<210> 47
   <211> 123
   <212> PRT
   <213> Artificial Sequence
<220>
   <223> VH CA5
<400> 47
   Gln Val Gln Leu Gln Gln Ser Gly Gly Gly Leu Val Gln Pro Gly Gly
   1               5                   10                  15
   Ser Leu Arg Leu Ser Cys Ala Thr Ser Gly Phe Thr Phe Ser Asp Phe
               20                  25                  30
   Tyr Met Glu Trp Val Arg Gln Pro Pro Gly Lys Arg Leu Glu Trp Ile
           35                  40                  45
   Ala Ala Ser Arg Asn Lys Ala Lys Asp Tyr Thr Thr Glu Tyr Ser Ala
       50                  55                  60
   Ser Val Lys Gly Arg Phe Ile Val Ser Arg Asp Thr Ser Gln Ser Ile
   65                  70                  75                  80
   Leu Tyr Leu Gln Met Asn Ala Leu Arg Ala Glu Asp Thr Ala Ile Tyr
                   85                  90                  95
   Phe Cys Ala Arg Asp Ala Arg Phe Ala Tyr Trp Gly Gln Gly Thr Leu
               100                 105                 110
   Val Thr Val Ser Ala His His His His His His
           115                 120
<210> 48
   <211> 756
   <212> DNA
   <213> Artificial Sequence
<220>
   <223> ScFv CA5 inverse
<400> 48
   gatattcaga tgacccagac cccgtcaagc ctggcggtgt cagtcggcga agagattact      60
   atgagctgta aaagctcgca gagcctgctg tactcatcga accagaaaaa ttacctggca     120
   tggtatcaac agaagccggg tcagtcgccg aaactgctga tctactgggc ctcaacccgt     180
   gagagcggcg taccggatcg ctttactggc agcggcagcg gcacggactt tacgctgacg     240
   attagctcgg tgaaggccga agacctggcg gtttattatt gccaacagta ctatagctac     300
   cctcgtacct tcggcggcgg cacgaaactt gagattaaag gtggtggcgg ttcaggtggt     360
   ggcggtagcg gtggcggtgg ctcacaggtg cagctgcagc agtcaggcgg cggcctggta     420
   caacctggtg gctcactgcg cctgagctgc gcaaccagcg gttttacctt tagcgatttc     480
   tacatggaat gggttcgcca accgccgggt aagcgtctgg aatggatcgc ggcgagccgt     540
   aacaaggcga aagattatac cactgaatat agcgcgtcgg tgaaaggtcg cttcattgtc     600
   tcgcgcgata ccagccagtc gattctgtat ctgcaaatga atgccctgcg tgccgaagac     660
   acggccatct acttctgtgc gcgtgatgca cgctttgcct attggggcca aggcaccctg     720
   gtgaccgtta gcgcccatca ccatcaccat cactaa                               756
<210> 49
   <211> 251
   <212> PRT
   <213> Artificial Sequence
<220>
   <223> ScFv CA5 inverse
<400> 49
   Asp Ile Gln Met Thr Gln Thr Pro Ser Ser Leu Ala Val Ser Val Gly
   1               5                   10                  15
   Glu Glu Ile Thr Met Ser Cys Lys Ser Ser Gln Ser Leu Leu Tyr Ser
               20                  25                  30
   Ser Asn Gln Lys Asn Tyr Leu Ala Trp Tyr Gln Gln Lys Pro Gly Gln
           35                  40                  45
   Ser Pro Lys Leu Leu Ile Tyr Trp Ala Ser Thr Arg Glu Ser Gly Val
       50                  55                  60
   Pro Asp Arg Phe Thr Gly Ser Gly Ser Gly Thr Asp Phe Thr Leu Thr
   65                  70                  75                  80
   Ile Ser Ser Val Lys Ala Glu Asp Leu Ala Val Tyr Tyr Cys Gln Gln
                   85                  90                  95
   Tyr Tyr Ser Tyr Pro Arg Thr Phe Gly Gly Gly Thr Lys Leu Glu Ile
               100                 105                 110
   Lys Gly Gly Gly Gly Ser Gly Gly Gly Gly Ser Gly Gly Gly Gly Ser
           115                 120                 125
   Gln Val Gln Leu Gln Gln Ser Gly Gly Gly Leu Val Gln Pro Gly Gly
       130                 135                 140
   Ser Leu Arg Leu Ser Cys Ala Thr Ser Gly Phe Thr Phe Ser Asp Phe
   145                 150                 155                 160
   Tyr Met Glu Trp Val Arg Gln Pro Pro Gly Lys Arg Leu Glu Trp Ile
                   165                 170                 175
   Ala Ala Ser Arg Asn Lys Ala Lys Asp Tyr Thr Thr Glu Tyr Ser Ala
               180                 185                 190
   Ser Val Lys Gly Arg Phe Ile Val Ser Arg Asp Thr Ser Gln Ser Ile
           195                 200                 205
   Leu Tyr Leu Gln Met Asn Ala Leu Arg Ala Glu Asp Thr Ala Ile Tyr
               210                 215                 220
   Phe Cys Ala Arg Asp Ala Arg Phe Ala Tyr Trp Gly Gln Gly Thr Leu
   225                 230                 235                 240
   Val Thr Val Ser Ala His His His His His His
                   245                 250
<210> 50
   <211> 92
   <212> PRT
   <213> Homo sapiens
<220>
   <221> misc_feature
   <223> sous-unité alpha hFSH, hCG et hLH
<220>
   <221> misc_feature
   <222> (17)..(17)
   <223> Xaa = Leu ou Phe
<400> 50
   Ala Pro Asp Val Gln Asp Cys Pro Glu Cys Thr Leu Gln Glu Asn Pro
   1               5                   10                  15
   Xaa Phe Ser Gln Pro Gly Ala Pro Ile Leu Gln Cys Met Gly Cys Cys
               20                  25                  30
   Phe Ser Arg Ala Tyr Pro Thr Pro Leu Arg Ser Lys Lys Thr Met Leu
           35                  40                  45
   Val Gln Lys Asn Val Thr Ser Glu Ser Thr Cys Cys Val Ala Lys Ser
       50                  55                  60
   Tyr Asn Arg Val Thr Val Met Gly Gly Phe Lys Val Glu Asn His Thr
   65                  70                  75                  80
   Ala Cys His Cys Ser Thr Cys Tyr Tyr His Lys Ser
                   85                  90
<210> 51
   <211> 96
   <212> PRT
   <213> Ovis aries
<220>
   <221> misc_feature
   <223> sous-unité alpha oLH et oFSH
<400> 51
   Phe Pro Asp Gly Glu Phe Thr Met Gln Gly Cys Pro Glu Cys Lys Leu
   1               5                   10                  15
   Lys Glu Asn Lys Tyr Phe Ser Lys Pro Asp Ala Pro Ile Tyr Gln Cys
               20                  25                  30
   Met Gly Cys Cys Phe Ser Arg Ala Tyr Pro Thr Pro Ala Arg Ser Lys
           35                  40                  45
   Lys Thr Met Leu Val Pro Lys Asn Ile Thr Ser Glu Ala Thr Cys Cys
       50                  55                  60
   Val Ala Lys Ala Phe Thr Lys Ala Thr Val Met Gly Asn Val Arg Val
   65                  70                  75                  80
   Glu Asn His Thr Glu Cys His Cys Ser Thr Cys Tyr Tyr His Lys Ser
                   85                  90                  95
<210> 52
   <211> 96
   <212> PRT
   <213> Sus scrofa
<220>
   <221> misc_feature
   <223> sous-unité alpha pLH et pFSH
<400> 52
   Phe Pro Asp Gly Glu Phe Thr Met Gln Gly Cys Pro Glu Cys Lys Leu
   1               5                   10                  15
   Lys Glu Asn Lys Tyr Phe Ser Lys Leu Gly Ala Pro Ile Tyr Gln Cys
               20                  25                  30
   Met Gly Cys Cys Phe Ser Arg Ala Tyr Pro Thr Pro Ala Arg Ser Lys
           35                  40                  45
   Lys Thr Met Leu Val Pro Lys Asn Ile Thr Ser Glu Ala Thr Cys Cys
       50                  55                  60
   Val Ala Lys Ala Phe Thr Lys Ala Thr Val Met Gly Asn Ala Arg Val
   65                  70                  75                  80
   Glu Asn His Thr Glu Cys His Cys Ser Thr Cys Tyr Tyr His Lys Ser
                   85                  90                  95
<210> 53
   <211> 111
   <212> PRT
   <213> Homo sapiens
<220>
   <221> misc_feature
   <223> sous-unité bêta hFSH
<400> 53
   Asn Ser Cys Glu Leu Thr Asn Ile Thr Ile Ala Ile Glu Lys Glu Glu
   1               5                   10                  15
   Cys Arg Phe Cys Ile Ser Ile Asn Thr Thr Trp Cys Ala Gly Tyr Cys
               20                  25                  30
   Tyr Thr Arg Asp Leu Val Tyr Lys Asp Pro Ala Arg Pro Lys Ile Gln
           35                  40                  45
   Lys Thr Cys Thr Phe Lys Glu Leu Val Tyr Glu Thr Val Arg Val Pro
       50                  55                  60
   Gly Cys Ala His His Ala Asp Ser Leu Tyr Thr Tyr Pro Val Ala Thr
   65                  70                  75                  80
   Gln Cys His Cys Gly Lys Cys Asp Ser Asp Ser Thr Asp Cys Thr Val
                   85                  90                  95
   Arg Gly Leu Gly Pro Ser Tyr Cys Ser Phe Gly Glu Met Lys Glu
               100                 105                 110
<210> 54
   <211> 132
   <212> PRT
   <213> Homo sapiens
<220>
   <221> misc_feature
   <223> sous-unité bêta hCG
<400> 54
   Ser Lys Glu Pro Leu Arg Pro Arg Cys Arg Pro Ile Asn Ala Thr Leu
   1               5                   10                  15
   Ala Val Glu Lys Glu Gly Cys Pro Val Cys Ile Thr Val Asn Thr Thr
               20                  25                  30
   Ile Cys Ala Gly Tyr Cys Pro Thr Met Thr Arg Val Leu Gln Gly Val
           35                  40                  45
   Leu Pro Ala Leu Pro Gln Val Val Cys Asn Tyr Arg Asp Val Arg Phe
   50 55 60
   Glu Ser Ile Arg Leu Pro Gly Cys Pro Arg Gly Val Asn Pro Val Val
   65 70 75 80
   Ser Tyr Ala Val Ala Leu Ser Cys Gln Cys Ala Leu Cys Arg Arg Ser
   85 90 95
   Thr Thr Asp Cys Gly Gly Pro Lys Asp His Pro Leu Thr Cys Asp Asp
   100 105 110
   Pro Arg Phe Gln Asp Ser Ser Ser Ser Lys Ala Pro Pro Pro Ser Leu
   115 120 125
   Pro Ser Pro Ser
   130
<210> 55
   <211> 123
   <212> PRT
   <213> Homo sapiens
<220>
   <221> misc_feature
   <223> sous-unité bêta hLH
<400> 55
   Ser Arg Glu Pro Leu Arg Pro Arg Pro Trp Cys His Pro Ile Asn Ala
   1 5 10 15
   Ile Leu Ala Val Glu Lys Glu Gly Cys Pro Val Cys Ile Thr Val Asn
   20 25 30
   Thr Thr Ile Cys Ala Gly Tyr Cys Pro Thr Met Met Arg Val Leu Gln
   35 40 45
   Ala Val Leu Pro Pro Leu Pro Gln Val Val Cys Thr Tyr Arg Asp Val
   50 55 60
   Arg Phe Glu Ser Ile Arg Leu Pro Gly Cys Pro Arg Gly Val Asp Pro
   65 70 75 80
   Val Val Ser Phe Pro Val Ala Leu Ser Cys Arg Cys Gly Pro Cys Arg
   85 90 95
   Arg Ser Thr Ser Asp Cys Gly Gly Pro Lys Asp His Pro Leu Thr Cys
   100 105 110
   Asp His Pro Gln Leu Ser Gly Leu Leu Phe Leu
   115 120
<210> 56
   <211> 121
   <212> PRT
   <213> Ovis aries
<220>
   <221> misc_feature
   <223> sous-unité bêta oLH
<400> 56
   Ser Arg Gly Pro Leu Arg Pro Leu Cys Gln Pro Ile Asn Ala Thr Leu
   1 5 10 15
   Ala Ala Glu Lys Glu Ala Cys Pro Val Cys Ile Thr Phe Thr Thr Ser
   20 25 30
   Ile Cys Ala Gly Tyr Cys Leu Ser Met Lys Arg Val Leu Pro Val Ile
   35 40 45
   Leu Pro Pro Met Pro Gln Arg Val Cys Thr Tyr His Glu Leu Arg Phe
   50 55 60
   Ala Ser Val Arg Leu Pro Gly Cys Pro Pro Gly Val Asp Pro Met Val
   65 70 75 80
   Ser Phe Pro Val Ala Leu Ser Cys His Cys Gly Pro Cys Arg Leu Ser
   85 90 95
   Ser Thr Asp Cys Gly Gly Pro Arg Thr Gln Pro Leu Ala Cys Asp His
   100 105 110
   Pro Pro Leu Pro Asp Ile Leu Phe Leu
   115 120
<210> 57
   <211> 121
   <212> PRT
   <213> Sus scrofa
<220>
   <221> misc_feature
   <223> sous-unité bêta pLH
<400> 57
   Ser Arg Gly Pro Leu Arg Pro Leu Cys Arg Pro Ile Asn Ala Thr Leu
   1 5 10 15
   Ala Ala Glu Asn Glu Ala Cys Pro Val Cys Ile Thr Phe Thr Thr Ser
               20                  25                  30
   Ile Cys Ala Gly Tyr Cys Pro Ser Met Val Arg Val Leu Pro Ala Ala
           35                  40                  45
   Leu Pro Pro Val Pro Gln Pro Val Cys Thr Tyr Arg Glu Leu Ser Phe
       50                  55                  60
   Ala Ser Ile Arg Leu Pro Gly Cys Pro Pro Gly Val Asp Pro Thr Val
   65                  70                  75                  80
   Ser Phe Pro Val Ala Leu Ser Cys His Cys Gly Pro Cys Arg Leu Ser
                   85                  90                  95
   Ser Ser Asp Cys Gly Gly Pro Arg Ala Gln Pro Leu Ala Cys Asp Arg
               100                 105                 110
   Pro Leu Leu Pro Gly Leu Leu Phe Leu
           115                 120
<210> 58
   <211> 110
   <212> PRT
   <213> Ovis aries
<220>
   <221> misc_feature
   <223> sous-unité bêta oFSH
<400> 58
   Ser Cys Glu Leu Thr Asn Ile Thr Ile Thr Val Glu Lys Glu Glu Cys
   1               5                   10                  15
   Ser Phe Cys Ile Ser Ile Asn Thr Thr Trp Cys Ala Gly Tyr Cys Tyr
               20                  25                  30
   Thr Arg Asp Leu Val Tyr Lys Asp Pro Ala Arg Pro Asn Ile Gln Lys
           35                  40                  45
   Ala Cys Thr Phe Lys Glu Leu Val Tyr Glu Thr Val Lys Val Pro Gly
       50                  55                  60
   Cys Ala His His Ala Asp Ser Leu Tyr Thr Tyr Pro Val Ala Thr Glu
   65                  70                  75                  80
   Cys His Cys Gly Lys Cys Asp Arg Asp Ser Thr Asp Cys Thr Val Arg
                   85                  90                  95
   Gly Leu Gly Pro Ser Tyr Cys Ser Phe Ser Asp Ile Arg Glu
               100                 105                 110
<210> 59
   <211> 109
   <212> PRT
   <213> Sus scrofa
<220>
   <221> misc_feature
   <223> sous-unité bêta pFSH
<400> 59
   Cys Glu Leu Thr Asn Ile Thr Ile Thr Val Glu Lys Glu Glu Cys Gly
   1               5                   10                  15
   Phe Cys Ile Ser Ile Asn Thr Thr Trp Cys Ala Gly Tyr Cys Tyr Thr
               20                  25                  30
   Arg Asp Leu Val Tyr Lys Asp Pro Ala Arg Pro Asn Ile Gln Lys Thr
           35                  40                  45
   Cys Thr Phe Lys Glu Leu Val Tyr Glu Thr Val Lys Val Pro Gly Cys
       50                  55                  60
   Ala His His Ala Asp Ser Leu Tyr Thr Tyr Pro Val Ala Thr Glu Cys
   65                  70                  75                  80
   His Cys Gly Lys Cys Asp Ser Asp Ser Thr Asp Cys Thr Val Arg Gly
                   85                  90                  95
   Leu Gly Pro Ser Tyr Cys Ser Phe Ser Glu Met Lys Glu
               100                 105
<210> 60
   <211> 49
   <212> PRT
   <213> Homo sapiens
<220>
   <221> misc_feature
   <223> Région N terminale du récepteur de la hFSH
<400> 60
   Cys His His Arg Ile Cys His Cys Ser Asn Arg Val Phe Leu Cys Gln
   1               5                   10                  15
   Glu Ser Lys Val Thr Glu Ile Pro Ser Asp Leu Pro Arg Asn Ala Ile
                       20                  25                  30
   Glu Leu Arg Phe Val Leu Thr Lys Leu Arg Val Ile Gln Lys Gly Ala
           35                  40                  45
   Phe
<210> 61
   <211> 7
   <212> PRT
   <213> Artificial Sequence
<220>
   <223> Région épitopique de CA5 sur bêtaFSH
<400> 61
   Arg Phe Cys Ile Ser Ile Asn
   1               5
<210> 62
   <211> 6
   <212> PRT
   <213> Artificial Sequence
<220>
   <223> Région épitopique de CA5 sur bêtaFSH
<400> 62
   Arg Val Pro Gly Cys Ala
   1               5
<210> 63
   <211> 12
   <212> PRT
   <213> Artificial Sequence
<220>
   <223> Région épitopique de CA5 sur bêtaFSH
<400> 63
   Arg Gly Leu Gly Ser Tyr Cys Ser Phe Gly Glu Met
   1               5                   10
<210> 64
   <211> 5
   <212> PRT
   <213> Artificial Sequence
<220>
   <223> Région épitopique de CA5 sur récepteur de la hFSH
<400> 64
   His His Arg Ile His
   1               5
<210> 65
   <211> 4
   <212> PRT
   <213> Artificial Sequence
<220>
   <223> Région épitopique de CA5 sur récepteur de la hFSH
<400> 65
   Leu Gln Glu Lys
   1
<210> 66
   <211> 5
   <212> PRT
   <213> Artificial Sequence
<220>
   <223> Région épitopique de CH10 sur alphaFSH
<400> 66
   Gln Asp Cys Pro Glu
   1               5
<210> 67
   <211> 8
   <212> PRT
   <213> Artificial Sequence
<220>
   <223> Région épitopique de CH10 sur bêtaFSH
<400> 67
   Ser Cys Glu Leu Thr Asn Ile Thr
   1               5
<210> 68
   <211> 6
   <212> PRT
   <213> Artificial Sequence
<220>
   <223> Région épitopique de CH10 sur bêtaFSH
<400> 68
   Ser Asn Trp Ala Gly Tyr
   1               5
<210> 69
   <211> 8
   <212> PRT
   <213> Artificial Sequence
<220>
   <223> Région épitopique de CH10 sur bêtaFSH
<400> 69
   Leu Gly Pro Ser Tyr Gly Glu Met
   1               5
<210> 70
   <211> 5
   <212> PRT
   <213> Artificial Sequence
<220>
   <223> Région épitopique de CH10 sur récepteur de la hFSH
<400> 70
   Cys His His Cys Ser
   1               5